(19) Europäisches Patentamt
European Patent Office
Office européen des brevets

(11) Veröffentlichungsnummer: **0 248 349**
**A2**

# (12) EUROPÄISCHE PATENTANMELDUNG

(21) Anmeldenummer: **87107730.1**

(22) Anmeldetag: **27.05.87**

(51) Int. Cl.[4]: **C07D 239/34** , C07D 239/42 , C07D 239/46 , C07D 239/48 , A01N 43/54

(30) Priorität: **04.06.86 DE 3618815**
**31.12.86 DE 3644799**

(43) Veröffentlichungstag der Anmeldung: **09.12.87 Patentblatt 87/50**

(84) Benannte Vertragsstaaten: **AT BE CH DE ES FR GB GR IT LI NL SE**

(71) Anmelder: **HOECHST AKTIENGESELLSCHAFT**
**Postfach 80 03 20**
**D-6230 Frankfurt am Main 80(DE)**

(72) Erfinder: **Giencke, Wolfgang, Dr.**
**Am Steinberg 45**
**D-6238 Hofheim am Taunus(DE)**
Erfinder: **Heubach, Günther, Dr.**
**Luisenstrasse 15**
**D-6233 Kelkheim (Taunus)(DE)**
Erfinder: **Mildenberger, Hilmar, Dr.**
**Fasanenstrasse 24**
**D-6233 Kelkheim (Taunus)(DE)**
Erfinder: **Fuss, Andreas, Dr.**
**Lindigstrasse 24**
**D-8757 Karlstein(DE)**
Erfinder: **Sachse, Burkhard, Dr.**
**An der Ziegelei 30**
**D-6233 Kelkheim (Taunus)(DE)**
Erfinder: **Kern, Manfred, Dr.**
**Im Traminer Weg 8**
**D-6501 Lörzweiler(DE)**
Erfinder: **Knauf, Werner, Dr.**
**Im Kirschgarten 24**
**D-6239 Eppstein/ Taunus(DE)**
Erfinder: **Waltersdorfer, Anna, Dr.**
**Rauenthaler Weg 28**
**D-6000 Frankfurt/M.(DE)**

## (54) Neue Pyrimidin-Derivate, deren Herstellung und Verwendung.

(57) Verbindungen der Formel I oder deren Salze,

EP 0 248 349 A2

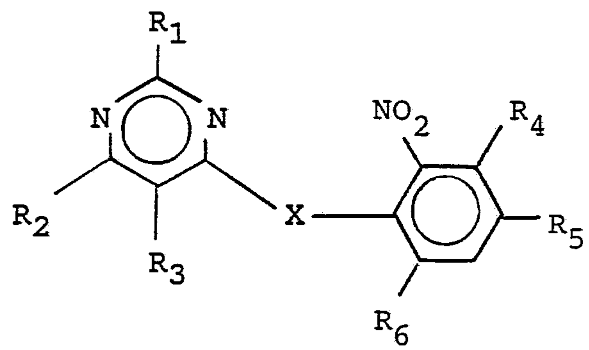

(I),

worin

$R_1$, $R_2$ Wasserstoff; Halogen, Cyano; (Subst.) (Cyclo)alkyl, (Halo)alkenyl; (Halo)alkinyl; (Subst)Amino, Alkylthio; (Halo)alkoxy; Alkysulfinyl oder -sulfonyl; (Subst.)Phenyl; (Subst.)Phenoxy; $R_3$ Wasserstoff, Halogen, Cyano, Nitro, (Halo)alkyl, (Halo)alkenyl, (Subst.)Phenyl oder (Subst.) Phenoxy; $R_4$ H, Alkoxy, Halogen, Alkylthio, (Subst.)Amino oder (Subst.)Phenoxy; $R_5$ (Halo)Alkyl, ein subst. Carbonyl-Rest, $S(O)_nR_9$; $S(O)_nOR_9$, $S(O)_nN(R_7)(R_8)$; Halogen; Cyano, Nitro, Haloalkoxy; $R_6$ $NO_2$ oder $CF_3$; X eine der Gruppen $-NR_{10}-NR-_{10}$, $-NR_{10}-$ oder --0-bedeuten mit der Maßgabe, daß diejenigen Verbindungen der Formel I, worin X eine Gruppe $-NR_{10}-$ und $R_5$ $CF_3$ oder $NO_2$ bedeuten, ausgenommen sind, besitzen hervorragende fungizide und insektizide Eigenschaften.

# Neue Pyrimidin-Derivate, deren Herstellung und Verwendung

Die vorliegende Erfindung betrifft neue Pyrimidin-Derivate der nachstehenden Formel I, deren Herstellung und Verwendung als Schädlingsbekämpfungsmittel.

Pyrimidin-Derivate mit mikrobioziden Eigenschaften sind bereits bekannt (vgl. EP-A-139 613). Die neuen Pyrimidin-Derivate der Formel I zeigen ausgezeichnete fungizide und insektizide Wirkungen.

Gegenstand der vorliegenden Erfindung sind daher die Pyrimidin-Derivate der Formel I oder deren Salze

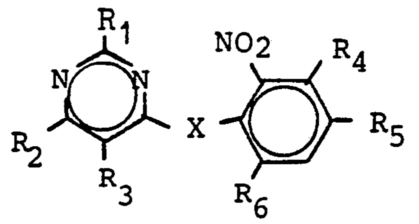

(I),

worin

$R_1$ und $R_2$ unabhängig voneinander Wasserstoff; Halogen; Cyano; ($C_1$-$C_8$)-Alkyl; ($C_1$-$C_8$)-Haloalkyl; ($C_1$-$C_8$)-Alkyl, das ein-oder zweifach durch Nitro, Cyano, ($C_1$-$C_4$-Alkoxy, ($C_1$-$C_4$)-Alkylthio oder -N($R_7$)($R_8$) substituiert ist; ($C_3$-$C_8$)-Cycloalkyl, das durch ($C_1$-$C_4$)Alkyl substituiert sein kann; ($C_2$-$C_4$)-Alkenyl; ($C_2$-$C_4$)-Haloalkenyl; ($C_3$-$C_4$)-Alkinyl; ($C_3$-$C_4$)-Haloalkinyl; ($C_5$-$C_6$)-Cycloalkenyl; eine Gruppe -N($R_7$)($R_8$); ($C_1$-$C_8$)-alkylthio; ($C_1$-$C_8$)-Alkoxy; ($C_1$-$C_8$)Haloalkoxy; ($C_1$-$C_8$)Alkylsulfinyl; ($C_1$-$C_8$)-Alkylsulfonyl; Phenyl, das gegebenenfalls durch Halogen, Nitro, Cyano, ($C_1$-$C_4$)-Alkyl, ($C_1$-$C_4$)-Alkoxy, ($C_1$-$C_4$)-Alkylthio oder ($C_1$-$C_4$)-Haloalkyl substituiert ist; oder Phenoxy, das gegebenenfalls durch Halogen, $NO_2$, ($C_1$-$C_4$)Alkyl, ($C_1$-$C_4$)Haloalkyl oder ($C_1$-$C_4$)Alkoxy substituiert ist;

$R_3$ Wasserstoff; Halogen; Cyano; Nitro; ($C_1$-$C_8$)-Alkyl; ($C_1$-$C_8$)-Haloalkyl; ($C_2$-$C_4$)-Alkenyl; ($C_2$-$C_4$)-Haloalkenyl; Phenyl, das gegebenenfalls durch Halogen, Nitro, Cyano, ($C_1$-$C_4$)-Alkyl, ($C_1$-$C_4$)-Alkoxy, ($C_1$-$C_4$)-Alkylthio oder ($C_1$-$C_4$)-Haloalkyl substituiert ist oder Phenoxy, das gegebenenfalls durch Halogen, $NO_2$, ($C_1$-$C_4$)Alkyl, ($C_1$-$C_4$)-Haloalkyl oder ($C_1$-$C_4$)Alkoxy substituiert ist;

$R_4$ Wasserstoff; Halogen; ($C_1$-$C_4$)Alkoxy; ($C_1$-$C_4$)Alkylthio; ein Gruppe N($R_7$)($R_8$) oder Phenoxy, das durch Halogen substituiert sein kann;

$R_5$ ($C_1$-$C_4$)Alkyl, ($C_1$-$C_8$-Alkoxycarbonyl), ($C_1$-$C_4$-Alkenyloxy)carbonyl, -$CO_2H$; ($C_3$-$C_4$-Alkinyloxy)carbonyl; ($C_1$-$C_4$-Haloalkoxy)carbonyl; ($C_2$-$C_4$-Haloalkenyloxy)carbonyl; ($C_3$-$C_4$-Haloalkinyloxy)carbonyl; -CON($R_7$)($R_8$); ($C_1$-$C_4$-Alkyl)carbonyl; ($C_2$-$C_4$-Alkenyl)carbonyl; ($C_3$-$C_4$-Alkinyl)carbonyl; ($C_3$-$C_4$-Haloalkyl)carbonyl; ($C_2$-$C_4$-Haloalkenyl)carbonyl; ($C_3$-$C_4$-Haloalkinyl)carbonyl; Cyano, Nitro; $S(O)_nR_9$; $S(O)_nOR_9$ oder $S(O)_nN(R_7)(R_8)$; Halogen; ($C_1$-$C_4$)Haloalkoxy; ($C_1$-$C_4$)Haloalkyl; Formyl;

n eine der Zahlen 0, 1 oder 2;

$R_6$ $NO_2$ oder $CF_3$;

$R_7$ und $R_8$ unabhängig voneinander Wasserstoff; ($C_1$-$C_4$)-Alkyl; ($C_3$-$C_7$)-Cycloalkyl, das durch ($C_1$-$C_4$)-Alkyl substituiert sein kann; oder Phenyl, das durch Halogen, ($C_1$-$C_4$)-Alkyl, ($C_1$-$C_4$)-Haloalkyl, ($C_1$-$C_4$)-Alkoxy, ($C_1$-$C_4$)Alkylthio, Nitro oder Cyano substituiert sein kann;

$R_9$ ($C_1$-$C_4$)-Alkyl; ($C_2$-$C_4$)-Alkenyl, ($C_2$-$C_4$)-Alkinyl oder ($C_3$-$C_7$)-Cycloalkyl, das durch ($C_1$-$C_4$)-Alkyl substituiert sein kann;

$R_{10}$ unabhängig voneinander Wasserstoff; ($C_1$-$C_4$)-Alkyl; ($C_3$-$C_7$)-Cycloalkyl, das durch ($C_1$-$C_4$)-Alkyl substituiert sein kann; ($C_1$-$C_4$-Alkoxy)carbonyl; ($C_1$-$C_4$-Alkyl)carbonyl; ($C_1$-$C_4$)-Alkoxy; Phenyl, das gegebenenfalls durch Halogen, Nitro, Cyano, ($C_1$-$C_4$)-Alkyl, ($C_1$-$C_4$)-Alkoxy, ($C_1$-$C_4$)-Alkylthio oder ($C_1$-$C_4$)-Haloalkyl substituiert sein kann, und

X eine der Grupper -$NR_{10}$-$NR_{10}$, -$NR_{10}$-oder -O-bedeuten, mit der Maßgabe, daß diejenigen Verbindungen der Formel I, worin X eine Gruppe -$NR_{10}$-und $R_5$ $CF_3$ oder $NO_2$ bedeuten, ausgenommen sind.

Als Salze kommen insbesondere die in der Landwirtschaft einsetzbaren Salze infrage, wie beispielsweise Alkali-, Erdalkali-, Ammonium-oder substituierte Ammoniumsalze. Die Salzbildung kann an der Gruppe X= $-NR_{10}$-oder $-NR_{10}NH$-oder im Falle $R_5$= COOH erfolgen. Die Salze werden nach üblichen Methoden aus den Vernindungen der Formel I hergestellt.

Halogen bedeutet insbesondere Fluor, Chlor oder Brom. Die Vorsilbe "Halo" in der Bezeichnung eines Substituenten bedeutet hier und im folgenden, daß dieser Substituent einfach oder mehrfach bei gleicher oder verschiedener Bedeutung auftreten kann. Die Vorsilbe Halo beinhaltet Fluor, Chlor, Brom und Jod, insbesondere Fluor, Chlor, Brom. Als Beispiele für Haloalkyl seien genannt: $CF_3$, $CHF_2$, $CH_2F$, $CCl_3$, $CHCl_2$, $CH_2Cl$, $CBr_3$, $CF_2Cl$, $CClF_2$, $CF_2CHF_2$, $CF_2CF_3$, $CF_2CHFCF_3$, $CF_2CHClF$, $CF_2CHCl_2$, $CCl_2CCl_3$, n-$C_3$-$F_7$, $-CH(CF_3)_2$, n-$C_4F_9$, n-$C_5F_{11}$, n-$C_6F_{13}$,n-$C_7F_{15}$ und n-$C_8F_{17}$.

Als Beispiele für Haloalkoxy seien genannt: $OCF_3$, $OCHF_2$, $OCF_2CHF_2$, $OCF_2CHFCF_3$, $OCF_2Cl$, $OCF_2CF_2CF_3$, $OCF_2CHFCl$ und $OCF_2CF_2CF_2CF_3$.

Als Beispiele für ($C_2$-$C_4$)-Haloalkenyl seien genannt:

$CH_2=CF$-; $F_2C=CF$-; $Cl_2C=CC1$-

Bevorzugte Verbindungen der Formel I gemäß obiger Definition sind solche, bei denen $R_1$ Wasserstoff; Halogen; Cyano; ($C_1$-$C_8$)-Alkyl; ($C_1$-$C_8$)-Haloalkyl; ($C_1$-$C_8$)-Alkyl, das ein-oder zweifach durch ($C_1$-$C_4$)-Alkoxy oder ($C_1$-$C_4$)-Alkylthio substituiert ist; ($C_3$-$C_8$)-Cycloalkyl, das durch ($C_1$-$C_4$)Alkyl substituiert sein kann; ($C_1$-$C_8$)-Alkylthio; ($C_1$-$C_8$)-Alkoxy; ($C_1$-$C_8$)Alkylsulfinyl; ($C_1$-$C_8$)-Alkylsulfonyl; Phenyl, das gegebenenfalls ein-oder mehrfach durch Halogen und/oder ein-oder dreifach durch Nitro, Cyano, ($C_1$-$C_4$)-Alkyl, ($C_1$-$C_4$)-Alkoxy oder ($C_1$-$C_4$)-Haloalkyl substituiert ist, $R_2$ die Bedeutung von $R_1$ außer gegenbenenfalls substituiertes Phenyl, $R_3$ Wasserstoff; Halogen; Cyano; Nitro; ($C_1$-$C_8$)-Alkyl; ($C_1$-$C_8$)-Haloalkyl oder Phenoxy, das gegebenenfalls ein- bis dreimal durch Halogen,$NO_2$, ($C_1$-$C_4$)Alkyl, ($C_1$-$C_4$)Haloalkyl oder ($C_1$-$C_4$)Alkoxy substituiert ist;

$R_4$ Wasserstoff; Halogen; ($C_1$-$C_4$)Alkoxy;

$R_5$ ($C_1$-$C_4$)Alkyl, ($C_1$-$C_4$-Alkoxy)carbonyl, $-CO_2H$; $-CON(R_7)(R_8)$; ($C_1$-$C_4$-Alkyl)carbonyl; ($C_3$-$C_4$-Haloalkyl)-carbonyl;Cyano, Halogen Nitro oder $S(O)_nN(R_7)(R_8)$;

n eine der Zahlen 0, 1 oder 2;

$R_6$ $NO_2$ oder $CF_3$;

$R_7$ und $R_8$ unabhängig voneinander Wasserstoff; ($C_1$-$C_4$)-Alkyl; ($C_3$-$C_7$)-Cycloalkyl, das durch ($C_1$-$C_4$)-Alkyl substituiert sein kann; oder Phenyl, das ein-bis dreifach durch Halogen, ($C_1$-$C_4$)-Alkyl, ($C_1$-$C_4$)-Haloalkyl oder ($C_1$-$C_4$)-Alkoxy, substituiert sein kann;

$R_{10}$ unabhängig von einander Wasserstoff; ($C_1$-$C_4$)-Alkyl; und

X eine der Gruppe $-NR_{10}-NR_{10}$ oder $-NR_{10}$-oder -O-bedeuten.

Insbesondere bevorzugt hiervon sind Verbindungen der Formel I, worin

$R_1$ ($C_1$-$C_8$)-Haloalkyl, ($C_1$-$C_4$)Alkyl; ($C_3$-$C_6$)-Cycloalkyl, Phenyl oder Phenyl, das wie oben angegeben substituiert ist, insbesondere hiervon ($C_1$-$C_8$)-Haloalkyl,

$R_2$ H oder ($C_1$-$C_4$)-Alkyl,

$R_3$ H oder Halogen,

$R_4$ H oder Halogen,

$R_5$ ($C_1$-$C_4$)Alkoxycarbonyl, Cyano; ($C_1$-$C_4$)-Haloalkoxy, ($C_1$-$C_4$)-Haloalkyl oder Chlor und

$R_6$ $NO_2$ und X = NH oder -O-bedeuten.

Gegenstand der vorliegenden Erfindung sind auch Verfahren zur Herstellung der Verbindungen der Formel I, dadurch gekennzeichnet, daß man eine Verbindung der Formel II in Gegenwart einer Base

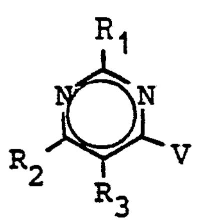
(II)

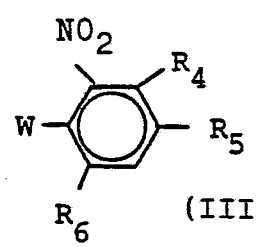
(III)

mit einer Verbindung der Formel III umsetzt.

Die Substituenten $R_1$, $R_2$, $R_3$, $R_4$, $R_5$ und $R_6$ haben die Bedeutungen wie in der Formel I. V und W stehen für Halogen, Hydroxy, eine Gruppe $-NR_{10}NHR_{10}$ oder eine Gruppe $-NHR_{10}$, wobei für den Fall, daß W für Halogen steht, V Hydroxy, eine Gruppe $-NR_{10}-NHR_{10}$ oder eine Gruppe $-NHR_{10}$ bedeuten muß und für den Fall, daß V für Halogen steht, W Hydroxy, eine Gruppe $-NR_{10}NHR_{10}$ oder eine Gruppe $-NHR_{10}$ bedeuten muß. Der Rest $R_{10}$ hat die in Formel I angegebene Bedeutung. Der Begriff Halogen repräsentiert Fluor, Chlor,, Brom und Jod, insbesondere Chlor und Brom.

Die Umsetzung der Verbindungen II mit III erfolgt vorzugsweise in inerten aprotischen Lösungsmitteln wie z.B. Acetonitril, Dichlormethan, Toluol, Xylol, Tetrahydrofuran,.Dioxan, Dialkylether wie Diethylenglykoldialkylether, insbesondere Diethylenglykoldiethylether, oder DMF bei Temperaturen zwischen -10°C und der Siedetemperatur des Lösungsmittels. Als Basen eignen sich die für diesen Reaktionstyp üblichen Basen wie beispielsweise Carbonate und Hydrogencarbonate von Alkali-und Erdalkalimetallen, Alkalihydroxide, Alkalialkoholate wie K-tert.-butylat, tert.-Amine, Pyridin oder substituierte Pyridinbasen (z.B. 4-Dimethylaminopyridin).

Auch ein zweites Äquivalent der Verbindungen der allgemeinen Formel II kann die Funktion der Base übernehmen.

Als Basen kommen dann nur diejenigen Verbindungen der Formeln II in Betracht, in denen V eine Gruppe $-NR_{10}NR_{10}$ oder eine Gruppe $-NHR_{10}$ bedeutet.

Die Umsetzung erfolgt sowohl in aprotischen Lösungsmitteln wie z.B. Acetonitril, Diethylenglykoldimethylether, Dichlormethan, Toluol, Tetrahydrofuran, Dioxan oder DMF als auch in protischen Lösungsmitteln wie z.B. Methanol, Ethanol oder Isopropanol im Temperaturbereich von 0° bis zur Siedetemperatur des Lösungsmittels.

Die Verbindungen der Formel II lassen sich nach grundsätzlich bekannten Verfahren synthetisieren (vgl. z.B. J.Org.Chem. 26, 4504; US-PS 4 343 803 und Houben-Weyl, Methoden der Organischen Chemie, Band 10/2, Seite 252). Die Verbindungen der Formel III sind sum großen Teil bekannt und nach üblichen Methoden leicht zugänglich (vgl. Liebig's Annalen der Chemie 366, 93; GB-PS 951.770; CA 79, 78364x; CA 84 30640k). Die Verbindungen der Formel III mit W= OH lassen sich aus den entsprechenden Verbindungen, bei denen anstelle der Nitrogruppen Wasserstoff steht durch übliche Nitrierungsreaktionen herstellen. Verbindungen mit W= Halogen werden durch anschließende Halogenierung erhalten. Spezielle Verbindungen der Formel III mit $R_5$ = Haloalkoxy und deren Herstellung werden in der Deutschen Patentanmeldung P 36 44 798.6 beschrieben.

Die erfindungsgemäßen Verbindungen der Formel I zeichnen sich durch eine hervorragende fungizide Wirkung aus. Bereits in da pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener wirtschaftlich wichtiger, phytopathogener Pilze, wie z.B. Piricularia oryzae, Echte Mehltauarten, Fusariumarten, Plasmopora viticola und Pseudoperonospora cubensis. Besonders gut werden Benzimidazol-und Dicarboximidsensible und -resistente Botrytis cinerea-Stämme erfaßt, sowie BCM sensible und -resistente Pseudocercosporella herpotrichoides Stämme. (BCM= Wirkstoff Carbendazim).

Die Verbindungen der Formel I eignen sich auch für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr-und Schneidölen.

Die Verbindungen der Formel I, insbesondere solche der nachfolgend aufgeführten Beispiele können auch vorteilhaft mit anderen bekannten Fungiziden kombiniert werden.

Als solche Kombinationspartner kommen in Betracht die Produkte:

Imazalil, Prochloraz, Fenapanil, SSF105, Triflumizol, PP969, Flutriafol, BAY-MEB 6401,
Propiconazol, Etaconazol, Diclobutrazol, Bitertanol, Triadimefon, Triadimenol, Fluotrimazol, Tridemorph, Dodemorph, Fenpropimorph, Falimorph, S-32165,
Chlobenzthiazone, Parinol, Buthiobat, Fenpropidin, Triforine, Fenarimol,
Nuarimol, Triarimol, Ethirimol, Dimethirimol, Bupirimate, Rabenzazole, Tricyclazole, Ofurace, Furalaxyl, Benalaxyl, Metalaxyl, Pencyuron, Oxadixyl, Cyprofuram, Dichlomezin, Probenazole, Fluobenzimine, Pyroxyfur, NK-483, PP-389, Pyroquilon, Hymexazole, Fenitropan, UHF-8227, Tolclofosmethyl, Ditalimfos, Edifenphos, Pyrazophos, Isoprothiolane, Cymoxanil, Dichloruanid, Captafol, Captan, Folpet, Tolylfluanid, Chlorothalonil, Etridiazol, Iprodione, Procymidon, Vinclozolin, Metomeclan, Myclozolin, Dichlozolinate, Fluorimide, Drazoxolon, Chinomethionate, Nitrothalisopropyl, Dithianon, Dinocap, Binapacryl,
Fentinacetate, Fentinhydroxide, Carboxin, Oxycarboxin, Pyracarbolid, Methfuroxam, Fenfuram, Furmecyclox, Benodanil, Mebenil, Mepronil,

Flutolanil, Fuberidazole, Thiabendazole, Carbendazim, Benomyl, Thiofanate, Thiofanate-methyl, CGD-94240F, IKF-1216.

Mancozeb, Maneb, Zineb, Nabam, Thiram, Probineb, Prothiocarb, Propamocarb, Dodine, Guazatine, Dicloran, Quintozene, Chloroneb, Tecnazene, Biphenyl, Anilazine, 2-Phenylphenol, Kupferverbindungen wie Cuoxychlorid, Oxine-Cu, Cu-oxide, Schwegel, Fosetylaluminium,

Natrium-dodecylbenzolsulfonat,

Natrium-dodecylsulfat,,

Natrium-C13/C15-alkoholethersulfonat,

Natrium-cetostearylphosphatester,

Dioctyl-natriumsulfosuccinat,

Natrium-isopropylnaphthalinsulfonat,

Natrium-methylenbisnaphthalinsulfonat,

Cetyl-trimethyl-ammoniumchlorid,

Salze von langkettigen primären, sekundären oder tertiären Aminen,

Alkyl-propylenamine,

Lauryl-pyridiniumbromid,

Ethoxilierte quaternierte Fettamine,,

Alkyl-dimethyl-benzyl-ammoniumchlorid und 1-Hydroxyethyl-2-alkyl-imidazolin.

Die oben genannten Wirkstoffe, für die common-names angegeben wurden, sind zum großen Teil in CH. R. Worthing, S.B. Walker, The Pesticide Manual, 7. Auflage (1983), British Crop Protection Council beschrieben. Verbindungen, für die Nummerncodes angegeben sind, besitzen folgende Strukturen

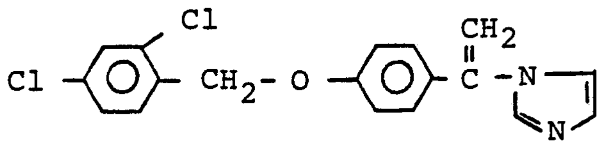

SS F 105

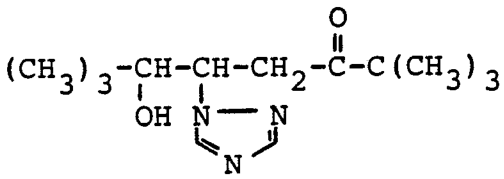

PP 969

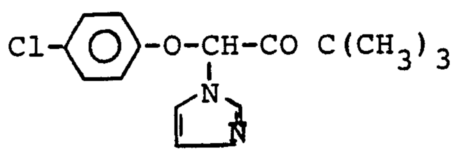

Bay-Meb 6401

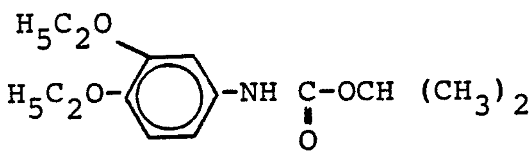

S - 32165

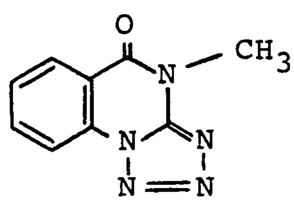

PP - 389

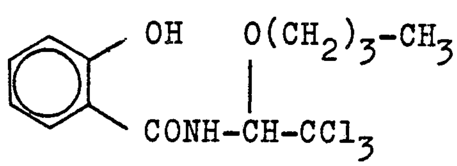

NK - 483

Unerwarteterweise ist die fungizide Wirkung der Wirkstoffkombinationen in vielen Fällen höher, als die Summe der Wirkung der Einzelkomponenten, die nach der sogenannten Colby-Formel berechnet wird, vgl. S.R. Colby, Weeds 15, 20-22 (1967). Damit zeigen die Wirkstoffkombinationen synergistische Effekte.

Die Gewichtsverhältnisse der Wirkstoffgruppen in den Wirkstoffkombinationen können in relativ großen Bereichen schwanken. Im allgemeinen entfallen auf 1 Gew.-Teil der Formel I 0,0005 bis 10 Gew.-Teile, vorzugsweise 0,001 bis 2,5 Gew.-Teile des Kombinationspartners.

Die erfindungsgemäßen Wirkstoffkombinationen weisen eine starke biozide Wirkung auf und können daher vorteilhaft zur Bekämpfung von Mikroorganismen, insbesondere im Pflanzenschutz, eingesetzt werden. Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut und des Bodens.

Die Wirkstoffkombinationen haben ein sehr breites Wirkungsspektrum und können angewandt werden gegen parasitäre Pilze, die oberirdische Pflanzenteile befallen oder die Pflanzen vom Boden aus angreifen.

Die erfindungsgemäßen Wirkstoffkombinationen zeigen eine ausgezeichnete Wirkung als Saatgutbeizmittel gegen phytopathogene Pilze, wie z.B. Tilletia-, Urocystis-, Ustilago-, Septoria-, Typhula-, Rhynchosporium-, Helminthosporium-und Fusarium-Arten.

Auch als Bodenbekämpfungsmittel lassen sie sich zur Bekämpfung phytopathogener Pilze einsetzen, die Wurzelfäulen und Tracheomykosen verursachen wie z.B. Krankheitserreger der Gattungen Pythium, Verticillium, Phialophora, Rhizoctonia, Fusarium und Thielaviopsis.

Bei direkter Applikation der Wirkstoffkombinationen auf die oberirdischen Pflanzenteile lassen sich eine Vielzahl wirtschaftlich wichtiger Krankheitserreger hervorrangend kontrollieren, wie z.B. echte Mehltaupilze (Erysiphe-, Uncinula-, Sphaerotheca-, Podosphaera-Arten, Leveillula tauriva), Rostpilze, Venturia-Arten, Cercospora-Arten, Alternaria-Arten, Botrytis-Arten, Phytophthora-Arten, Peronospora-Arten, Piricularia oryzae und Pellicularia sasakii.

Die Verbindungen der Formel I eignen sich bei guter Pflanzenverträglichkeit und günstiger Warmblütertoxizität auch zur Bekämpfung von tierischen Schädlingen, insbesondere Insekten, Spinnentieren und Nematoden, besonders bevorzugt zur Bekämpfung von Insekten, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp.

Aus der Ordnung der Symphyla z.B. Scutigerella immaculata.

Aud der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.,

Aus der Ordnung der Anoplura z.B. Phylloera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadratum, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicornyne brassicae,, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp, Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Phizopertha dominica, Bruchidius obtectus, Acanthos celides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus

spp., Hypobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Archnida z.B. Scorpio maurus, Latrodectus mactans.

Gegenstand der Erfindung sind daher auch Schädlingsbekämpfungsmittel, welche die Verbindungen der Formel I neben geeigneten Formulierungshilfsmitteln enthalten.

Unter Schädlingen sind hierbei die obengenannten Mikroorganismen, insbesondere Pilze, und tierische Schädlinge, insbesondere Pflanzenschädlinge, zu verstehen.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer einem Verdünnungs-oder Inertstoff noch Netzmittel, z.B. polyoxethylierte Alkylphenole, polyoxethylierte Fettalkohole, Alkyl-oder Alkylphenol-sulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten. Ihre Herstellung erfolgt in üblicher Weise, z.B. durch Mahlen und Vermischen der Komponenten.

Emulgierbare Konzentrate können z.B. durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden

Arylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel kann man durch Vermahlen des Wirkstoffes mit fein verteilten festen Stoffen, z.B. Talkum, natürlichen Tonen wie Kaolin, Bentonit, Poryphillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinite, oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration beider Wirkstoffe etwa 10 bis 90 Gew.-%; der Rest zu 100 Gew.-% besteht aus üblichen Formulierungsbestandteilen. Bei emulgierbaren Konzentraten kann die Wirkstoffkonzentration beider Wirkstoffe etwa 10 bis 80 Gew.-% betragen. Staubförmige Formulierungen enthalten meistens 5 bis 20 Gew.-% der Wirkstoffe, versprühbare Lösungen etwa 1 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstoffformulierungen gegebenenfalls die jeweils üblichen Haft-, Netz-, Dispergier-, Emulgier-, Penetrations-, Lösungsmittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Die Aufwandmengen der Wirkstoffe der Formel I können innerhalb weiter Grenzen variieren und liegen im allgemeinen zwischen 0,010 und 5 kg/ha.

Die nachfolgenden Beispiele dienen zur Erläuterung der Erfindung.

A. Formulierungsbeispiele

Beispiel A:

Ein Stäubemittel wird erhalten, indem man

10 Gewichtsteile Wirkstoff
und 90 Gewichtsteile Talkum als Inertstoff mischt und in einer Schlagmühle zerkleinert.

Beispiel B:

Ein in Wasser leicht dispergierbares, benetzbares Pulver wird erhalten, indem man

25 Gewichtsteile Wirkstoff
64 Gewichtsteile kaolinhaltigen Quarz als Inertstoff
10 Gewichtsteile ligninsulfonsaures Kalium
und 1 Gewichtsteil oleylmethyltaurinsaures Natrium als Netz-und Dispergiermittel mischt und in einer Stiftmühle mahlt.

Beispiele C:

Ein in Wasser leicht dispergierbares Dispersionskonznetrat wird erhalten, indem man

20 Gewichtsteile Wirkstoff mit
6 Gewichtsteilen Alkylphenolpolyglykoläther (Triton $^{\times}$ 207)
3 Gewichtsteilen Isotridecanolpolyglykoläther (8 AeO)
und
71 Gewichtsteilen paraffinischem Mineralöl (Siedebereich z.B. ca. 255 bis über 377°C)
mischt und in einer Reibkugelmühle auf eine Feinheit von unter 5 Mikron vermahlt.

Beispiel D:

Ein emulgierbares Konzentrat wird erhalten aus

15 Gewichtsteilen Wirkstoff
75 Gewichtsteilen Cyclohexanon als Lösungsmittel und 10 Gewichtsteilen oxäthyliertes Nonylphenol (10 AeO) als Emulgator

B. CHEMISCHE BEISPIELE

Beispiel (1.32)

4-(5-Chlor-2-trichlormethyl-4-pyrimidinylamino)-3,5-dinitro-benzonitril

Zu einer Lösung von 4,45 g (0,018 mol) 5-Chlor-2-trichlormethyl-4-pyrimidyl-amin in 130 ml abs. THF wurden bei -5°C 5,15 g (0,092 mol) KOH-Pulver gefügt. Anschließend tropfte man eine Lösung von 4,10 g (0,018 mol) 4-Chlor-3,5-dinitrobenzonitril in 30 ml absolutem THF so hinzu, daß die Temperatur nicht über 0°C stieg. Nach 5-stündiger Nachrührzeit wurden die festen Komponenten abgesaugt und das Lösungsmittel im Vakuum eingedampft. Der Rückstand wurde in $H_2O$ aufgenommen und mit verdünnter Salzsäure angesäuert. Danach extrahierte man mit Chloroform, trocknete über $MgSO_4$, dampft im Vakuum ein und kristallisierte den erhaltenen Feststoff aus Diisopropylether um.
Schmp. 214 - 215°C

Beispiel 2.10

Kalium-5-chlor-N-(2,6-dinitro-4-ethoxycarbonyl-phenyl)-2-(1,1,2,2-tetrafluorethyl)-4-pyrimidinyl-aminat

Zu einer Lösung von 9,18 g (0,04 mol) 5-Chlor-2-(1,1,2,2-tetrafluorethyl)-4-pyrimidinyl-amin in 200 ml absolutem THF wurden bei -5°C 4,48 g (0,08 mol) KOH-Pulver gefügt. Danach tropfte man eine Lösung von 10,98 g (0,04 mol) Ethyl-4-chloro-3,5-dinitro-benzoat in 100 ml absolutem THF so hinzu, daß die Temperatur nicht über 0°C stieg. Man ließ 3h bei 0°C nachrühren and ließ dann das Reaktionsgemisch innerhalb von 2h auf Raumtemperatur erwärmen. Die festen Bestandteile wurden anschließend abgesaugt und die Mutterlauge eingedampft. Der feste Rückstand wurde aus Ethylacetat umkristallisiert.
Schmp. 257 - 260°C

Beispiel 3.26

4-(2,6-Dinitro-4-trifluormethyl-phenoxy)-5-methyl-2-perfluorethyl-pyrimidin

Zu einer Suspension von 3,3 g $K_2CO_3$ in 50 ml Acetonitril tropfte man nacheinander eine Lösung von 4,5 g (0,16 mol) 4-Chlor-3,5-dinitro-benzotrifluorid und 3,8 g (0,16 mol) 5-Methyl-2-perfluorethyl-4-pyrimidinol. Man erhitzte das Gemisch 3h zum Rückfluß, saugte den Niederschlag ab und dampfte die Lösung im Vakuum ein. Der Rückstand wurde aus Diisopropylether umkristallisiert.
Schmp. 90 - 92°C

Beispiel 4.167

N'-(2,6-Dinitro-4-trifluormethyl-phenyl)-5-methyl-2-perfluorethyl-4-pyrimidinyl-hydrazin

Zu einer Lösung von 2,7 g (0,01 mol) 4-Chlor-3,5-dinitrobenzotrifluorid in 80 ml EtOH wurden 4,84 g (0,02 mol) 5-Methyl-2-perfluorethyl-4-pyrimidinylhydrazin, gelöst in 30 ml EtOH, innerhalb von 30 Minuten hinzugetropft. Man ließ dann 5h am Rückfluß kochen und dampfte anschließend das Lösungsmittel im Vakuum ein. Der Rückstand wurde in Wasser aufgenommen und mit Chloroform extrahiert. Die organische Phase wurde mit $Na_2SO_4$ getrocknet und im Vakuum eingedampft. Der anfallende Feststoff wurde aus Diisopropylether umkristallisiert.
Schmp. 135 - 137°

Beispiel 5.16

N'-(2,6-Dinitro-4-trifluormethyl-phenyl)-6,N-dimethyl2-trichlormethyl-4-pyrimidinyl-hydrazin

Zu einer Lösung von 2,7 g (0,01 mol) 4-Chlor-3,5-dinitrobenzotrifluorid in EtOH tropfte man 5,1 g (0,02 mol) N-Methyl-N-(6-methyl-2-trichlormethyl-4-pyrimidinyl)-hydrazin, gelöst in 30 ml EtOH innerhalb von 30 Minuten hinzu. Man ließ das Gemisch 5 Stunden am Rückfluß kochen und dampfte anschließend zur Trockne ein. Der Rückstand wurde in Chloroform gelöst, mit Wasser gewaschen, über $Na_2SO_4$ getrocknet und im Vakuum eingedampft. Der ausgefallene Feststoff wurde aus Diisopropylether umkristallisiert.
Schmp. 160 - 163°C

Analog Beispiel 1 lassen sich die Verbindungen der Tabelle 1, analog Beispiel 2 die Verbindungen der Tabelle 2, analog Beispiel 3 der Verbindungen der Tabelle 3 analog beispiel 4 die Verbindungen der Tabelle 4 und analog Beispiel 5 die Verbindungen der Tabelle 5 herstellen.

Tabelle 1

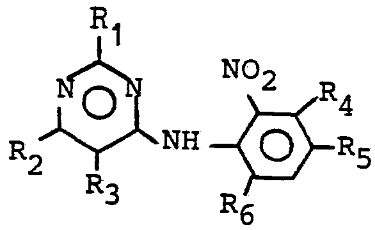

| Nr. | $R_1$ | $R_2$ | $R_3$ | $R_4$ | $R_5$ | $R_6$ | physik. Konstanten [°C] |
|---|---|---|---|---|---|---|---|
| 1.1 | $CCl_3$ | H | H | H | C≡N | $NO_2$ | Smp. 157-160 |
| 1.2 | $CCl_3$ | H | H | H | $CO_2Et$ | $NO_2$ | Smp. 121-124 |
| 1.3 | $CCl_3$ | H | H | H | $CH_3$ | $NO_2$ | Smp. 132-134 |
| 1.4 | $CCl_3$ | H | H | Cl | $CO_2Et$ | $NO_2$ | Sirup |
| 1.5 | $CCl_3$ | H | H | H | $CCl_3$ | $NO_2$ | |
| 1.6 | $CCl_3$ | $CH_3$ | H | H | $CCl_3$ | $NO_2$ | |
| 1.7 | $CCl_3$ | $CH_3$ | H | Cl | $CCl_3$ | $NO_2$ | |
| 1.8 | $CCl_3$ | $CH_3$ | H | H | C≡N | $NO_2$ | Smp. 180-183 |
| 1.9 | $CCl_3$ | $CH_3$ | H | Cl | C≡N | $NO_2$ | |
| 1.10 | $CCl_3$ | $CH_3$ | H | H | $CO_2Et$ | $NO_2$ | Smp. 111-114 |
| 1.11 | $CCl_3$ | $CH_3$ | H | Cl | $CO_2Et$ | $NO_2$ | |
| 1.12 | $CCl_3$ | $CH_3$ | H | H | $CH_3$ | $NO_2$ | Smp. 111-115 |
| 1.13 | $CCl_3$ | $CH_3$ | H | Cl | $CH_3$ | $NO_2$ | |
| 1.14 | $CCl_3$ | $CH_3$ | H | H | 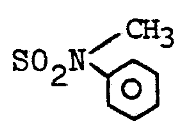 | $NO_2$ | |
| 1.15 | $CCl_3$ | $CH_3$ | H | Cl | 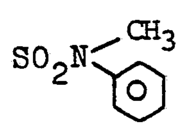 | $NO_2$ | |
| 1.16 | $CH_3$ | H | H | H | C≡N | $NO_2$ | |
| 1.17 | $CH_3$ | H | H | H | $CCl_3$ | $NO_2$ | |
| 1.18 | $CH_3$ | H | H | H | $CH_3$ | $NO_2$ | |
| 1.19 | $CH_3$ | H | H | H | $CO_2Et$ | $NO_2$ | |
| 1.20 | $CF_2CHF_2$ | H | H | H | $CCl_3$ | $NO_2$ | |
| 1.21 | $CF_2CHF_2$ | H | H | Cl | $CCl_3$ | $NO_2$ | |
| 1.22 | $CF_2CHF_2$ | H | H | H | C≡N | $NO_2$ | Smp. 168-170 |
| 1.23 | $CF_2CHF_2$ | H | H | H | $CO_2Et$ | $NO_2$ | Smp. 156-157 |
| 1.24 | $CF_2CHF_2$ | H | H | Cl | $CO_2Et$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.25 | $CF_2CHF_2$ | H | H | H | $CH_3$ | $NO_2$ | |
| 1.26 | $CF_3$ | H | H | H | C≡N | $NO_2$ | |
| 1.27 | $CF_3$ | H | H | H | $CO_2Et$ | $NO_2$ | |
| 1.28 | $CF_3$ | H | H | H | $CCl_3$ | $NO_2$ | |
| 1.29 | $CF_3$ | H | H | H | $CH_3$ | $NO_2$ | |
| 1.30 | $CF_2CHF_2$ | $CH_3$ | H | H | $CO_2Et$ | $NO_2$ | |
| 1.31 | $CCl_3$ | H | Cl | H | $CO_2Et$ | $NO_2$ | Smp. 127-131 |
| 1.32 | $CCl_3$ | H | Cl | H | C≡N | $NO_2$ | Smp.214-215 |
| 1.33 | $CCl_3$ | H | Cl | Cl | C≡N | $NO_2$ | |
| 1.34 | $CCl_3$ | H | Cl | H | $CCl_3$ | $NO_2$ | |
| 1.35 | $CCl_3$ | H | Cl | Cl | $CCl_3$ | $NO_2$ | |
| 1.36 | $CCl_3$ | H | Cl | H | $CH_3$ | $NO_2$ | |
| 1.37 | $CCl_3$ | H | Cl | Cl | $CH_3$ | $NO_2$ | |
| 1.38 | $CF_2CHF_2$ | $CH_3$ | H | H | $CO_2CH_3$ | $NO_2$ | Smp. 167-131 |
| 1.39 | $CCl_3$ | H | Cl | Cl | $CO_2Et$ | $NO_2$ | |
| 1.40 | $CF_3$ | H | Cl | H | $CCl_3$ | $NO_2$ | |
| 1.41 | $CF_3$ | H | Cl | Cl | $CFCl_2$ | $NO_2$ | |
| 1.42 | $CF_3$ | H | Cl | H | $CF_2CHF_2$ | $NO_2$ | |
| 1.43 | $CF_3$ | H | Cl | F | $CF_2CClF_2$ | $NO_2$ | |
| 1.44 | $CF_2CHF_2$ | H | Cl | H | $CCl_3$ | $NO_2$ | Smp. 67-68 |
| 1.45 | $CF_2CHF_2$ | H | Cl | Cl | $CCl_3$ | $NO_2$ | |
| 1.46 | $CF_2CHF_2$ | H | Cl | H | C≡N | $NO_2$ | Smp. 193-196 |
| 1.47 | $CF_2CHF_2$ | H | Cl | Cl | C≡N | $NO_2$ | |
| 1.48 | $CF_2CHF_2$ | H | Cl | H | $CO_2Et$ | $NO_2$ | Smp. 109-110 |
| 1.49 | $CF_2CHF_2$ | H | Cl | Cl | $CO_2Et$ | $NO_2$ | Smp. 115-118 |
| 1.50 | $CF_2CHF_2$ | H | Cl | H | $CClF_2$ | $CF_3$ | |
| 1.51 | $CF_2CHF_2$ | H | Cl | Cl | $CClF_2$ | $CF_3$ | |
| 1.52 | $CF_2CHF_2$ | H | Cl | Cl | 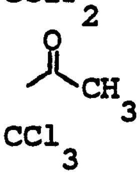 | $NO_2$ | |
| 1.53 | $CCl_3$ | Cl | Cl | H | $CCl_3$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.54 | $CCl_3$ | Cl | Cl | Cl | $CO_2Et$ | $NO_2$ | |
| 1.55 | $CCl_3$ | Cl | Cl | H | C≡N | $NO_2$ | |
| 1.56 | $CCl_3$ | Cl | Cl | Cl | $CH_3$ | $NO_2$ | |
| 1.57 | $CCl_3$ | $CH_3$ | Cl | H | $CCl_3$ | $NO_2$ | |
| 1.58 | $CCl_3$ | $CH_3$ | Cl | F | $CCl_3$ | $NO_2$ | |
| 1.59 | $CCl_3$ | $CH_3$ | Cl | Cl | C≡N | $NO_2$ | |
| 1.60 | $CCl_3$ | $CH_3$ | Cl | H | C≡N | $NO_2$ | Smp. 198-201 |
| 1.61 | $CF_2CHF_2$ | Cl | Cl | H | $CH_3$ | $NO_2$ | |
| 1.62 | $CF_2CHF_2$ | Cl | Cl | Cl | C≡N | $NO_2$ | |
| 1.63 | $CH_3$ | $CH_3$ | Cl | H | C≡N | $CF_3$ | |
| 1.64 | $CH_3$ | $CH_3$ | Cl | Cl | C≡N | $CF_3$ | |
| 1.65 | C≡N | H | Cl | H | C≡N | $NO_2$ | |
| 1.66 | C≡N | H | Cl | H | $CO_2Et$ | $NO_2$ | |
| 1.67 | C≡N | H | Cl | H | $CCl_3$ | $NO_2$ | |
| 1.68 | C≡N | H | Cl | H | $CH_3$ | $NO_2$ | |
| 1.69 | $CF_2CF_3$ | H | Cl | H | C≡N | $NO_2$ | |
| 1.70 | $CF_2CF_3$ | $CH_3$ | Cl | Cl | C≡N | $NO_2$ | |
| 1.71 | $CF_2CF_3$ | $CH_3$ | Cl | H | 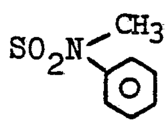 | $NO_2$ | |
| 1.72 | $CF_2CF_3$ | $ClCH_2$ | Cl | H | $CCl_3$ | $NO_2$ | |
| 1.73 | $CF_2Cl$ | H | Cl | Cl | $CCl_3$ | $NO_2$ | |
| 1.74 | $CF_2Cl$ | H | Cl | H | $CH_3$ | $NO_2$ | |
| 1.75 | $CClF_2$ | H | Cl | H | $CH_3$ | $NO_2$ | |
| 1.76 | $CClF_2$ | $CH_3$ | Cl | Cl | Et | $NO_2$ | |
| 1.77 | $CH_3$ | H | Cl | H | $CF_2Cl$ | $NO_2$ | |
| 1.78 | $CH_3$ | H | Cl | Cl | C≡N | $NO_2$ | |
| 1.79 | $CH_3$ | Cl | Cl | Cl | $CO_2Et$ | $NO_2$ | |
| 1.80 | Et | H | Cl | Cl | C≡N | $NO_2$ | |
| 1.81 | Et | H | Cl | H | C≡N | $NO_2$ | |
| 1.82 | 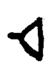 | H | Cl | H | $CCl_3$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.83 | 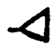 | H | Cl | H | C≡N | $NO_2$ | |
| 1.84 | OEt | Cl | Cl | F | $CCl_2CCl_3$ | $CF_3$ | |
| 1.85 | OEt | OEt | Cl | F | $CCl_2CCl_3$ | $CF_3$ | |
| 1.86 | $SCH_3$ | $SCH_3$ | Cl | F | $CCl{=}CCl_2$ | $NO_2$ | |
| 1.87 | 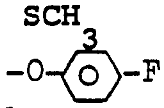 | Cl | Cl | H | $CF_2CHF_2$ | $NO_2$ | |
| 1.88 | 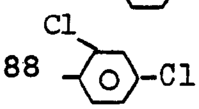 | H | Cl | H | $CO_2Et$ | $NO_2$ | |
| 1.89 | Cl | Cl | Cl | Cl | $CCl_3$ | $NO_2$ | |
| 1.90 | F | H | Cl | Cl | $CCl_3$ | $NO_2$ | |
| 1.91 | $N(CH_3)_2$ | $CCl_3$ | Cl | H | $CHCl_2$ | $NO_2$ | |
| 1.92 | $CCl_3$ | H | Br | H | C≡N | $NO_2$ | Smp. 220-22 |
| 1.93 | $CCl_3$ | H | Br | H | $CCl_3$ | $NO_2$ | |
| 1.94 | $CCl_3$ | H | Br | Cl | $CO_2Et$ | $NO_2$ | |
| 1.95 | $CCl_3$ | H | Br | H | $CH_3$ | $NO_2$ | Smp. 153-154 |
| 1.96 | $CCl_3$ | H | Br | Cl | $CCl_3$ | $NO_2$ | |
| 1.97 | $CF_2CHF_2$ | H | Br | H | $CH_3$ | $NO_2$ | |
| 1.98 | $CF_2CHF_2$ | H | Br | H | $CF_2Cl$ | $NO_2$ | |
| 1.99 | $CF_2CF_3$ | H | Br | Cl | $C_2H_5$ | $NO_2$ | |
| 1.100 | $CF_2CF_3$ | H | Br | H | $CF_2CF_3$ | $NO_2$ | |
| 1.101 | $CH_3$ | H | Br | H | $CO_2Et$ | $NO_2$ | Smp. 122-125 |
| 1.102 | $CH_3$ | H | Br | Cl | $CCl_3$ | $NO_2$ | |
| 1.103 | $CH_3$ | H | Br | H | C≡N | $NO_2$ | |
| 1.104 | 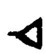 | H | Br | H | C≡N | $NO_2$ | |
| 1.105 | $C_2H_5$ | H | Br | Cl | C≡N | $NO_2$ | |
| 1.106 | $C_3F_7$ | H | Br | F | $CO_2Et$ | $NO_2$ | |
| 1.107 | $C_3F_7$ | H | Br | F | C≡N | $NO_2$ | |
| 1.108 | C≡N | H | Br | J | $CCl_3$ | $NO_2$ | |
| 1.109 | $CCl_3$ | H | C≡N | H | $CO_2Et$ | $NO_2$ | |
| 1.110 | $CCl_3$ | H | C≡N | Cl | $CO_2Et$ | $NO_2$ | |
| 1.111 | $CCl_3$ | H | C≡N | H | C≡N | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.112 | $CCl_3$ | H | C≡N | H | $CCl_3$ | $NO_2$ | |
| 1.113 | $CCl_3$ | H | C≡N | H | $CH_3$ | $NO_2$ | |
| 1.114 | $CH_3$ | H | C≡N | H | C≡N | $NO_2$ | |
| 1.115 | $CH_3$ | H | C≡N | H | $CO_2Et$ | $NO_2$ | |
| 1.116 | $CH_3$ | H | C≡N | H | $CCl_3$ | $NO_2$ | |
| 1.117 | $CH_3$ | H | C≡N | H | $CH_3$ | $NO_2$ | |
| 1.118 | $CH_3$ | H | C≡N | Cl | $CO_2Et$ | $NO_2$ | |
| 1.119 | $CH_3$ | H | C≡N | Cl | $CCl_3$ | $NO_2$ | |
| 1.120 | 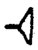 | H | C≡N | H | $CCl_3$ | $NO_2$ | |
| 1.121 | 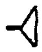 | H | C≡N | Cl | $CCl_3$ | $NO_2$ | |
| 1.122 | 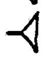 | H | C≡N | H | $CO_2Et$ | $NO_2$ | |
| 1.123 | 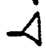 | H | C≡N | Cl | $CO_2Et$ | $NO_2$ | |
| 1.124 | 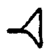 | H | C≡N | H | $CH_3$ | $NO_2$ | |
| 1.125 | $CF_2CHF_2$ | H | C≡N | H | C≡N | $NO_2$ | Smp. 188-190 |
| 1.126 | $CF_2CHF_2$ | H | C≡N | H | $CO_2Et$ | $NO_2$ | |
| 1.127 | $CF_2CHF_2$ | H | C≡N | H | $CO_2H$ | $CF_3$ | |
| 1.128 | $CF_2CHF_2$ | H | C≡N | Cl | $CO_2H$ | $NO_2$ | |
| 1.129 | $CF_2CHF_2$ | H | C≡N | H | $CCl_3$ | $NO_2$ | |
| 1.130 | $CF_2CHF_2$ | H | C≡N | H | $CH_3$ | $NO_2$ | |
| 1.131 | Cl | H | C≡N | H | $CCl_3$ | $NO_2$ | |
| 1.132 | $CCl_3$ | H | $CH_3$ | H | C≡N | $NO_2$ | |
| 1.133 | $CCl_3$ | H | $CH_3$ | Cl | $CCl_3$ | $NO_2$ | |
| 1.134 | $CCl_3$ | H | $CH_3$ | H | $CCl_3$ | $NO_2$ | |
| 1.135 | $CCl_3$ | H | $CH_3$ | H | $CO_2Et$ | $NO_2$ | |
| 1.136 | $CCl_3$ | H | $CH_3$ | H | $CH_3$ | $NO_2$ | |
| 1.137 | $CF_3$ | H | $CH_3$ | H | C≡N | $NO_2$ | |
| 1.138 | $CF_3$ | H | $CH_3$ | H | $CO_2Et$ | $NO_2$ | |
| 1.139 | $CF_2CHF_2$ | H | $CH_3$ | H | C≡N | $NO_2$ | Smp. 178-179 |
| 1.140 | $CF_2CHF_2$ | H | $CH_3$ | H | $CO_2Et$ | $NO_2$ | Smp. 82-83 |
| 1.141 | $CF_2CHF_2$ | H | $CH_3$ | H | $CCl_3$ | $NO_2$ | |
| 1.142 | $CF_2CHF_2$ | H | $CH_3$ | H | $CH_3$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.143 | $CF_2CF_3$ | H | $CH_3$ | H | C≡N | $NO_2$ | |
| 1.144 | $CF_2CF_3$ | H | $CH_3$ | H | $CO_2Et$ | $NO_2$ | |
| 1.145 | $CH_3$ | H | $CH_3$ | H | $CCl_3$ | $NO_2$ | |
| 1.146 | Et | H | $CH_3$ | H | C≡N | $NO_2$ | |
| 1.147 | C≡N | H | $CH_3$ | H | $CCl_3$ | $NO_2$ | |
| 1.148 | C≡N | H | $CH_3$ | H | $CO_2Et$ | $NO_2$ | |
| 1.149 | $OCH_3$ | $CH_3$ | $CH_3$ | H | $CCl_3$ | $NO_2$ | |
| 1.150 | OEt | $CH_3$ | $CH_3$ | Cl | C≡N | $NO_2$ | |
| 1.151 | $SCH_3$ | $CH_3$ | $CH_3$ | Cl | C≡N | $NO_2$ | |
| 1.152 | 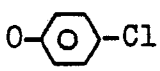 | $CH_3$ | $CH_3$ | Cl | C≡N | $NO_2$ | |
| 1.153 | $CCl_3$ | H | F | H | C≡N | $NO_2$ | |
| 1.154 | $CF_2CHF_2$ | H | F | Cl | $CO_2CH_3$ | $NO_2$ | |
| 1.155 | $CHCl_2$ | H | $OCH_3$ | F | $CHF_2$ | $NO_2$ | |
| 1.156 | $CF_3$ | H | $CF_3$ | H | $CCl_3$ | $NO_2$ | |
| 1.157 | $CCl=CCl_2$ | H | $CCl_3$ | H | $CH_3$ | $NO_2$ | |
| 1.158 | $CF=CF_2$ | H | $CF=CF_2$ | H | 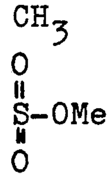 | $NO_2$ | |
| 1.159 | $CH_3$ | $CH_3$ | $CH_2Cl$ | H | C≡N | $NO_2$ | |
| 1.160 | $CH_3$ | H | 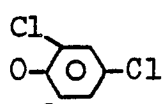 | H | C≡N | $NO_2$ | |
| 1.161 | $CH_3$ | H | 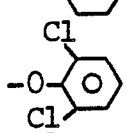 | Cl | $CO_2Et$ | $NO_2$ | |
| 1.162 | $CH_3$ | H | 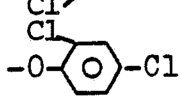 | H | $CO_2Me$ | $NO_2$ | |
| 1.163 | $CCl_3$ | H | 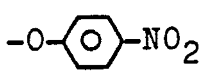 | H | $CO_2Bu$ | $NO_2$ | |
| 1.164 | $CCl_3$ | H | 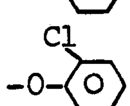 | Cl | $CO_2Et$ | $NO_2$ | |
| 1.165 | $CCl_3$ | H | 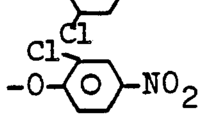 | H | $CCl_3$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.166 | $CF_2CHF_2$ | H | Cl | H | $CO_2H$ | $NO_2$ | Smp. 244-247 |
| 1.167 | $CCl_3$ | H | Cl | H | $CO_2H$ | $NO_2$ | |
| 1.168 | $CCl_3$ | H | CN | H | $CO_2H$ | $NO_2$ | |
| 1.169 | $CF_2CHF_2$ | H | CN | H | $CO_2H$ | $NO_2$ | |
| 1.170 | $CF_2CHF_2$ | H | Br | H | $CO_2H$ | $NO_2$ | |
| 1.171 | $CF_2CHF_2$ | H | $CH_3$ | H | $CO_2H$ | $NO_2$ | |
| 1.172 | $CCl_3$ | H | Br | H | $CO_2H$ | $NO_2$ | |
| 1.173 | $CF_3$ | H | Cl | H | $CO_2H$ | $NO_2$ | |
| 1.174 | $CF_3$ | H | Br | H | $CO_2H$ | $NO_2$ | |
| 1.175 | $CF_3$ | H | CN | H | $CO_2H$ | $NO_2$ | |
| 1.176 | $CF_2Cl$ | H | Cl | H | $CO_2H$ | $NO_2$ | |
| 1.177 | $CF_2Cl$ | H | Br | H | $CO_2H$ | $NO_2$ | |
| 1.178 | $CF_2Cl$ | H | C≡N | H | $CO_2H$ | $NO_2$ | |
| 1.179 | $CF_3$ | H | Cl | H | C≡N | $NO_2$ | Smp. 230-232 |
| 1.180 | $F_2C{=}CF$ | H | Br | Cl | $CO_2Bu$ | $NO_2$ | |
| 1.181 | $Cl_2C{=}CCl$ | H | Cl | H | $CF_2Cl$ | $NO_2$ | |
| 1.182 | $F_2C{=}CCl$ | Cl | Cl | H | $CClCF_2$ | $NO_2$ | |
| 1.183 | C≡N | H | Cl | H | $CO_2H$ | $NO_2$ | |
| 1.184 | $CCl_3$ | H | Cl | OEt | C≡N | $NO_2$ | |
| 1.185 | $CClF_2$ | Cl | H | 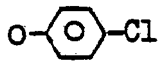 | $CO_2Pr$ | $NO_2$ | |
| 1.186 | $CH_2CH_3$ | F | H | $N(CH_3)_2$ | $CH_3$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.187 | $CF_2Cl$ | H | H | H | $CO_2Et$ | $NO_2$ | |
| 1.188 | $CF_2Cl$ | H | H | H | C≡N | $NO_2$ | |
| 1.189 | $CF_2Cl$ | H | Cl | H | C≡N | $NO_2$ | Smp. 250-252 |
| 1.190 | $CF_2Cl$ | H | Cl | H | $CO_2Et$ | $NO_2$ | Smp. 117-118 |
| 1.191 | $CF_2Cl$ | H | Br | H | C≡N | $NO_2$ | Smp. 254-256 |
| 1.192 | $CF_2Cl$ | H | Br | H | $CO_2Et$ | $NO_2$ | Smp. 136-137 |
| 1.193 | $CF_2Cl$ | H | Br | H | Cl | $NO_2$ | |
| 1.194 | $CF_2Cl$ | H | C≡N | H | C≡N | $NO_2$ | Smp. 217-219 |
| 1.195 | $CF_2Cl$ | H | C≡N | H | $CO_2Et$ | $NO_2$ | Smp. 169-170 |
| 1.196 | $CF_2CHClF$ | H | Cl | H | C≡N | $NO_2$ | Smp. 187-188 |
| 1.197 | $CF_2CHClF$ | H | Cl | H | $CO_2Et$ | $NO_2$ | Smp. 100-101 |
| 1.198 | $n-C_3F_7$ | H | H | H | C≡N | $NO_2$ | |
| 1.199 | $n-C_3F_7$ | H | H | H | $CO_2Et$ | $NO_2$ | |
| 1.200 | $n-C_3F_7$ | H | Cl | H | $CO_2Et$ | $NO_2$ | Smp. 114-115 |
| 1.201 | $n-C_3F_7$ | H | Cl | H | C≡N | $NO_2$ | Smp. 124-126 |
| 1.202 | $n-C_3F_7$ | H | Br | H | C≡N | $NO_2$ | |
| 1.203 | $n-C_3F_7$ | H | Br | H | $CO_2Et$ | $NO_2$ | |
| 1.204 | $CF_3$ | H | Cl | H | C≡N | $NO_2$ | Smp. 231-232 |
| 1.205 | $CF_2CHF_2$ | H | H | Cl | C≡N | $NO_2$ | |
| 1.206 | $CF_2CHF_2$ | H | H | H | $CO_2CH_3$ | $NO_2$ | |
| 1.207 | $CF_2CHF_2$ | H | H | H | $CO_2Bu$ | $NO_2$ | |
| 1.208 | $CF_2CHF_2$ | H | H | H | $CON(CH_3)_2$ | $NO_2$ | Smp. 241-243 |
| 1.209 | $CF_2CHF_2$ | H | Cl | H | Cl | $NO_2$ | Smp. 110-112 |
| 1.210 | $CF_2CHF_2$ | H | Cl | H | $CO_2H$ | $NO_2$ | Smp. 247-249 |
| 1.211 | $CF_2CHF_2$ | H | Cl | H | $CO_2Bu$ | $NO_2$ | Smp. 94-97 |
| 1.212 | $CF_2CHF_2$ | H | Cl | H | $CH_3$ | $NO_2$ | |
| 1.213 | $CF_2CHF_2$ | H | Cl | H | $CO_2CH_3$ | $NO_2$ | Smp. 110-112 |
| 1.214 | $CF_2CHF_2$ | H | Br | H | $CO_2Et$ | $NO_2$ | Smp. 126-128 |
| 1.215 | $CF_2CHF_2$ | H | Br | H | C≡N | $NO_2$ | Smp. 211-213 |
| 1.216 | $CF_2CHF_2$ | H | Br | Cl | $CO_2Et$ | $NO_2$ | |
| 1.217 | $CF_2CHF_2$ | H | Br | Cl | C≡N | $NO_2$ | |
| 1.218 | $CF_2CHF_2$ | H | Br | H | $CO_2CH_3$ | $NO_2$ | |
| 1.219 | $CF_2CHF_2$ | H | Br | H | $CO_2Bu$ | $NO_2$ | |
| 1.220 | $CF_2CHF_2$ | H | $CH_3$ | Cl | $CO_2Et$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.221 | $CF_2CHF_2$ | H | $CH_3$ | Cl | C≡N | $NO_2$ | |
| 1.222 | $CF_2CHF_2$ | Cl | H | H | C≡N | $NO_2$ | Smp. 112-114 |
| 1.223 | $CF_2CHF_2$ | Cl | H | H | $CO_2Et$ | $NO_2$ | Smp. 90-92 |
| 1.224 | $CF_2CHF_2$ | Cl | H | H | $CH_3$ | $NO_2$ | |
| 1.225 | $CF_2CHF_2$ | Cl | H | H | Cl | $NO_2$ | |
| 1.226 | $CF_2CF_3$ | H | H | H | C≡N | $NO_2$ | |
| 1.227 | $CF_2CF_3$ | H | H | Cl | C≡N | $NO_2$ | |
| 1.228 | $CF_2CF_3$ | H | H | H | $CO_2Et$ | $NO_2$ | |
| 1.229 | $CF_2CF_3$ | H | H | Cl | $CO_2Et$ | $NO_2$ | |
| 1.230 | $CF_2CF_3$ | H | H | H | $CO_2CH_3$ | $NO_2$ | |
| 1.231 | $CF_2CF_3$ | H | H | H | $CO_2Bu$ | $NO_2$ | |
| 1.232 | $CF_2CF_3$ | H | H | H | $CH_3$ | $NO_2$ | |
| 1.233 | $CF_2CF_3$ | H | Cl | H | C≡N | $NO_2$ | |
| 1.234 | $CF_2CF_3$ | H | Cl | H | $CO_2Et$ | $NO_2$ | |
| 1.235 | $CF_2CF_3$ | H | Cl | Cl | C≡N | $NO_2$ | |
| 1.236 | $CF_2CF_3$ | H | Cl | Cl | $CO_2Et$ | $NO_2$ | |
| 1.237 | $CF_2CF_3$ | H | Cl | H | $CO_2CH_3$ | $NO_2$ | |
| 1.238 | $CF_2CF_3$ | H | Cl | H | $CO_2Bu$ | $NO_2$ | |
| 1.239 | $CF_2CF_3$ | H | Cl | H | $CH_3$ | $NO_2$ | |
| 1.240 | $CF_2CF_3$ | H | Cl | H | Cl | $NO_2$ | |
| 1.241 | $CF_2CF_3$ | H | H | H | Cl | $NO_2$ | |
| 1.242 | $CF_2CF_3$ | H | Br | H | $CO_2Et$ | $NO_2$ | |
| 1.243 | $CF_2CF_3$ | H | Br | H | $CO_2CH_3$ | $NO_2$ | |
| 1.244 | $CF_2CF_3$ | H | Br | H | C≡N | $NO_2$ | |
| 1.245 | $CF_2CF_3$ | H | Br | Cl | $CO_2Et$ | $NO_2$ | |
| 1.246 | $CF_2CF_3$ | H | Br | H | $CON(Et)_2$ | $NO_2$ | |
| 1.247 | $CF_2CF_3$ | H | Br | H | $CON(CH_3)_2$ | $NO_2$ | |
| 1.248 | $CF_2CF_3$ | H | Br | H | Cl | $NO_2$ | |
| 1.249 | $CCl_3$ | H | Cl | H | $CON(Et)_2$ | $NO_2$ | |
| 1.250 | $CCl_3$ | H | Br | H | $CO_2Et$ | $NO_2$ | Smp. 126-127 |
| 1.251 | $CCl_3$ | H | Br | H | Cl | $NO_2$ | Smp. 146-49 |
| 1.252 | $CCl_3$ | H | Br | Cl | C≡N | $NO_2$ | |
| 1.253 | $CCl_3$ | H | $CH_3$ | Cl | $CO_2Et$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.254 | $CCl_3$ | H | $CH_3$ | Cl | C≡N | $NO_2$ | |
| 1.255 | $CCl_3$ | $CH_3$ | H | Cl | $CO_2H$ | $NO_2$ | Smp. 240-243 |
| 1.256 | $CCl_3$ | $CH_3$ | H | H | $CO_2CH_3$ | $NO_2$ | Smp. 104-105 |
| 1.257 | $CCl_3$ | $CH_3$ | H | H | $CONH_2$ | $NO_2$ | |
| 1.258 | $CCl_3$ | Cl | Cl | H | $CO_2Et$ | $NO_2$ | |
| 1.259 | $CCl_3$ | Cl | Cl | Cl | C≡N | $NO_2$ | |
| 1.260 | $CCl_3$ | $CH_3$ | Cl | H | $CO_2Et$ | $NO_2$ | Smp. 145-146 |
| 1.261 | $CCl_3$ | $CH_3$ | Cl | Cl | $CO_2Et$ | $NO_2$ | |
| 1.262 | $CCl_3$ | $CH_3$ | Cl | H | Cl | $NO_2$ | |
| 1.263 | $CCl_3$ | Cl | H | H | $CO_2Et$ | $NO_2$ | |
| 1.264 | $CCl_3$ | Cl | H | H | C≡N | $NO_2$ | |
| 1.265 | $CCl_3$ | Cl | H | H | Cl | $NO_2$ | |
| 1.266 | $CCl_3$ | Cl | H | H | $CON(Et)_2$ | $NO_2$ | |
| 1.267 | $CCl_3$ | Cl | Br | H | $CO_2Et$ | $NO_2$ | Smp. 205-207 |
| 1.268 | $CCl_3$ | Cl | Br | H | C≡N | $NO_2$ | Smp. 200-202 |
| 1.269 | $CH_3$ | $OCH_3$ | $CF_3$ | H | $CO_2Et$ | $NO_2$ | Smp. 151-155 |
| 1.270 | $CH_3$ | $OCH_3$ | $CF_3$ | H | C≡N | $NO_2$ | Smp. 142-146 |
| 1.271 | $CH_3$ | $OCH_3$ | $CF_3$ | Cl | $CO_2Et$ | $NO_2$ | |
| 1.272 | $CH_3$ | $OCH_3$ | $CF_3$ | H | Cl | $NO_2$ | |
| 1.273 | 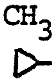 | H | H | H | $CO_2Et$ | $NO_2$ | |
| 1.274 | 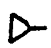 | H | H | Cl | C≡N | $NO_2$ | |
| 1.275 | 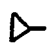 | H | H | H | Cl | $NO_2$ | |
| 1.276 | 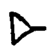 | H | Cl | H | $CO_2Et$ | $NO_2$ | |
| 1.277 | 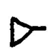 | H | Cl | H | C≡N | $NO_2$ | |
| 1.278 | 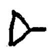 | H | Cl | H | Cl | $NO_2$ | |
| 1.279 | 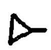 | H | Br | Cl | C≡N | $NO_2$ | |
| 1.280 | 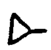 | H | Br | H | $CO_2Et$ | $NO_2$ | |
| 1.281 | 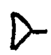 | H | Br | H | $CO_2CH_3$ | $NO_2$ | |
| 1.282 | 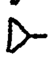 | H | Br | H | Cl | $NO_2$ | |
| 1.283 | 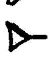 | H | Br | H | $CH_3$ | $NO_2$ | |
| 1.284 | $CCl_3$ | H | H | H | $CO_2Bu$ | $NO_2$ | Smp. 57-63 |
| 1.285 | $CCl_3$ | H | H | Cl | C≡N | $NO_2$ | |
| 1.286 | $CCl_3$ | H | H | H | $CO_2CH_3$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.287 | $CCl_3$ | H | H | H | $CH_3$ | $NO_2$ | |
| 1.288 | $CCl_3$ | H | H | H | $CON(Et)_2$ | $NO_2$ | |
| 1.289 | $CCl_3$ | H | Cl | H | Cl | $NO_2$ | Smp. 145-147 |
| 1.290 | $CCl_3$ | H | Cl | H | $CO_2CH_3$ | $NO_2$ | Smp. 142-144 |
| 1.291 | $CCl_3$ | H | Cl | H | $CO_2Bu$ | $NO_2$ | Smp. 127-128 |
| 1.292 | $CH_3$ | H | C≡N | H | Cl | $NO_2$ | |
| 1.293 | $CH_3$ | H | C≡N | H | $CON(Et)_2$ | $NO_2$ | |
| 1.294 | $CH_3$ | H | C≡N | Cl | $CO_2Bu$ | $NO_2$ | |
| 1.295 | $CH_3$ | H | C≡N | H | $CON(CH_3)_2$ | $NO_2$ | |
| 1.296 | $CH_3$ | H | C≡N | Cl | C≡N | $NO_2$ | |
| 1.297 | $CH_3$ | H | C≡N | H | $CO_2CH_3$ | $NO_2$ | |
| 1.298 | $CH_3$ | H | Cl | H | $CO_2Et$ | $NO_2$ | Smp. 159-162 |
| 1.299 | $CH_3$ | H | Cl | Cl | C≡N | $NO_2$ | |
| 1.300 | $CH_3$ | H | Cl | H | Cl | $NO_2$ | |
| 1.301 | $CH_3$ | H | Cl | Cl | $CO_2Et$ | $NO_2$ | |
| 1.302 | $CH_3$ | H | Cl | H | Cl | $NO_2$ | |
| 1.303 | $CH_3$ | H | Cl | H | $CO_2CH_3$ | $NO_2$ | Smp. 159-162 |
| 1.304 | $CH_3$ | H | Br | H | $CH_3$ | $NO_2$ | |
| 1.305 | $CH_3$ | H | Br | Cl | C≡N | $NO_2$ | |
| 1.306 | $CH_3$ | H | Br | H | $CONH_2$ | $NO_2$ | |
| 1.307 | $CH_3$ | H | Br | H | $CON(CH_3)_2$ | $NO_2$ | |
| 1.308 | $CH_3$ | H | Br | H | Cl | $NO_2$ | |
| 1.309 | $CH_3$ | H | Br | H | $CO_2Et$ | $NO_2$ | |
| 1.310 | $CH_3$ | H | Br | Cl | $CO_2Et$ | $NO_2$ | |
| 1.311 | $CH_3$ | H | H | H | $CH_3$ | $NO_2$ | |
| 1.312 | $CH_3$ | H | H | Cl | $CO_2Et$ | $NO_2$ | |
| 1.313 | $CH_3$ | H | H | H | Cl | $NO_2$ | |
| 1.314 | $CH_3$ | H | H | Cl | C≡N | $NO_2$ | |
| 1.315 | $CCl_3$ | H | H | H | $CHF_2$ | $NO_2$ | |
| 1.316 | $CCl_3$ | $CH_3$ | H | H | $CHF_2$ | $NO_2$ | |
| 1.317 | $CCl_3$ | H | Cl | H | $CHF_2$ | $NO_2$ | |
| 1.318 | $CCl_3$ | H | Br | H | $CHF_2$ | $NO_2$ | |
| 1.319 | $CCl_3$ | H | F | H | $CHF_2$ | $NO_2$ | |
| 1.320 | $CCl_3$ | H | $CH_3$ | H | $CHF_2$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.321 | $CCl_3$ | H | C≡N | H | $CHF_2$ | $NO_2$ | |
| 1.322 | $CF_2CHF_2$ | H | H | H | $CHF_2$ | $NO_2$ | |
| 1.323 | $CF_2CHF_2$ | $CH_3$ | H | H | $CHF_2$ | $NO_2$ | |
| 1.324 | $CF_2CHF_2$ | H | Cl | H | $CHF_2$ | $NO_2$ | |
| 1.325 | $CF_2CHF_2$ | H | Br | H | $CHF_2$ | $NO_2$ | |
| 1.326 | $CF_2CHF_2$ | H | F | H | $CHF_2$ | $NO_2$ | |
| 1.327 | $CF_2CHF_2$ | $CH_3$ | H | H | $CHF_2$ | $NO_2$ | |
| 1.328 | $CF_2CHF_2$ | $CH_3$ | Cl | H | $CHF_2$ | $NO_2$ | |
| 1.329 | $CF_2CHF_2$ | H | C≡N | H | $CHF_2$ | $NO_2$ | |
| 1.330 | $CCl_3$ | $CH_3$ | Cl | H | $CHF_2$ | $NO_2$ | |
| 1.331 | $CCl_3$ | H | Cl | Cl | $CHF_2$ | $NO_2$ | |
| 1.332 | $CCl_3$ | H | Br | Cl | $CHF_2$ | $NO_2$ | |
| 1.333 | $CF_2CHF_2$ | H | Cl | Cl | $CHF_2$ | $NO_2$ | |
| 1.334 | $CF_2CHF_2$ | H | Br | Cl | $CHF_2$ | $NO_2$ | |
| 1.335 | $CF_2CF_3$ | H | H | H | $CHF_2$ | $NO_2$ | |
| 1.336 | $CF_2CF_3$ | $CH_3$ | H | H | $CHF_2$ | $NO_2$ | |
| 1.337 | $CF_2CF_3$ | $CH_3$ | Cl | H | $CHF_2$ | $NO_2$ | |
| 1.338 | $CF_2CF_3$ | H | Cl | H | $CHF_2$ | $NO_2$ | |
| 1.339 | $CF_2CF_3$ | H | F | H | $CHF_2$ | $NO_2$ | |
| 1.340 | $CF_2CF_3$ | H | Br | H | $CHF_2$ | $NO_2$ | |
| 1.341 | $CF_2CF_3$ | H | $CH_3$ | H | $CHF_2$ | $NO_2$ | |
| 1.342 | $CF_2CF_3$ | H | C≡N | H | $CHF_2$ | $NO_2$ | |
| 1.343 | $CH_3$ | H | Cl | H | $CHF_2$ | $NO_2$ | |
| 1.344 | $CH_3$ | H | Br | H | $CHF_2$ | $NO_2$ | |
| 1.345 | $CH_3$ | H | F | H | $CHF_2$ | $NO_2$ | |
| 1.346 | $CF_2Cl$ | H | Cl | H | $CHF_2$ | $NO_2$ | |
| 1.347 | $CF_2Cl$ | H | Br | H | $CHF_2$ | $NO_2$ | |
| 1.348 | $CF_3$ | H | Cl | H | $CHF_2$ | $NO_2$ | |
| 1.349 | $CF_3$ | Cl | Cl | H | $CHF_2$ | $NO_2$ | |
| 1.350 | $CF_3$ | H | Br | H | $CHF_2$ | $NO_2$ | |
| 1.351 | $CF_3$ | H | J | H | $CHF_2$ | $NO_2$ | |
| 1.352 | $CF_2CF_3$ | H | Cl | Cl | $CHF_2$ | $NO_2$ | |
| 1.353 | $CF_2CF_3$ | H | Br | Cl | $CHF_2$ | $NO_2$ | |
| 1.354 | $CCl_3$ | H | H | H | $CH_2F$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.355 | $CCl_3$ | H | Cl | H | $CH_2F$ | $NO_2$ | |
| 1.356 | $CCl_3$ | H | Br | H | $CH_2F$ | $NO_2$ | |
| 1.357 | $CF_2CHF_2$ | H | H | H | $CH_2F$ | $NO_2$ | |
| 1.358 | $CF_2CHF_2$ | H | Cl | H | $CH_2F$ | $NO_2$ | |
| 1.359 | $CF_2CHF_2$ | H | Br | H | $CH_2F$ | $NO_2$ | |
| 1.360 | $CF_2CF_3$ | H | Cl | H | $CH_2F$ | $NO_2$ | |
| 1.361 | $CF_2CF_3$ | H | Cl | Cl | $CH_2F$ | $NO_2$ | |
| 1.362 | $CF_2Cl$ | H | Cl | H | $CH_2F$ | $NO_2$ | |
| 1.363 | $CCl_3$ | H | H | H | $CF_2Cl$ | $NO_2$ | |
| 1.364 | $CCl_3$ | H | Cl | H | $CF_2Cl$ | $NO_2$ | |
| 1.365 | $CCl_3$ | H | Br | H | $CF_2Cl$ | $NO_2$ | |
| 1.366 | $CCl_3$ | H | Cl | Cl | $CF_2Cl$ | $NO_2$ | |
| 1.367 | $CCl_3$ | $CH_3$ | Cl | H | $CF_2Cl$ | $NO_2$ | |
| 1.368 | $CF_2CHF_2$ | H | Cl | H | $CF_2Cl$ | $NO_2$ | |
| 1.369 | $CF_2CHF_2$ | H | Br | Cl | $CF_2Cl$ | $NO_2$ | |
| 1.370 | $CF_2CHF_2$ | H | H | Cl | $CF_2Cl$ | $NO_2$ | |
| 1.371 | $CF_2CHF_2$ | H | $CH_3$ | Cl | $CF_2Cl$ | $NO_2$ | |
| 1.372 | $CF_2CF_3$ | H | Cl | H | $CF_2Cl$ | $NO_2$ | |
| 1.373 | $CF_2CF_3$ | H | Br | H | $CF_2Cl$ | $NO_2$ | |
| 1.375 | $CF_2CF_3$ | H | H | Cl | $CF_2Cl$ | $NO_2$ | |
| 1.376 | $CF_2CF_3$ | H | C≡N | H | $CF_2Cl$ | $NO_2$ | |
| 1.377 | $CF_2Cl$ | H | Cl | H | $CF_2Cl$ | $NO_2$ | |
| 1.378 | $CF_2Cl$ | H | Br | H | $CF_2Cl$ | $NO_2$ | |
| 1.379 | $CF_3$ | H | Cl | H | $CF_2Cl$ | $NO_2$ | |
| 1.380 | $CF_3$ | H | Br | H | $CF_2Cl$ | $NO_2$ | |
| 1.381 | $CF_3$ | H | Cl | Cl | $CF_2Cl$ | $NO_2$ | |
| 1.382 | $CF_3$ | H | Br | Cl | $CF_2Cl$ | $NO_2$ | |
| 1.383 | $CCl_3$ | H | Cl | H | $CFCl_2$ | $NO_2$ | |
| 1.384 | $CCl_3$ | H | Cl | Cl | $CFCl_2$ | $NO_2$ | |
| 1.385 | $CCl_3$ | H | Br | H | $CFCl_2$ | $NO_2$ | |
| 1.386 | $CCl_3$ | H | Br | Cl | $CFCl_2$ | $NO_2$ | |
| 1.387 | $CCl_3$ | H | H | H | $CFCl_2$ | $NO_2$ | |
| 1.388 | $CCl_3$ | $CH_3$ | H | Cl | $CFCl_2$ | $NO_2$ | |
| 1.389 | $CCl_3$ | $CF_3$ | H | H | $CFCl_2$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.390 | $CF_2CHF_2$ | H | Cl | H | $CFCl_2$ | $NO_2$ | |
| 1.391 | $CF_2CHF_2$ | H | H | H | $CFCl_2$ | $NO_2$ | |
| 1.392 | $CF_2CHF_2$ | H | Br | H | $CFCl_2$ | $NO_2$ | |
| 1.393 | $CF_2CHF_2$ | H | Cl | Cl | $CFCl_2$ | $NO_2$ | |
| 1.394 | $CF_2CHF_2$ | H | H | Cl | $CFCl_2$ | $NO_2$ | |
| 1.395 | $CF_2CHF_2$ | H | Br | Cl | $CFCl_2$ | $NO_2$ | |
| 1.396 | $CF_2CHF_2$ | $CH_3$ | Cl | Cl | $CFCl_2$ | $NO_2$ | |
| 1.397 | $CF_2CF_3$ | H | Cl | H | $CFCl_2$ | $NO_2$ | |
| 1.398 | $CF_2CF_3$ | H | Br | H | $CFCl_2$ | $NO_2$ | |
| 1.399 | $CF_2CF_3$ | H | $CH_3$ | H | $CFCl_2$ | $NO_2$ | |
| 1.400 | $CF_2CF_3$ | $CH_3$ | H | H | $CFCl_2$ | $NO_2$ | |
| 1.401 | $CF_2CF_3$ | $CF_3$ | H | H | $CFCl_2$ | $NO_2$ | |
| 1.402 | $CF_2CF_3$ | H | Cl | Cl | $CFCl_2$ | $NO_2$ | |
| 1.403 | $CF_2CF_3$ | H | H | Cl | $CFCl_2$ | $NO_2$ | |
| 1.404 | $CF_2CF_3$ | H | C≡N | H | $CFCl_2$ | $NO_2$ | |
| 1.405 | $CCl_3$ | H | Cl | H | $CF_2CHF_2$ | $NO_2$ | |
| 1.406 | $CCl_3$ | H | H | H | $CF_2CHF_2$ | $NO_2$ | |
| 1.407 | $CCl_3$ | $CH_3$ | Cl | H | $CF_2CHF_2$ | $NO_2$ | |
| 1.408 | $CCl_3$ | H | Br | H | $CF_2CHF_2$ | $NO_2$ | |
| 1.409 | $CCl_3$ | H | C≡N | H | $CF_2CHF_2$ | $NO_2$ | |
| 1.410 | $CCl_3$ | H | Cl | Cl | $CF_2CHF_2$ | $NO_2$ | |
| 1.411 | $CCl_3$ | H | Br | Cl | $CF_2CHF_2$ | $NO_2$ | |
| 1.412 | $CF_2CHF_2$ | H | Cl | H | $CF_2CHF_2$ | $NO_2$ | |
| 1.413 | $CF_2CHF_2$ | H | H | H | $CF_2CHF_2$ | $NO_2$ | |
| 1.414 | $CF_2CHF_2$ | H | Br | H | $CF_2CHF_2$ | $NO_2$ | |
| 1.415 | $CF_2CHF_2$ | H | Cl | Cl | $CF_2CHF_2$ | $NO_2$ | |
| 1.416 | $CF_2CHF_2$ | H | Br | Cl | $CF_2CHF_2$ | $NO_2$ | |
| 1.417 | $CF_2CHF_2$ | $CH_3$ | H | H | $CF_2CHF_2$ | $NO_2$ | |
| 1.418 | $CF_2CHF_2$ | $CH_3$ | Cl | H | $CF_2CHF_2$ | $NO_2$ | |
| 1.419 | $CF_2CF_3$ | H | Cl | H | $CF_2CHF_2$ | $NO_2$ | |
| 1.420 | $CF_2CF_3$ | H | Br | H | $CF_2CHF_2$ | $NO_2$ | |
| 1.421 | $CF_2CF_3$ | $CH_3$ | H | Cl | $CF_2CHF_2$ | $NO_2$ | |
| 1.422 | $CF_2CF_3$ | $CH_3$ | Cl | H | $CF_2CHF_2$ | $NO_2$ | |
| 1.423 | $CF_2CF_3$ | H | C≡N | Cl | $CF_2CHF_2$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.424 | $CCl_3$ | H | Cl | H | $OCF_2CHF_2$ | $NO_2$ | Smp. 127-128 |
| 1.425 | $CCl_3$ | H | Br | H | $OCF_2CHF_2$ | $NO_2$ | Smp. 124-125 |
| 1.426 | $CCl_3$ | H | H | H | $OCF_2CHF_2$ | $NO_2$ | |
| 1.427 | $CCl_3$ | H | $CH_3$ | H | $OCF_2CHF_2$ | $NO_2$ | |
| 1.428 | $CCl_3$ | H | C≡N | H | $OCF_2CHF_2$ | $NO_2$ | |
| 1.429 | $CCl_3$ | $CH_3$ | H | H | $OCF_2CHF_2$ | $NO_2$ | |
| 1.430 | $CCl_3$ | $CH_3$ | Cl | H | $OCF_2CHF_2$ | $NO_2$ | |
| 1.431 | $CCl_3$ | H | Cl | Cl | $OCF_2CHF_2$ | $NO_2$ | |
| 1.432 | $CCl_3$ | H | Br | Cl | $OCF_2CHF_2$ | $NO_2$ | |
| 1.433 | $CCl_3$ | H | H | Cl | $OCF_2CHF_2$ | $NO_2$ | |
| 1.434 | $CCl_3$ | H | $CH_3$ | Cl | $OCF_2CHF_2$ | $NO_2$ | |
| 1.435 | $CCl_3$ | $CH_3$ | H | Cl | $OCF_2CHF_2$ | $NO_2$ | |
| 1.436 | $CCl_3$ | $CH_3$ | Cl | Cl | $OCF_2CHF_2$ | $NO_2$ | |
| 1.437 | $CF_2CHF_2$ | H | Cl | H | $OCF_2CHF_2$ | $NO_2$ | Smp. 101-104 |
| 1.438 | $CF_2CHF_2$ | H | Br | H | $OCF_2CHF_2$ | $NO_2$ | |
| 1.439 | $CF_2CHF_2$ | H | H | H | $OCF_2CHF_2$ | $NO_2$ | |
| 1.440 | $CF_2CHF_2$ | H | F | H | $OCF_2CHF_2$ | $NO_2$ | |
| 1.441 | $CF_2CHF_2$ | H | C≡N | H | $OCF_2CHF_2$ | $NO_2$ | |
| 1.442 | $CF_2CHF_2$ | H | $CH_3$ | H | $OCF_2CHF_2$ | $NO_2$ | Smp. 81-82 |
| 1.443 | $CF_2CHF_2$ | $CH_3$ | H | H | $OCF_2CHF_2$ | $NO_2$ | |
| 1.444 | $CF_2CHF_2$ | $CH_3$ | Cl | H | $OCF_2CHF_2$ | $NO_2$ | |
| 1.445 | $CF_2CHF_2$ | H | Cl | Cl | $OCF_2CHF_2$ | $NO_2$ | |
| 1.446 | $CF_2CHF_2$ | H | Br | Cl | $OCF_2CHF_2$ | $NO_2$ | |
| 1.447 | $CF_2CHF_2$ | H | $CH_3$ | Cl | $OCF_2CHF_2$ | $NO_2$ | |
| 1.448 | $CF_2CHF_2$ | $CH_3$ | H | Cl | $OCF_2CHF_2$ | $NO_2$ | |
| 1.449 | $CF_2CHF_2$ | $CH_3$ | Cl | Cl | $OCF_2CHF_2$ | $NO_2$ | |
| 1.450 | $CF_2CHF_2$ | $CF_3$ | H | H | $OCF_2CHF_2$ | $NO_2$ | |
| 1.451 | $CF_2CHF_2$ | $CF_3$ | H | Cl | $OCF_2CHF_2$ | $NO_2$ | |
| 1.452 | $CCl_3$ | $CF_3$ | H | H | $OCF_2CHF_2$ | $NO_2$ | |
| 1.453 | $CCl_3$ | $CF_3$ | H | Cl | $OCF_2CHF_2$ | $NO_2$ | |
| 1.454 | $CF_2CF_3$ | H | H | H | $OCF_2CHF_2$ | $NO_2$ | |
| 1.455 | $CF_2CF_3$ | H | Cl | H | $OCF_2CHF_2$ | $NO_2$ | |
| 1.456 | $CF_2CF_3$ | H | Br | H | $OCF_2CHF_2$ | $NO_2$ | |
| 1.457 | $CF_2CF_3$ | H | F | H | $OCF_2CHF_2$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.458 | $CF_2CF_3$ | H | $CH_3$ | H | $OCF_2CHF_2$ | $NO_2$ | |
| 1.459 | $CF_2CF_3$ | $CH_3$ | H | H | $OCF_2CHF_2$ | $NO_2$ | |
| 1.460 | $CF_2CF_3$ | $CF_3$ | H | H | $OCF_2CHF_2$ | $NO_2$ | |
| 1.461 | $CF_2CF_3$ | H | H | Cl | $OCF_2CHF_2$ | $NO_2$ | |
| 1.462 | $CF_2CF_3$ | H | Cl | Cl | $OCF_2CHF_2$ | $NO_2$ | |
| 1.463 | $CF_2CF_3$ | H | Br | Cl | $OCF_2CHF_2$ | $NO_2$ | |
| 1.464 | $CF_2CF_3$ | H | F | Cl | $OCF_2CHF_2$ | $NO_2$ | |
| 1.465 | $CF_2CF_3$ | H | $CH_3$ | Cl | $OCF_2CHF_2$ | $NO_2$ | |
| 1.466 | $CF_2CF_3$ | H | C≡N | Cl | $OCF_2CHF_2$ | $NO_2$ | |
| 1.467 | $CF_2CF_3$ | $CH_3$ | H | Cl | $OCF_2CHF_2$ | $NO_2$ | |
| 1.468 | $CF_2CF_3$ | $CF_3$ | H | Cl | $OCF_2CHF_2$ | $NO_2$ | |
| 1.469 | $CClF_2$ | H | Cl | H | $OCF_2CHF_2$ | $NO_2$ | Smp. 100-102 |
| 1.470 | $CCl_3$ | H | H | H | $OCF_2CHFCF_3$ | $NO_2$ | Sirup |
| 1.471 | $CCl_3$ | H | Cl | H | $OCF_2CHFCF_3$ | $NO_2$ | Sirup |
| 1.472 | $CCl_3$ | H | Br | H | $OCF_2CHFCF_3$ | $NO_2$ | |
| 1.473 | $CCl_3$ | H | F | H | $OCF_2CHFCF_3$ | $NO_2$ | |
| 1.474 | $CCl_3$ | $CH_3$ | H | H | $OCF_2CHFCF_3$ | $NO_2$ | |
| 1.475 | $CCl_3$ | $CH_3$ | Cl | H | $OCF_2CHFCF_3$ | $NO_2$ | |
| 1.476 | $CCl_3$ | $CF_3$ | H | H | $OCF_2CHFCF_3$ | $NO_2$ | |
| 1.477 | $CF_3$ | $CF_3$ | H | H | $OCF_2CHFCF_3$ | $NO_2$ | |
| 1.478 | $CF_3$ | H | Cl | H | $OCF_2CHFCF_3$ | $NO_2$ | |
| 1.479 | $CF_2Cl$ | H | $CH_3$ | H | $OCF_2CHFCF_3$ | $NO_2$ | |
| 1.480 | $CHCl_2$ | H | H | H | $OCF_2CHFCF_3$ | $NO_2$ | |
| 1.481 | $CF_2CHF_2$ | H | Cl | H | $OCF_2CHFCF_3$ | $NO_2$ | Sirup |
| 1.482 | $CF_2CHF_2$ | H | Br | H | $OCF_2CHFCF_3$ | $NO_2$ | |
| 1.483 | $CF_2CHF_2$ | H | H | H | $OCF_2CHFCF_3$ | $NO_2$ | |
| 1.484 | $CF_2CHF_2$ | H | $CH_3$ | H | $OCF_2CHFCF_3$ | $NO_2$ | |
| 1.485 | $CF_2CHF_2$ | $CH_3$ | H | Cl | $OCF_2CHFCF_3$ | $NO_2$ | |
| 1.486 | $CF_2CHF_2$ | H | H | Cl | $OCF_2CHFCF_3$ | $NO_2$ | |
| 1.487 | $CF_2CF_3$ | H | H | H | $OCF_2CHFCF_3$ | $NO_2$ | |
| 1.488 | $CF_2CF_3$ | H | Cl | H | $OCF_2CHFCF_3$ | $NO_2$ | |
| 1.489 | $CF_2CF_3$ | H | Br | H | $OCF_2CHFCF_3$ | $NO_2$ | |
| 1.490 | $CF_2CF_3$ | H | Cl | Cl | $OCF_2CHFCF_3$ | $NO_2$ | |
| 1.491 | $CCl_3$ | H | H | Cl | $OCF_2CHFCF_3$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.492 | $CCl_3$ | H | Br | Cl | $OCF_2CHFCF_3$ | $NO_2$ | |
| 1.493 | $CCl_3$ | H | Cl | H | $OCF_2CHClF$ | $NO_2$ | |
| 1.494 | $CCl_3$ | H | Br | H | $OCF_2CHClF$ | $NO_2$ | |
| 1.495 | $CCl_3$ | H | F | H | $OCF_2CHClF$ | $NO_2$ | |
| 1.496 | $CCl_3$ | $CH_3$ | H | H | $OCF_2CHClF$ | $NO_2$ | |
| 1.497 | $CCl_3$ | $CF_3$ | H | H | $OCF_2CHClF$ | $NO_2$ | |
| 1.498 | $CCl_3$ | H | H | H | $OCF_2CHClF$ | $NO_2$ | |
| 1.499 | $CCl_3$ | H | $CH_3$ | H | $OCF_2CHClF$ | $NO_2$ | |
| 1.500 | $CCl_3$ | H | C≡N | H | $OCF_2CHClF$ | $NO_2$ | |
| 1.501 | $CF_3$ | H | H | H | $OCF_2CHClF$ | $NO_2$ | |
| 1.502 | $CF_3$ | H | Cl | H | $OCF_2CHClF$ | $NO_2$ | |
| 1.503 | $CF_2Cl$ | H | H | H | $OCF_2CHClF$ | $NO_2$ | |
| 1.504 | $CFBrCF_3$ | H | Cl | H | $OCF_2CHClF$ | $NO_2$ | |
| 1.505 | $CF_2CF_2CF_3$ | H | Cl | H | $OCF_2CHClF$ | $NO_2$ | |
| 1.506 | $CF_2CHF_2$ | H | Cl | H | $OCF_2CHClF$ | $NO_2$ | |
| 1.507 | $CF_2CHF_2$ | H | Br | H | $OCF_2CHClF$ | $NO_2$ | |
| 1.508 | $CF_2CHF_2$ | H | $CH_3$ | H | $OCF_2CHClF$ | $NO_2$ | |
| 1.509 | $CF_2CHF_2$ | $CH_3$ | H | H | $OCF_2CHClF$ | $NO_2$ | |
| 1.510 | $CF_2CF_2CF_3$ | H | Cl | H | $OCF_2CHClF$ | $NO_2$ | |
| 1.511 | $CF_2CF_2CF_3$ | H | H | H | $OCF_2CHClF$ | $NO_2$ | |
| 1.512 | $CF_2CF_3$ | H | Cl | H | $OCF_2CHClF$ | $NO_2$ | |
| 1.513 | $CF_2CF_3$ | H | Br | H | $OCF_2CHClF$ | $NO_2$ | |
| 1.514 | $CF_2CF_3$ | $CH_3$ | H | H | $OCF_2CHClF$ | $NO_2$ | |
| 1.515 | $CF_2CF_3$ | H | H | H | $OCF_2CHClF$ | $NO_2$ | |
| 1.515 | $CCl_3$ | H | H | Cl | $OCF_2CHFCl$ | $NO_2$ | |
| 1.517 | $CCl_3$ | H | Cl | Cl | $OCF_2CHFCl$ | $NO_2$ | |
| 1.518 | $CCl_3$ | H | Br | Cl | $OCF_2CHFCl$ | $NO_2$ | |
| 1.519 | $CCl_3$ | $CH_3$ | H | Cl | $OCF_2CHFCl$ | $NO_2$ | |
| 1.520 | $CF_2CHF_2$ | H | H | Cl | $OCF_2CHFCl$ | $NO_2$ | |
| 1.521 | $CF_2CHF_2$ | H | Cl | Cl | $OCF_2CHFCl$ | $NO_2$ | |
| 1.522 | $CF_2CHF_2$ | H | Br | Cl | $OCF_2CHFCl$ | $NO_2$ | |
| 1.523 | $CF_2CHF_2$ | H | C≡N | Cl | $OCF_2CHFCl$ | $NO_2$ | |
| 1.524 | $CF_2CHF_2$ | H | $CH_3$ | H | $OCF_2CHFCl$ | $NO_2$ | |
| 1.525 | $CH_3$ | H | H | H | $OCF_2CHF_2$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.526 | $CH_3$ | H | Br | H | $OCF_2CHF_2$ | $NO_2$ | |
| 1.527 | $CH_3$ | H | Cl | H | $OCF_2CHF_2$ | $NO_2$ | |
| 1.528 | $CF_2Cl$ | H | H | H | $OCF_2CHF_2$ | $NO_2$ | |
| 1.529 | $CF_3CF_2CF_2$ | H | Cl | H | $OCF_2CHF_2$ | $NO_2$ | |
| 1.530 | $CF_2CHFCl$ | H | Cl | H | $OCF_2CHF_2$ | $NO_2$ | |
| 1.531 | Cl | H | Br | H | $OCF_2CHF_2$ | $NO_2$ | |
| 1.532 | $CH_3$ | H | H | H | $OCF_2CHFCF_3$ | $NO_2$ | |
| 1.533 | $CH_3$ | H | Br | H | $OCF_2CHFCF_3$ | $NO_2$ | |
| 1.534 | 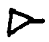 | H | H | H | $OCF_2CHFCF_3$ | $NO_2$ | |
| 1.535 | 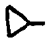 | H | Br | H | $OCF_2CHFCF_3$ | $NO_2$ | |
| 1.536 | $CF_2Cl$ | H | Cl | H | $OCF_2CHFCF_3$ | $NO_2$ | |
| 1.537 | $CF_2CF_2CF_3$ | H | Cl | H | $OCF_2CHFCF_3$ | $NO_2$ | |
| 1.538 | $CCl_3$ | H | H | H | $OCFCl_2$ | $NO_2$ | |
| 1.539 | $CCl_3$ | H | Cl | H | $OCFCl_2$ | $NO_2$ | |
| 1.540 | $CCl_3$ | H | Br | H | $OCFCl_2$ | $NO_2$ | |
| 1.541 | $CCl_3$ | H | $CH_3$ | H | $OCFCl_2$ | $NO_2$ | |
| 1.542 | $CCl_3$ | H | C≡N | H | $OCFCl_2$ | $NO_2$ | |
| 1.543 | $CCl_3$ | H | H | H | $OCF_2Cl$ | $NO_2$ | |
| 1.544 | $CCl_3$ | H | Cl | H | $OCF_2Cl$ | $NO_2$ | |
| 1.545 | $CCl_3$ | H | Br | H | $OCF_2Cl$ | $NO_2$ | |
| 1.546 | $CCl_3$ | H | $CH_3$ | H | $OCF_2Cl$ | $NO_2$ | |
| 1.547 | $CCl_3$ | H | C≡N | H | $OCF_2Cl$ | $NO_2$ | |
| 1.548 | $CF_2CHF_2$ | H | H | H | $OCFCl_2$ | $NO_2$ | |
| 1.549 | $CF_2CHF_2$ | H | Cl | H | $OCFCl_2$ | $NO_2$ | |
| 1.550 | $CF_2CHF_2$ | H | Br | H | $OCFCl_2$ | $NO_2$ | |
| 1.551 | $CF_2CHF_2$ | $CH_3$ | H | H | $OCF_2Cl$ | $NO_2$ | |
| 1.552 | $CF_2CHF_2$ | H | H | H | $OCF_2Cl$ | $NO_2$ | |
| 1.553 | $CF_2CHF_2$ | H | Cl | H | $OCF_2Cl$ | $NO_2$ | |
| 1.554 | $CF_2CF_3$ | H | H | H | $OCF_2Cl$ | $NO_2$ | |
| 1.555 | $CF_2CF_3$ | H | Cl | H | $OCF_2Cl$ | $NO_2$ | |
| 1.556 | $CF_2CF_3$ | H | H | H | $OCFCl_2$ | $NO_2$ | |
| 1.557 | $CF_2CF_3$ | H | Cl | H | $OCFCl_2$ | $NO_2$ | |
| 1.558 | $CCl_3$ | H | H | H | $OCHF_2$ | $NO_2$ | |
| 1.559 | $CCl_3$ | H | Cl | H | $OCHF_2$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.560 | $CCl_3$ | H | Br | H | $OCHF_2$ | $NO_2$ | |
| 1.561 | $CCl_3$ | $CH_3$ | H | H | $OCHF_2$ | $NO_2$ | |
| 1.562 | $CCl_3$ | H | H | Cl | $OCHF_2$ | $NO_2$ | |
| 1.563 | $CCl_3$ | H | Cl | Cl | $OCHF_2$ | $NO_2$ | |
| 1.564 | $CCl_3$ | H | Br | Cl | $OCHF_2$ | $NO_2$ | |
| 1.565 | $CCl_3$ | $CH_3$ | H | Cl | $OCHF_2$ | $NO_2$ | |
| 1.566 | $CF_2CHF_2$ | H | H | H | $OCHF_2$ | $NO_2$ | |
| 1.567 | $CF_2CHF_2$ | H | Cl | H | $OCHF_2$ | $NO_2$ | |
| 1.568 | $CF_2CHF_2$ | H | Br | H | $OCHF_2$ | $NO_2$ | |
| 1.569 | $CF_2CHF_2$ | H | $CH_3$ | H | $OCHF_2$ | $NO_2$ | |
| 1.570 | $CF_2CHF_2$ | H | H | Cl | $OCHF_2$ | $NO_2$ | |
| 1.571 | $CF_2CHF_2$ | H | Cl | Cl | $OCHF_2$ | $NO_2$ | |
| 1.572 | $CF_2CHF_2$ | H | Br | Cl | $OCHF_2$ | $NO_2$ | |
| 1.573 | $CF_2CHF_2$ | H | C≡N | Cl | $OCHF_2$ | $NO_2$ | |
| 1.574 | $CF_2CF_3$ | H | H | H | $OCHF_2$ | $NO_2$ | |
| 1.575 | $CF_2CF_3$ | H | Cl | H | $OCHF_2$ | $NO_2$ | |
| 1.576 | $CCl_3$ | H | Cl | H | $OCF_3$ | $NO_2$ | |
| 1.577 | $CCl_3$ | H | H | H | $OCF_3$ | $NO_2$ | Smp. 44-48 |
| 1.578 | $CCl_3$ | H | C≡N | H | $OCF_3$ | $NO_2$ | |
| 1.579 | $CF_2CHF_2$ | H | $CH_3$ | H | $OCF_3$ | $NO_2$ | |
| 1.580 | $CF_2CHF_2$ | H | H | H | $OCF_3$ | $NO_2$ | |
| 1.581 | $CF_2CHF_2$ | H | Cl | H | $OCF_3$ | $NO_2$ | Smp. 94-96 |
| 1.582 | $CF_2CF_3$ | H | H | H | $OCF_3$ | $NO_2$ | |
| 1.583 | $CF_2CF_3$ | H | Cl | H | $OCF_3$ | $NO_2$ | |
| 1.584 | $CF_2CF_3$ | $CH_3$ | H | H | $OCF_3$ | $NO_2$ | |
| 1.585 | $CH_3$ | H | Cl | H | $OCF_3$ | $NO_2$ | |
| 1.586 | $CH_3$ | H | H | H | $OCF_3$ | $NO_2$ | |
| 1.587 | $i-C_3H_7$ | $CH_3$ | H | H | $OCF_3$ | $NO_2$ | |
| 1.588 | $i-C_3H_7$ | $CH_3$ | Cl | H | $OCF_3$ | $NO_2$ | |
| 1.589 | $CF_3$ | H | Cl | H | $OCF_3$ | $NO_2$ | |
| 1.590 | $CF_3$ | H | H | H | $OCF_3$ | $NO_2$ | |
| 1.591 | $CF_2Cl$ | H | Cl | H | $OCF_3$ | $NO_2$ | |
| 1.592 | $n-C_3F_7$ | H | Cl | H | $OCF_3$ | $NO_2$ | |
| 1.593 | $CCl_3$ | H | H | H | $OCF_2CHCl_2$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.594 | $CCl_3$ | $CH_3$ | H | H | $OCF_2CHCl_2$ | $NO_2$ | |
| 1.595 | $CCl_3$ | H | Cl | H | $OCF_2CHCl_2$ | $NO_2$ | |
| 1.596 | $CH_3$ | H | H | H | $OCF_2CHCl_2$ | $NO_2$ | |
| 1.597 | $CH_3$ | H | Br | H | $OCF_2CHCl_2$ | $NO_2$ | |
| 1.598 | $CF_2CHF_2$ | H | H | H | $OCF_2CHCl_2$ | $NO_2$ | |
| 1.599 | $CF_2CHF_2$ | H | Cl | H | $OCF_2CHCl_2$ | $NO_2$ | |
| 1.600 | $CF_2CHF_2$ | H | Br | H | $OCF_2CHCl_2$ | $NO_2$ | |
| 1.601 | $CF_2CF_3$ | H | Cl | H | $OCF_2CHCl_2$ | $NO_2$ | |
| 1.602 | $CF_2CF_3$ | H | Br | H | $OCF_2CHCl_2$ | $NO_2$ | |
| 1.603 | $CF_2CF_3$ | H | H | H | $OCF_2CHCl_2$ | $NO_2$ | |
| 1.604 | $CF_2Cl$ | H | Cl | H | $OCF_2CHCl_2$ | $NO_2$ | |
| 1.605 | $CF_2CF_2CF_3$ | H | Cl | H | $OCF_2CHCl_2$ | $NO_2$ | |
| 1.606 | $CCl_3$ | H | H | H | $OCF_2CF_3$ | $NO_2$ | |
| 1.607 | $CCl_3$ | H | Cl | H | $OCF_2CF_3$ | $NO_2$ | |
| 1.608 | $CF_2CHF_2$ | H | Cl | H | $OCF_2CF_3$ | $NO_2$ | |
| 1.609 | $CF_2CHF_2$ | H | H | H | $OCF_2CF_3$ | $NO_2$ | |
| 1.610 | $C_2H_5$ | H | Cl | H | $OCF_2CF_3$ | $NO_2$ | |
| 1.611 | $OCH_3$ | H | Cl | H | $OCF_2CF_3$ | $NO_2$ | |
| 1.612 | Cl | H | Br | H | $OCF_2CF_3$ | $NO_2$ | |
| 1.613 | $CCl_3$ | H | H | H | $OCF_2CHFBr$ | $NO_2$ | |
| 1.614 | $CCl_3$ | H | Cl | H | $OCF_2CHFBr$ | $NO_2$ | |
| 1.615 | $CF_2CHF_2$ | H | H | H | $OCF_2CHFBr$ | $NO_2$ | |
| 1.616 | $CF_2CHF_2$ | H | Cl | H | $OCF_2CHFBr$ | $NO_2$ | |
| 1.617 | $CF_2CHF_2$ | H | Br | H | $OCF_2CHFBr$ | $NO_2$ | |
| 1.618 | $CH_3$ | H | H | H | $OCF_2CHFBr$ | $NO_2$ | |
| 1.619 | $CH_3$ | H | Br | H | $OCF_2CHFBr$ | $NO_2$ | |
| 1.620 | 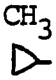 | H | H | H | $OCF_2CHFBr$ | $NO_2$ | |
| 1.621 | 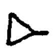 | H | Cl | H | $OCF_2CHFBr$ | $NO_2$ | |
| 1.622 | Cl | H | Cl | H | $OCF_2CHFBr$ | $NO_2$ | |
| 1.623 | Cl | H | Br | H | $OCF_2CHFBr$ | $NO_2$ | |
| 1.624 | $CF_2CF_3$ | H | Cl | H | $OCF_2CHFBr$ | $NO_2$ | |
| 1.625 | $CF_2CF_3$ | H | Br | H | $OCF_2CHFBr$ | $NO_2$ | |
| 1.626 | $CCl_3$ | $CH_3$ | H | H | $OCH_2CF_3$ | $NO_2$ | |
| 1.627 | $CCl_3$ | H | Cl | H | $OCH_2CF_3$ | $NO_2$ | |

Tabelle 1 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 1.628 | $CCl_3$ | H | Br | H | $OCH_2CF_3$ | $NO_2$ | |
| 1.629 | $CCl_3$ | H | C≡N | H | $OCH_2CF_3$ | $NO_2$ | |
| 1.630 | $CH_3$ | H | Cl | H | $OCH_2CF_3$ | $NO_2$ | |
| 1.631 | $CH_3$ | H | H | H | $OCH_2CF_3$ | $NO_2$ | |
| 1.632 | 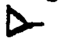 | H | H | H | $OCH_2CF_3$ | $NO_2$ | |
| 1.633 | 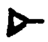 | H | Cl | H | $OCH_2CF_3$ | $NO_2$ | |
| 1.634 | $CF_3$ | H | H | H | $OCH_2CF_3$ | $NO_2$ | |
| 1.635 | $CF_2CHF_2$ | H | Cl | H | $OCH_2CF_3$ | $NO_2$ | |
| 1.636 | $CF_2CHF_2$ | H | H | H | $OCH_2CF_3$ | $NO_2$ | |
| 1.637 | C≡N | H | Br | H | $CO_2Et$ | $NO_2$ | |
| 1.638 | C≡N | H | Br | H | C≡N | $NO_2$ | |
| 1.639 | C≡N | H | Br | H | Cl | $NO_2$ | |
| 1.640 | $CF_2CHF_2$ | H | Cl | Cl | $CO_2CH(CH_3)_2$ | $NO_2$ | semi-kristallin |
| 1.641 | $CCl_3$ | H | H | Cl | $CO_2CH(CH_3)_2$ | $NO_2$ | Smp. 84-86 |
| 1.642 | $CCl_3$ | H | Cl | Cl | $CO_2CH(CH_3)_2$ | $NO_2$ | |
| 1.643 | $CF_2Cl$ | H | Br | H | $OCF_2CHF_2$ | $NO_2$ | Smp. 103-108 |
| 1.644 | $CCl_3$ | H | Cl | H | Br | $NO_2$ | |
| 1.645 | $CCl_3$ | H | Br | H | Br | $NO_2$ | |
| 1.646 | $CCl_3$ | H | Br | H | F | $NO_2$ | |
| 1.647 | $CCl_3$ | H | Cl | H | F | $NO_2$ | |
| 1.648 | $CF_2CF_2CF_3$ | H | Cl | H | $CO_2(CH_2)_7CH_3$ | $NO_2$ | |
| 1.649 | $CF_2CHFCF_3$ | H | Cl | H | $CO_2(CH_2)_7CH_3$ | $NO_2$ | |
| 1.650 | $CF_2CHFCF_3$ | H | Br | H | $CO_2(CH_2)_7CH_3$ | $NO_2$ | |
| 1.651 | $CF_2CHFCF_3$ | H | Cl | H | $CO_2C_6F_5$ | $NO_2$ | |
| 1.652 | $CH_3$ | H | Cl | H | $CO_2CH(CH_3)_2$ | $NO_2$ | Smp. 165 |
| 1.653 | $C_6F_5$ | H | Cl | H | $CO_2Et$ | $NO_2$ | |
| 1.654 | $C_6F_5$ | $CH_3$ | H | H | $CO_2Et$ | $NO_2$ | |
| 1.655 | $C_6F_5$ | H | H | H | C≡N | $NO_2$ | |
| 1.656 | $C_6F_5$ | H | Cl | H | C=N | $NO_2$ | |
| 1.657 | $C_6F_5$ | H | Br | H | C≡N | $NO_2$ | |
| 1.658 | $C_6F_5$ | H | Br | H | $CO_2Et$ | $NO_2$ | |
| 1.659 | $CF_2CHF_2$ | H | Cl | H | $CO_2N(CH_3)_2$ | $NO_2$ | Smp. 256-258 |
| 1.660 | $CF_2CHF_2$ | Cl | H | Cl | C≡N | $NO_2$ | semi-kristallin |
| 1.661 | $CF_2CF_2CF_3$ | H | Cl | Cl | $CO_2CH(CH_3)_2$ | $NO_2$ | Smp. 81-85 |

Fortsetzung Tabelle 1

| Nr. | $R_1$ | $R_2$ | $R_3$ | $R_4$ | $R_5$ | $R_6$ | physik. Konstanten [°C] |
|---|---|---|---|---|---|---|---|
| 1.662 | n-$C_7F_{15}$ | H | Cl | H | $CO_2Et$ | $NO_2$ | Sirup |
| 1.663 | n-$C_7F_{15}$ | H | Cl | H | C≡N | $NO_2$ | Smp. 88-89 |
| 1.664 | n-$C_7F_{15}$ | H | Cl | H | $CO_2CH(CH_3)_2$ | $NO_2$ | |
| 1.665 | n-$C_7F_{15}$ | H | Br | H | $CO_2Et$ | $NO_2$ | |
| 1.666 | n-$C_7F_{15}$ | H | Br | H | C≡N | $NO_2$ | |
| 1.667 | n-$C_7F_{15}$ | H | Br | H | Cl | $NO_2$ | |
| 1.668 | $CF_2CHF_2$ | $CH_3$ | H | H | C≡N | $NO_2$ | |
| 1.669 | $CF_2CHF_2$ | $CH_3$ | Br | H | $CO_2CH_3$ | $NO_2$ | |
| 1.570 | $CF_2CHF_2$ | $CH_3$ | Br | H | $CO_2Et$ | $NO_2$ | |
| 1.671 | $CF_2CHF_2$ | $CH_3$ | Br | Cl | $CO_2CH(CH_3)_2$ | $NO_2$ | |
| 1.672 | $CF_2CHF_2$ | H | Br | H | Br | $NO_2$ | |
| 1.673 | $CF_2CHF_2$ | H | Cl | H | Br | $NO_2$ | |
| 1.674 | $CF_2CHF_2$ | $CH_3$ | Br | H | Br | $NO_2$ | |
| 1.675 | $CF_2CHFCF_3$ | H | Br | H | Br | $NO_2$ | |
| 1.676 | $CCl_3$ | H | Br | H | F | $NO_2$ | |
| 1.677 | $CF_2CHF_2$ | H | Br | H | F | $NO_2$ | |

Tabelle 2

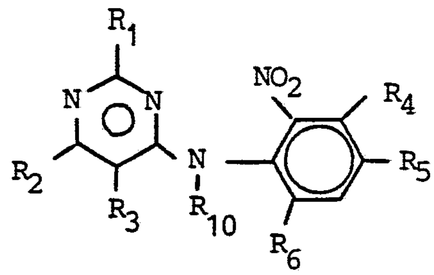

| Nr. | $R_1$ | $R_2$ | $R_3$ | $R_4$ | $R_5$ | $R_6$ | $R_{10}$ | physik. Konstanten [°C] |
|---|---|---|---|---|---|---|---|---|
| 2.1 | $CCl_3$ | H | H | H | C≡N | $NO_2$ | K | |
| 2.2 | $CCl_3$ | H | H | Cl | $CO_2Et$ | $NO_2$ | K | |
| 2.3 | $CF_2CHF_2$ | H | H | H | $CCl_3$ | $NO_2$ | K | |
| 2.4 | $CF_2CHF_2$ | H | H | H | $CO_2Et$ | $NO_2$ | K | |
| 2.5 | $CCl_3$ | H | Cl | H | $CH_3$ | $NO_2$ | K | Smp. 147-150 |
| 2.6 | $CCl_3$ | H | Cl | H | $CF_2Cl$ | $NO_2$ | K | |
| 2.7 | $CCl_3$ | H | Cl | H | C≡N | $NO_2$ | K | Smp. 103-107 |
| 2.8 | $CCl_3$ | H | Cl | H | 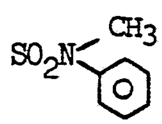 | $NO_2$ | K | Smp. 108-111 |
| 2.9 | $CCl_3$ | H | Cl | Cl | $CO_2Et$ | $NO_2$ | K | |
| 2.10 | $CF_2CHF_2$ | H | Cl | H | $CO_2Et$ | $NO_2$ | K | Smp. 257-260 |
| 2.11 | $CF_2CHF_2$ | H | Cl | Cl | $CO_2Et$ | $NO_2$ | K | |
| 2.12 | $CF_2CHF_2$ | H | Cl | H | C≡N | $NO_2$ | K | Smp. 117-121 |
| 2.13 | $CF_2CHF_2$ | H | Cl | Cl | C≡N | $NO_2$ | K | |
| 2.14 | $CF_2CHF_2$ | H | Cl | H | $CH_3$ | $NO_2$ | K | |
| 2.15 | $CCl_3$ | H | $CH_3$ | H | C≡N | $NO_2$ | K | |
| 2.16 | $CCl_3$ | H | $CH_3$ | Cl | $CO_2Et$ | $NO_2$ | Na | |
| 2.17 | $CCl_3$ | H | $CH_3$ | H | $CH_3$ | $NO_2$ | K | |
| 2.18 | $CF_2CF_3$ | H | Cl | H | C≡N | $NO_2$ | K | Smp. 125-129 |
| 2.19 | $CF_2CHF_2$ | H | Cl | Cl | $CF_3$ | $NO_2$ | K | Smp. 272-274 |

Tabelle 2 (Fortsetzung

| Nr. | $R_1$ | $R_2$ | $R_3$ | $R_4$ | $R_5$ | $R_6$ | $R_{10}$ | physik. Konstanten [°C] |
|---|---|---|---|---|---|---|---|---|
| 2.20 | $CCl_3$ | H | Cl | H | $CO_2Et$ | $NO_2$ | $OCH_3$ | |
| 2.21 | $CF_2Cl$ | H | Br | H | C≡N | $NO_2$ | $OCH_3$ | |
| 2.22 | $CF_2CHF_2$ | H | Cl | H | $CCl_3$ | $NO_2$ | $CH_3$ | |
| 2.23 | $CF_2CF_3$ | H | C≡N | H | $CH_3$ | $NO_2$ | $C_2H_5$ | |
| 2.24 | $CCl_3$ | H | Br | H | $CF_2Cl$ | $NO_2$ | $OCH_3$ | |
| 2.25 | $CF_3$ | H | H | Cl | $CO_2Et$ | $NO_2$ | $OC_2H_5$ | |
| 2.26 | C≡N | H | Cl | Cl | C≡N | $NO_2$ | 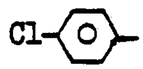 | |
| 2.27 | $CCl_3$ | Cl | Cl | H | $CCl_2F$ | $NO_2$ | $OCH_3$ | |
| 2.28 | $CCl_3$ | H | Cl | Cl | $CO_2Et$ | $NO_2$ | $CH_3$ | |
| 2.29 | $CF_3$ | H | F | H | C≡N | $NO_2$ | $C_2H_5$ | |
| 2.30 | $CH_3$ | H | Cl | H | $CCl_3$ | $NO_2$ | $OC_3H_7$ | |
| 2.31 | $CCl_2CHF_2$ | Cl | H | Cl | $CF_2Cl$ | $NO_2$ | $CH_3$ | |
| 2.32 | Cl | H | Cl | Cl | $CO_2Bu$ | $NO_2$ | $OCH_3$ | |
| 2.33 | $CF_3$ | H | H | J | CONHMe | $NO_2$ | $C_2H_5$ | |
| 2.34 | $CHCl_2$ | $CH_3$ | H | Br | CONHEt | $NO_2$ | $CH_3$ | |
| 2.35 | $CH_2{=}CF$ | $CH_3$ | H | Cl | $CONMe_2$ | $NO_2$ | $C_2H_5$ | |
| 2.36 | $CCl_2{=}CCl$ | Cl | H | Cl | $CH_3$ | $NO_2$ | $OCH_3$ | |
| 2.37 | $CHCl_2$ | H | Cl | Cl | $C_2H_5$ | $NO_2$ | Na | |
| 2.38 | $CHBr_2$ | $C_2H_5$ | H | $SCH_3$ | $C_4H_9$ | $NO_2$ | Li | |
| 2.39 | $CF_2CHF_2$ | H | Cl | $OC_4H_9$ | $CO_2N(CH_3)_2$ | $NO_2$ | Na | |
| 2.40 | $CH_3$ | $OCH_3$ | $CF_3$ | H | $CO_2Et$ | $NO_2$ | K | Smp. > 275 |
| 2.41 | $CH_3$ | $OCH_3$ | $CF_3$ | H | C≡N | $NO_2$ | K | Smp. 213-218 |
| 2.42 | $CCl_3$ | $CH_3$ | H | H | $CO_2K$ | $NO_2$ | K | Smp. 137-141 |
| 2.43 | $CCl_3$ | $CH_3$ | H | H | C≡N | $NO_2$ | K | Smp. 180-183 |
| 2.44 | $CCl_3$ | $CH_3$ | H | H | $CH_3$ | $NO_2$ | K | Smp. 113-117 |
| 2.45 | $CCl_3$ | $CH_3$ | Cl | H | $CO_2Et$ | $NO_2$ | K | Smp. 187-190 |
| 2.46 | $CCl_3$ | $CH_3$ | Cl | H | C≡N | $NO_2$ | K | Smp. 120-123 |
| 2.47 | $CF_2CF_3$ | H | Cl | H | C≡N | $NO_2$ | K | Smp. 125-129 |
| 2.48 | $CF_2CHF_2$ | H | Cl | H | Cl | $NO_2$ | K | Smp. 265-267 |

<u>Tabelle 2</u> (Fortsetzung)

| Nr. | $R_1$ | $R_2$ | $R_3$ | $R_4$ | $R_5$ | $R_6$ | $R_{10}$ | physik. Konstanten [°C] |
|---|---|---|---|---|---|---|---|---|
| 2.49 | $CF_2CHF_2$ | H | Cl | H | C≡N | $NO_2$ | K | Smp. 180-183 |
| 2.50 | $CClF_2$ | H | Cl | H | C≡N | $NO_2$ | K | Smp. 277-278 |
| 2.51 | $CClF_2$ | H | Cl | H | $CO_2Et$ | $NO_2$ | K | Smp. 263-267 |
| 2.52 | $CClF_2$ | H | Cl | H | $OCF_2CHF_2$ | $NO_2$ | K | Smp. 243-245 |
| 2.53 | $CClF_2$ | H | C≡N | H | $CO_2Et$ | $NO_2$ | K | Smp. 185-189 |
| 2.54 | $CClF_2$ | H | C≡N | H | C≡N | $NO_2$ | K | Smp. 267-268 |
| 2.55 | $CF_2CHF_2$ | H | Cl | H | $OCF_2CHF_2$ | $NO_2$ | K | Smp. 260-262 |
| 2.56 | $CCl_3$ | H | Cl | H | $CO_2Et$ | $NO_2$ | K | Sirup |

Tabelle 3

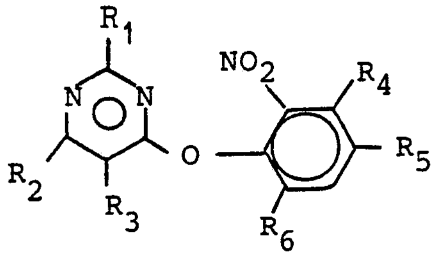

| Nr. | $R_1$ | $R_2$ | $R_3$ | $R_4$ | $R_5$ | $R_6$ | physik. Konstanten Smp. [°C] |
|---|---|---|---|---|---|---|---|
| 3.1 | $CCl_3$ | H | H | H | $CF_3$ | $NO_2$ | 106-108 |
| 3.2 | $CCl_3$ | Cl | H | Cl | $CF_3$ | $NO_2$ | |
| 3.3 | $CCl_3$ | H | H | Cl | $CF_3$ | $NO_2$ | |
| 3.4 | $CCl_3$ | Cl | H | H | $CF_3$ | $NO_2$ | |
| 3.5 | $CCl_3$ | $CH_3$ | H | H | $CF_3$ | $NO_2$ | 128-131 |
| 3.6 | $CCl_3$ | $CH_3$ | H | Cl | $CF_3$ | $NO_2$ | 205-207 |
| 3.7 | $CF_2CF_3$ | H | H | H | $CF_3$ | $NO_2$ | |
| 3.8 | $CF_2CF_3$ | H | H | Cl | $CF_3$ | $NO_2$ | Sirup |
| 3.9 | $CCl_3$ | $CH_3$ | H | H | $CO_2Et$ | $NO_2$ | Sirup |
| 3.10 | $CCl_3$ | H | Cl | H | $CF_3$ | $NO_2$ | 125-127 |
| 3.11 | $CCl_3$ | H | Cl | Cl | $CF_3$ | $NO_2$ | |
| 3.12 | $CF_2CHF_2$ | H | Cl | H | $CF_3$ | $NO_2$ | |
| 3.13 | $CF_2CHF_2$ | H | Cl | Cl | $CF_3$ | $NO_2$ | Sirup |
| 3.14 | $CCl_3$ | H | Cl | H | C≡N | $NO_2$ | |
| 3.15 | $CCl_3$ | H | Cl | Cl | C≡N | $NO_2$ | |
| 3.16 | $CCl_3$ | H | Cl | H | $CO_2Et$ | $NO_2$ | |
| 3.17 | $CCl_3$ | H | Cl | Cl | $CO_2Et$ | $NO_2$ | |
| 3.18 | $CCl_3$ | H | Cl | H | $CCl_3$ | $NO_2$ | |
| 3.19 | $CCl_3$ | H | Cl | Cl | $CCl_3$ | $NO_2$ | |
| 3.20 | $CF_2CHF_2$ | H | Cl | H | C≡N | $NO_2$ | |
| 3.21 | $CF_2CHF_2$ | H | Cl | Cl | C≡N | $NO_2$ | |
| 3.22 | $CF_2CHF_2$ | H | Cl | H | $CO_2Et$ | $NO_2$ | |

Tabelle 3 (Fortsetzung)

| Nr. | $R_1$ | $R_2$ | $R_3$ | $R_4$ | $R_5$ | $R_6$ | physik. Konstanten Smp. [°C] |
|---|---|---|---|---|---|---|---|
| 3.23 | $CF_2CHF_2$ | H | Cl | Cl | $CO_2Et$ | $NO_2$ | |
| 3.24 | $CF_2CHF_2$ | H | Cl | H | $CCl_3$ | $NO_2$ | |
| 3.25 | $CF_2CHF_2$ | H | Cl | Cl | $CF_2Cl$ | $NO_2$ | |
| 3.26 | $CF_2CF_3$ | H | $CH_3$ | H | $CF_3$ | $NO_2$ | 90-92 |
| 3.27 | $CF_2CF_3$ | H | $CH_3$ | Cl | $CF_3$ | $NO_2$ | 98-100 |
| 3.28 | $CCl_3$ | H | $CH_3$ | H | $CF_2Cl$ | $NO_2$ | |
| 3.29 | $CCl_3$ | H | $CH_3$ | Cl | $CF_2Cl$ | $NO_2$ | |
| 3.30 | $CCl_3$ | H | $CH_3$ | H | $CF_3$ | $NO_2$ | |
| 3.31 | $CCl_3$ | H | $CH_3$ | Cl | $CF_3$ | $NO_2$ | |
| 3.32 | $CH_3$ | H | C≡N | H | C≡N | $CF_3$ | |
| 3.33 | $OCH_3$ | H | Br | Cl | $CO_2CH_3$ | $CF_3$ | |
| 3.34 | Cl | Cl | Cl | H | $CF_3$ | $NO_2$ | |
| 3.35 | $SCH_3$ | H | Br | H | $CF_3$ | $NO_2$ | |
| 3.36 | 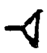 | H | Br | Cl | C≡N | $NO_2$ | |
| 3.37 | $CF_3$ | $CH_3$ | Cl | H | $CO_2Et$ | $NO_2$ | |
| 3.38 | $CF_3$ | $CH_3$ | Cl | Cl | $CO_2Et$ | $NO_2$ | |
| 3.39 | $CH_3$ | H | Cl | Cl | $CO_2Et$ | $NO_2$ | |
| 3.40 | $NMe_2$ | $NMe_2$ | H | Cl | $CCl_3$ | $NO_2$ | |
| 3.41 | $NMe_2$ | $NMe_2$ | H | H | $CCl_3$ | $NO_2$ | |
| 3.42 | $NHC_2H_5$ | $NHC_2H_5$ | H | Cl | $CH_3$ | $NO_2$ | |
| 3.43 | $CCl_3$ | H | Cl | H | $NO_2$ | $CF_3$ | |
| 3.44 | $CCl_3$ | H | Cl | Cl | $NO_2$ | $CF_3$ | |
| 3.45 | H | $CCl_3$ | H | Cl | $NO_2$ | $CF_3$ | |
| 3.46 | H | $CCl_3$ | H | Cl | $CF_3$ | $NO_2$ | |
| 3.47 | $CF_2CHF_2$ | H | $CH_3$ | H | $CF_3$ | $NO_2$ | 87-88 |
| 3.48 | $CF_2CHF_2$ | H | $CH_3$ | Cl | $CF_3$ | $NO_2$ | 93-95 |
| 3.49 | $CCl_3$ | $ClCH_2$ | H | H | $CF_3$ | $NO_2$ | 129-131 |
| 3.50 | $CCl_3$ | $CH_3$ | H | H | $CCl_3$ | $NO_2$ | semi-kristallin |
| 3.51 | $CCl_3$ | $CH_3$ | H | H | $CH_3$ | $NO_2$ | 189-191 |
| 3.52 | $CCl_3$ | $CH_3$ | H | Cl | $CO_2Et$ | $NO_2$ | semi-kristallin |
| 3.53 | $CCl_3$ | $CH_3$ | H | H | C≡N | $NO_2$ | 175-177 |

Tabelle 3 (Fortsetzung)

| Nr. | $R_1$ | $R_2$ | $R_3$ | $R_4$ | $R_5$ | physik. Konstanten Smp.[°C] |
|---|---|---|---|---|---|---|
| 3.54 | $CF_2CHF_2$ | $CH_3$ | H | Cl | $CF_3$ | |
| 3.55 | $CF_2CHF_2$ | $CH_3$ | H | Cl | $CO_2CH(CH_3)_2$ | |
| 3.56 | $CCl_3$ | $CH_3$ | Cl | Cl | $CF_3$ | |
| 3.57 | $CCl_3$ | $CH_3$ | H | Cl | $CO_2CH(CH_3)_2$ | |
| 3.58 | $CCl_3$ | $CH_3$ | H | Cl | $CO_2C_6F_5$ | |
| 3.59 | $CCl_3$ | H | H | Cl | $CO_2CH(CH_3)_2$ | |
| 3.60 | $CCl_3$ | H | Br | Cl | $CO_2CH(CH_3)_2$ | |
| 3.61 | $CCl_3$ | H | $CH_3$ | Cl | $CO_2CH(CH_3)_2$ | |
| 3.62 | $CF_2CHF_2$ | H | $CH_3$ | Cl | $CO_2CH(CH_3)_2$ | |
| 3.63 | $CF_2CHF_2$ | H | $CH_3$ | Cl | $CO_2C_6F_5$ | |
| 3.64 | $CF_2CHF_2$ | H | Br | Cl | $CF_3$ | |
| 3.65 | $CCl_3$ | H | Br | Cl | $CF_3$ | |
| 3.66 | $CF_2CHF_2$ | H | H | Cl | $CF_3$ | |
| 3.67 | $CF_2CHF_2$ | H | H | Cl | $CO_2CH(CH_3)_2$ | |
| 3.68 | $CCl_3$ | H | Cl | Cl | $OCF_3$ | |
| 3.69 | $CCl_3$ | $CH_3$ | H | Cl | $CO_2(CH_2)_3CH_3$ | |
| 3.70 | $CCl_3$ | $CH_3$ | Cl | Cl | $CO_2(CH_2)_3CH_3$ | |
| 3.71 | $CF_2CHF_2$ | H | $CH_3$ | Cl | $CO_2(CH_2)_3CH_3$ | |
| 3.72 | $CCl_3$ | H | Cl | Cl | C≡N | |
| 3.73 | $CCl_3$ | $CH_3$ | H | Cl | C≡N | |
| 3.74 | $CF_2CHF_2$ | H | Br | Cl | C≡N | |
| 3.75 | $C_6F_5$ | H | Br | Cl | $CF_3$ | |
| 3.76 | $C_6F_5$ | $CH_3$ | H | Cl | $CF_3$ | |
| 3.77 | $CF_2CHF_2$ | $CH_3$ | Br | Cl | $CF_3$ | |

Tabelle 4

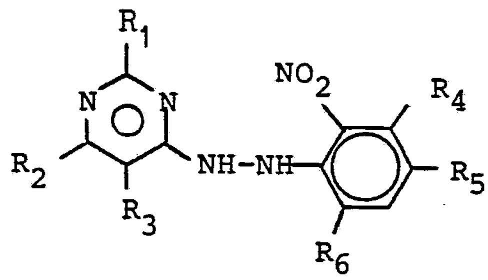

| Nr. | $R_1$ | $R_2$ | $R_3$ | $R_4$ | $R_5$ | $R_6$ | physik. Konstanten Smp. [°C] |
|---|---|---|---|---|---|---|---|
| 4.1 | $CCl_3$ | H | H | H | $CF_3$ | $NO_2$ | 138-141 |
| 4.2 | $CCl_3$ | H | H | Cl | $CF_3$ | $NO_2$ | 73-75 |
| 4.3 | $CCl_3$ | H | H | H | C≡N | $NO_2$ | |
| 4.4 | $CCl_3$ | H | H | Cl | C≡N | $NO_2$ | |
| 4.5 | $CCl_3$ | H | H | H | $CO_2Et$ | $NO_2$ | |
| 4.6 | $CCl_3$ | H | H | Cl | $CH_3$ | $NO_2$ | |
| 4.7 | $CCl_3$ | H | H | H | $CCl_3$ | $NO_2$ | |
| 4.8 | $CCl_3$ | H | H | H | $NO_2$ | $CF_3$ | |
| 4.9 | $CCl_3$ | H | H | Cl | $NO_2$ | $CF_3$ | |
| 4.10 | $CF_3$ | H | H | H | $CF_3$ | $NO_2$ | |
| 4.11 | $CF_3$ | H | H | Cl | $CF_3$ | $NO_2$ | |
| 4.12 | $CF_3$ | H | H | H | C≡N | $NO_2$ | |
| 4.13 | $CF_3$ | H | H | Cl | C≡N | $NO_2$ | |
| 4.14 | $CF_3$ | H | H | H | $CO_2Et$ | $NO_2$ | |
| 4.15 | $CF_3$ | H | H | Cl | $CO_2Et$ | $NO_2$ | |
| 4.16 | $CF_3$ | H | H | H | $CCl_3$ | $NO_2$ | |
| 4.17 | $CF_3$ | H | H | Cl | $CCl_3$ | $NO_2$ | |
| 4.18 | $CF_2CHF_2$ | H | H | H | $CF_3$ | $NO_2$ | |
| 4.19 | $CF_2CHF_2$ | H | H | Cl | $CF_3$ | $NO_2$ | |
| 4.20 | $CF_2CHF_2$ | H | H | H | C≡N | $NO_2$ | |
| 4.21 | $CF_2CHF_2$ | H | H | Cl | C≡N | $NO_2$ | |
| 4.22 | $OCH_3$ | $CH_3$ | H | Cl | $CF_2Cl$ | $NO_2$ | |
| 4.23 | $SCH_3$ | $SCH_3$ | H | H | $CHCl_2$ | $NO_2$ | |
| 4.24 | $CCl_3$ | $CH_3$ | H | H | $CF_3$ | $NO_2$ | 52-55 |

Tabelle 4 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 4.25 | $CCl_3$ | $CH_3$ | H | Cl | $CF_3$ | $NO_2$ | Smp. 175-177 |
| 4.26 | $CCl_3$ | $CH_3$ | H | H | C≡N | $NO_2$ | |
| 4.27 | $CCl_3$ | $CH_3$ | H | Cl | C≡N | $NO_2$ | |
| 4.28 | $CCl_3$ | $CH_3$ | H | H | $CH_3$ | $NO_2$ | |
| 4.29 | $CCl_3$ | $CH_3$ | H | H | $CO_2Et$ | $NO_2$ | |
| 4.30 | $CCl_3$ | $CH_3$ | H | H | $CCl_3$ | $NO_2$ | |
| 4.31 | $CCl_3$ | $CH_3$ | H | Cl | $CCl_3$ | $NO_2$ | |
| 4.32 | $CCl_3$ | $CH_3$ | H | H | $NO_2$ | $CF_3$ | |
| 4.33 | $CCl_3$ | $CH_3$ | H | Cl | $NO_2$ | $CF_3$ | |
| 4.34 | $CF_2CHF_2$ | $CH_3$ | H | H | $CF_3$ | $NO_2$ | |
| 4.35 | $CF_2CHF_2$ | $CH_3$ | H | Cl | $CF_3$ | $NO_2$ | |
| 4.36 | $CF_2CHF_2$ | $CH_3$ | H | H | $CCl_3$ | $NO_2$ | |
| 4.37 | $CF_2CHF_2$ | $CH_3$ | H | Cl | $CCl_3$ | $NO_2$ | |
| 4.38 | $CF_2CHF_2$ | $CH_3$ | H | H | $CH_3$ | $NO_2$ | |
| 4.39 | $CF_2CHF_2$ | $CH_3$ | H | Cl | $CO_2Et$ | $NO_2$ | |
| 4.40 | $CF_2CHF_2$ | $CH_3$ | H | H | C≡N | $NO_2$ | |
| 4.41 | $C_3F_7$ | H | H | Cl | $CF_2Cl$ | $NO_2$ | |
| 4.42 | $CH_3$ | H | H | Cl | $CF_3$ | $NO_2$ | |
| 4.43 | C≡N | H | H | H | $CO_2CH_3$ | $NO_2$ | |
| 4.44 | $C_7F_{15}$ | H | H | H | $CCl_3$ | $NO_2$ | |
| 4.45 | $CF_2CHCl_2$ | H | H | Cl | $CF_2CHF_2$ | $NO_2$ | |
| 4.46 | Cl | H | H | H | $CF_3$ | $NO_2$ | |
| 4.47 | $CCl_3$ | H | Cl | H | $CF_3$ | $NO_2$ | Smp. 97-100 |
| 4.48 | $CCl_3$ | H | Cl | Cl | $CF_3$ | $NO_2$ | Smp. 157-160 |
| 4.49 | $CCl_3$ | H | Cl | H | C≡N | $NO_2$ | |
| 4.50 | $CCl_3$ | H | Cl | Cl | C≡N | $NO_2$ | |
| 4.51 | $CCl_3$ | H | Cl | H | $CO_2Et$ | $NO_2$ | Smp. 180-182 |
| 4.52 | $CCl_3$ | H | Cl | Cl | $CO_2Et$ | $NO_2$ | |
| 4.53 | $CCl_3$ | H | Cl | H | $CH_3$ | $NO_2$ | Smp. 112-114 |
| 4.54 | $CCl_3$ | H | Cl | Cl | $CH_3$ | $NO_2$ | |

<u>Tabelle 4</u> (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 4.55 | $CCl_3$ | H | Cl | H | $CCl_3$ | $NO_2$ | Smp. 138-140 |
| 4.56 | $CCl_3$ | H | Cl | Cl | $CCl_3$ | $NO_2$ | |
| 4.57 | $CCl_3$ | H | Cl | H | 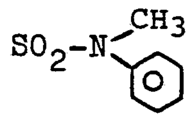 | $NO_2$ | |
| 4.58 | $CCl_3$ | H | Cl | Cl | 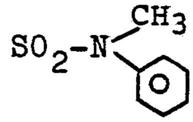 | $NO_2$ | |
| 4.59 | $CCl_3$ | H | Cl | H | $CF_2Cl$ | $NO_2$ | |
| 4.60 | $CCl_3$ | H | Cl | Cl | $CF_2Cl$ | $NO_2$ | |
| 4.61 | $CCl_3$ | H | Cl | H | $NO_2$ | $CF_3$ | |
| 4.62 | $CCl_3$ | H | Cl | Cl | $NO_2$ | $CF_3$ | |
| 4.63 | $CF_2Cl$ | H | Cl | H | C≡N | $NO_2$ | |
| 4.64 | $CF_2Cl$ | H | Cl | Cl | C≡N | $NO_2$ | |
| 4.65 | $CF_2Cl$ | H | Cl | H | $CF_3$ | $NO_2$ | |
| 4.66 | $CF_2Cl$ | H | Cl | Cl | $CF_3$ | $NO_2$ | |
| 4.67 | $CHCl_2$ | H | Cl | H | $CF_3$ | $NO_2$ | |
| 4.68 | $CHCl_2$ | H | Cl | Cl | $CF_3$ | $NO_2$ | |
| 4.69 | OEt | H | Cl | H | $CF_3$ | $NO_2$ | |
| 4.70 | $NHCH_3$ | H | Cl | Cl | $CF_3$ | $NO_2$ | |
| 4.71 | $N(CH_3)_2$ | H | Cl | Cl | C≡N | $NO_2$ | |
| 4.72 | $SCH_3$ | H | Cl | H | C≡N | $NO_2$ | |
| 4.73 | $SCH(CH_3)_2$ | H | Cl | H | $CO_2Et$ | $NO_2$ | |
| 4.74 | 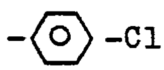 | H | Cl | Cl | $CO_2Et$ | $NO_2$ | |
| 4.75 | 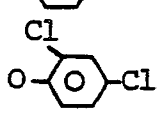 | H | Cl | H | $CF_3$ | $NO_2$ | |
| 4.76 | $CCl_2CCl_3$ | $CH_3$ | Cl | H | C≡N | $NO_2$ | |
| 4.77 | $CCl_3$ | $CH_3$ | Cl | Cl | $CF_3$ | $NO_2$ | |

Tabelle 4 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 4.78 | $CF_2CHF_2$ | $CH_3$ | Cl | H | $CO_2Et$ | $NO_2$ | |
| 4.79 | $CH_3$ | $CH_3$ | Cl | F | $CF_3$ | $NO_2$ | |
| 4.80 | $CF_3$ | H | Cl | H | C≡N | $NO_2$ | |
| 4.81 | $CF_3$ | H | Cl | Cl | $CO_2Et$ | $NO_2$ | |
| 4.82 | $CF_3$ | H | Cl | Cl | $CCl_3$ | $NO_2$ | |
| 4.83 | $CH_3$ | H | Cl | H | $CCl_3$ | $NO_2$ | |
| 4.84 | $CH_3$ | H | Cl | Cl | $CF_3$ | $NO_2$ | |
| 4.85 | $CH_3$ | H | Cl | Cl | C≡N | $NO_2$ | |
| 4.86 | $CH_3$ | H | Cl | H | $CO_2CH_3$ | $NO_2$ | |
| 4.87 | 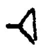 | H | Cl | H | $CO_2Et$ | $NO_2$ | |
| 4.88 | 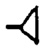 | H | Cl | Cl | $CF_2Cl$ | $NO_2$ | |
| 4.89 | 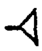 | H | Cl | Cl | C≡N | $NO_2$ | |
| 4.90 | C≡N | H | Cl | H | $CCl_3$ | $NO_2$ | |
| 4.91 | C≡N | H | Cl | Cl | $NO_2$ | $CF_3$ | |
| 4.92 | Cl | H | Cl | Cl | C≡N | $NO_2$ | |
| 4.93 | $CF_2CHF_2$ | H | Cl | H | $CF_3$ | $NO_2$ | Smp. 88-90 |
| 4.94 | $CF_2CHF_2$ | H | Cl | Cl | $CF_3$ | $NO_2$ | Smp. 80-85 |
| 4.95 | $CF_2CHF_2$ | H | Cl | H | $CCl_3$ | $NO_2$ | |
| 4.96 | $CF_2CHF_2$ | H | Cl | Cl | $CCl_3$ | $NO_2$ | |
| 4.97 | $CF_2CHF_2$ | H | Cl | H | $CH_3$ | $NO_2$ | |
| 4.98 | $CF_2CF_3$ | H | Cl | Cl | $CF_3$ | $NO_2$ | Sirup |
| 4.99 | $CF_2CHF_2$ | H | Cl | H | C≡N | $NO_2$ | |
| 4.100 | $CF_2CHF_2$ | H | Cl | Cl | C≡N | $NO_2$ | |
| 4.101 | $CF_2CHF_2$ | H | Cl | H | $CO_2Et$ | $NO_2$ | |
| 4.102 | $CF_2CHF_2$ | H | Cl | Cl | $CO_2Et$ | $NO_2$ | |
| 4.103 | $CF_2CHF_2$ | H | Cl | H | $NO_2$ | $CF_3$ | |
| 4.104 | $CF_2CHF_2$ | H | Cl | Cl | $NO_2$ | $CF_3$ | |
| 4.105 | $C_3F_7$ | H | Cl | H | $CF_3$ | $NO_2$ | |
| 4.106 | $C_3F_7$ | H | Cl | Cl | $CF_3$ | $NO_2$ | |
| 4.107 | $C_7F_{15}$ | H | Cl | H | $CF_3$ | $NO_2$ | |

Tabelle 4 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 4.108 | $C_7F_{15}$ | H | Cl | Cl | C≡N | $NO_2$ | |
| 4.109 | $CF_2CHClF$ | H | Cl | H | $CO_2Et$ | $NO_2$ | |
| 4.110 | $CF_2Cl$ | H | Cl | H | $CO_2Et$ | $NO_2$ | |
| 4.111 | $CF_2Cl$ | H | Cl | Cl | $CO_2Et$ | $NO_2$ | |
| 4.112 | $CHCl_2$ | H | Cl | H | C≡N | $NO_2$ | |
| 4.113 | $CHCl_2$ | H | Cl | Cl | C≡N | $NO_2$ | |
| 4.114 | $CF_3$ | H | Cl | H | $CF_3$ | $NO_2$ | |
| 4.115 | $CF_3$ | H | Cl | Cl | $CF_3$ | $NO_2$ | |
| 4.116 | $CCl_3$ | H | Br | H | $CF_3$ | $NO_2$ | Smp. 64-68 |
| 4.117 | $CCl_3$ | H | Br | Cl | $CF_3$ | $NO_2$ | Smp. 124-128 |
| 4.118 | $CCl_3$ | H | Br | H | C≡N | $NO_2$ | |
| 4.119 | $CCl_3$ | H | Br | Cl | C≡N | $NO_2$ | |
| 4.120 | $CCl_3$ | H | Br | H | $CO_2Et$ | $NO_2$ | |
| 4.121 | $CCl_3$ | H | Br | Cl | $CO_2Et$ | $NO_2$ | |
| 4.122 | $CCl_3$ | H | Br | H | $CH_3$ | $NO_2$ | |
| 4.123 | $CCl_3$ | H | Br | Cl | $CH_3$ | $NO_2$ | |
| 4.124 | $CCl_3$ | H | Br | H | $CCl_3$ | $NO_2$ | |
| 4.125 | $CCl_3$ | H | Br | Cl | $CCl_3$ | $NO_2$ | |
| 4.126 | OEt | OEt | Br | H | $CH_3$ | $NO_2$ | |
| 4.127 | SEt | SEt | Br | H | $SO_2NMe_2$ | $NO_2$ | |
| 4.128 | 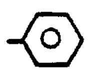 | Cl | Br | Cl | C≡N | $NO_2$ | |
| 4.129 | 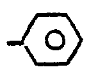 | $OCH_3$ | Br | Cl | $NO_2$ | $CF_3$ | |
| 4.130 | 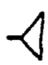 | H | Br | H | $CHCl_2$ | $NO_2$ | |
| 4.131 | 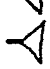 | H | Br | Cl | $CHCl_2$ | $NO_2$ | |
| 4.132 | $CHCl_2$ | Cl | Br | H | $CF_2Cl$ | $NO_2$ | |
| 4.133 | $CHCl_2$ | Cl | Br | Cl | $CF_2Cl$ | $NO_2$ | |
| 4.134 | $OCH_3$ | $CH_3$ | Br | H | $CF_3$ | $NO_2$ | |

<u>Tabelle 4</u> (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 4.135 | $CF_2CHF_2$ | H | Br | H | $CF_3$ | $NO_2$ | |
| 4.136 | $CF_2CHF_2$ | H | Br | Cl | $CF_3$ | $NO_2$ | |
| 4.137 | $CF_2CHF_2$ | H | Br | H | C≡N | $NO_2$ | |
| 4.138 | $CF_2CHF_2$ | H | Br | Cl | C≡N | $NO_2$ | |
| 4.139 | $CF_2CHF_2$ | H | Br | H | $CCl_3$ | $NO_2$ | |
| 4.140 | $CF_2CHF_2$ | H | Br | Cl | $CCl_3$ | $NO_2$ | |
| 4.141 | $CF_2CHF_2$ | H | Br | H | $CO_2Et$ | $NO_2$ | |
| 4.142 | $CF_2CHF_2$ | H | Br | Cl | $CO_2Et$ | $NO_2$ | |
| 4.143 | $CF_2CHF_2$ | H | Br | H | $CH_3$ | $NO_2$ | |
| 4.144 | $CF_2CHF_2$ | H | Br | Cl | $CH_3$ | $NO_2$ | |
| 4.145 | $CF_2CHF_2$ | H | Br | H | $NO_2$ | $CF_3$ | |
| 4.146 | $CF_2CHF_2$ | H | Br | Cl | $NO_2$ | $CF_3$ | |
| 4.147 | $CF_2CF_3$ | H | Br | H | $CF_3$ | $NO_2$ | |
| 4.148 | $CF_2CF_3$ | H | Br | Cl | $CF_3$ | $NO_2$ | |
| 4.149 | $C_3F_7$ | H | Br | Cl | C≡N | $NO_2$ | |
| 4.150 | $CH_3$ | H | Br | H | $CO_2Et$ | $NO_2$ | |
| 4.151 | 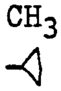 | H | Br | Cl | $CCl_3$ | $NO_2$ | |
| 4.152 | OMe | H | Br | H | C≡N | $NO_2$ | |
| 4.153 | S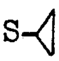 | H | Br | H | C≡N | $NO_2$ | |
| 4.154 | $CCl_3$ | H | $CH_3$ | H | $CF_3$ | $NO_2$ | |
| 4.155 | $CCl_3$ | H | $CH_3$ | Cl | $CF_3$ | $NO_2$ | |
| 4.156 | $CCl_3$ | H | $CH_3$ | H | C≡N | $NO_2$ | |
| 4.157 | $CCl_3$ | H | $CH_3$ | Cl | C≡N | $NO_2$ | |
| 4.158 | $CCl_3$ | H | $CH_3$ | H | $CCl_3$ | $NO_2$ | Smp. 115-118 |
| 4.159 | $CCl_3$ | H | $CH_3$ | H | $CH_3$ | $NO_2$ | Smp. 76-78 |
| 4.160 | $CCl_3$ | H | $CH_3$ | H | $CO_2Et$ | $NO_2$ | Smp. 111-112 |
| 4.161 | $CCl_3$ | H | $CH_3$ | Cl | $CO_2Et$ | $NO_2$ | Smp. 180-182 |
| 4.162 | $CF_2CHF_2$ | H | $CH_3$ | F | $NO_2$ | $CF_3$ | |
| 4.163 | $CF_2CHF_2$ | H | $CH_3$ | H | C≡N | $NO_2$ | |
| 4.164 | $CF_2CHF_2$ | H | $CH_3$ | Br | $CF_3$ | $NO_2$ | |

Tabelle 4 (Fortsetzung)

| Nr. | $R_1$ | $R_2$ | $R_3$ | $R_4$ | $R_5$ | $R_6$ | physik. Konstanten [°C] |
|---|---|---|---|---|---|---|---|
| 4.165 | $CF_2CHF_2$ | H | $CH_3$ | H | $CCl_3$ | $NO_2$ | |
| 4.166 | $CF_2CHF_2$ | H | $CH_3$ | Cl | $CF_2CHF_2$ | $NO_2$ | |
| 4.167 | $CF_2CF_3$ | H | $CH_3$ | H | $CF_3$ | $NO_2$ | Smp. 135-137 |
| 4.168 | $CF_2CF_3$ | H | $CH_3$ | Cl | $CF_3$ | $NO_2$ | Smp. 166-168 |
| 4.169 | $CH_3$ | H | $CH_3$ | Cl | $CCl_3$ | $NO_2$ | |
| 4.170 | 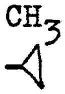 | H | $CH_3$ | H | $CF_2Cl$ | $NO_2$ | |
| 4.171 | C≡N | $CH_3$ | $CH_3$ | F | $CHF_2$ | $NO_2$ | |
| 4.172 | OMe | Et | $CH_3$ | F | $CHCl_2$ | $NO_2$ | |
| 4.173 | $CCl_3$ | H | C≡N | H | $CF_3$ | $NO_2$ | |
| 4.174 | $CCl_3$ | H | C≡N | Cl | $CF_3$ | $NO_2$ | |
| 4.175 | $CCl_3$ | H | C≡N | H | C≡N | $NO_2$ | |
| 4.176 | $CCl_3$ | H | C≡N | Cl | $CO_2Et$ | $NO_2$ | |
| 4.177 | $CCl_3$ | H | C≡N | H | $CH_3$ | $NO_2$ | |
| 4.178 | $CCl_3$ | H | F | H | $CF_3$ | $NO_2$ | |
| 4.179 | $C_2Cl_5$ | H | F | Cl | $CCl_3$ | $NO_2$ | |
| 4.180 | $CCl_2CHCl_2$ | H | F | Cl | $CO_2Et$ | $NO_2$ | |
| 4.181 | $CF_2CHCl_2$ | H | $OCH_3$ | H | $CF_3$ | $NO_2$ | |
| 4.182 | $CF_3$ | H | $OCH_3$ | Cl | $CF_2Cl$ | $NO_2$ | |
| 4.183 | $CH_2Cl$ | H | OEt | Cl | $CH_3$ | $NO_2$ | |
| 4.184 | $CCl_3$ | $CH_3$ | SEt | H | $CF_3$ | $NO_2$ | |
| 4.185 | O-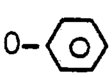-Cl | $CH_3$ | C≡N | H | $CF_3$ | $NO_2$ | |
| 4.186 | $-OCH_3$ | $CH_3$ | $NO_2$ | F | $CF_3$ | $NO_2$ | |
| 4.187 | -SEt | $CH_3$ | $NO_2$ | O-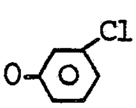 | $CF_3$ | $NO_2$ | |
| 4.188 | Cl | $CF_3$ | H | Cl | $CF_3$ | $NO_2$ | |
| 4.189 | F | $CF_3$ | $CH_3$ | H | $CF_3$ | $NO_2$ | |
| 4.190 | $CF_2CF_3$ | H | H | Cl | $CF_3$ | $NO_2$ | Smp. 115-120 |

Tabelle 5

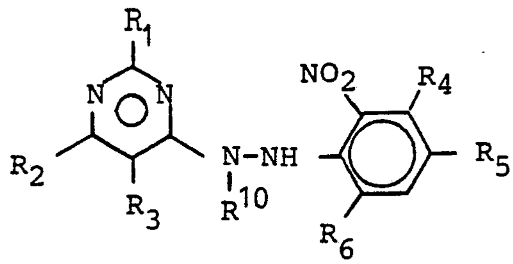

| Nr. | $R_1$ | $R_2$ | $R_3$ | $R_4$ | $R_5$ | $R_6$ | $R_{10}$ | physik. Konstanten [°C] |
|---|---|---|---|---|---|---|---|---|
| 5.1 | $CCl_3$ | H | H | H | $CF_3$ | $NO_2$ | $CH_3$ | |
| 5.2 | $CCl_3$ | H | H | Cl | $CF_3$ | $NO_2$ | $CH_3$ | |
| 5.3 | $CCl_3$ | H | H | H | $CF_3$ | $NO_2$ | Et | |
| 5.4 | $CCl_3$ | H | H | Cl | $CF_3$ | $NO_2$ | Et | |
| 5.5 | $CF_2Cl$ | H | H | H | $NO_2$ | $CF_3$ | $CH_3$ | |
| 5.6 | $CF_2Cl$ | H | H | Cl | $CF_3$ | $NO_2$ | Et | |
| 5.7 | $CCl_3$ | H | H | H | $CH_3$ | $NO_2$ | $CH_3$ | |
| 5.8 | $CCl_3$ | H | H | Cl | $CH_3$ | $NO_2$ | $CH_3$ | |
| 5.9 | $CCl_3$ | H | H | H | $CCl_3$ | $NO_2$ | $CH_3$ | |
| 5.10 | $CCl_3$ | H | H | Cl | $CCl_3$ | $NO_2$ | $CH_3$ | |
| 5.11 | $CCl_3$ | H | H | H | C≡N | $NO_2$ | $CH_3$ | |
| 5.12 | $CCl_3$ | H | H | Cl | C≡N | $NO_2$ | $CH_3$ | |
| 5.13 | $CCl_3$ | H | H | H | $CO_2Et$ | $NO_2$ | $CH_3$ | |
| 5.14 | $CF_3$ | H | H | Cl | $CO_2Et$ | $NO_2$ | $CH_3$ | |
| 5.15 | $CF_3$ | H | H | H | 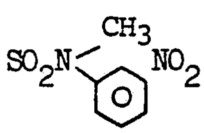 | $NO_2$ | $CH_3$ | |
| 5.16 | $CCl_3$ | $CH_3$ | H | H | $CF_3$ | $NO_2$ | $CH_3$ | Smp. 160–163 |
| 5.17 | $CCl_3$ | $CH_3$ | H | Cl | $CF_3$ | $NO_2$ | $CH_3$ | Smp. 197–199 |
| 5.18 | $CCl_3$ | $CH_3$ | H | Cl | $CO_2Et$ | $NO_2$ | $CH_3$ | Smp. 162–164 |
| 5.19 | OMe | $CH_3$ | H | H | C≡N | $NO_2$ | $CH_3$ | |
| 5.20 | SMe | $CH_3$ | H | Cl | C≡N | $NO_2$ | $CH_3$ | |
| 5.21 | 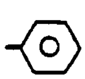 | H | H | H | $CCl_3$ | $NO_2$ | Et | |

Tabelle 5 (Fortsetzung)

| Nr. | $R_1$ | $R_2$ | $R_3$ | $R_4$ | $R_5$ | $R_6$ | $R_{10}$ | physik. Konstanten [°C] |
|---|---|---|---|---|---|---|---|---|
| 5.22 | $CH_3$ | H | H | F | $CCl_3$ | $NO_2$ | $CH_3$ | |
| 5.23 | 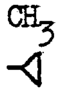 | H | H | F | $CF_2Cl$ | $NO_2$ | $CH_3$ | |
| 5.24 | $CF_2CHF_2$ | H | H | H | $CF_3$ | $NO_2$ | $CH_3$ | |
| 5.25 | $CF_2CHF_2$ | H | H | Cl | $CF_3$ | $NO_2$ | $CH_3$ | |
| 5.26 | $CF_2CHF_2$ | H | H | H | $CCl_3$ | $NO_2$ | $CH_3$ | |
| 5.27 | $CF_2CHF_2$ | H | H | Cl | $CCl_3$ | $NO_2$ | $CH_3$ | |
| 5.28 | $CF_2CHF_2$ | H | H | H | C≡N | $NO_2$ | $CH_3$ | |
| 5.29 | $CF_2CHF_2$ | H | H | H | $CO_2Et$ | $NO_2$ | $CH_3$ | |
| 5.30 | $CF_2CF_3$ | H | H | H | $CF_3$ | $NO_2$ | $CH_3$ | |
| 5.31 | $CF_2CF_3$ | H | H | Cl | $CF_3$ | $NO_2$ | $CH_3$ | |
| 5.32 | $CF_2CF_3$ | H | H | H | $CO_2Et$ | $NO_2$ | $CH_3$ | |
| 5.33 | $CF_2CF_3$ | H | H | H | C≡N | $NO_2$ | $CH_3$ | |
| 5.34 | $CF_2CHF_2$ | H | H | H | $CF_3$ | $NO_2$ | Et | |
| 5.35 | $CF_2CHF_2$ | H | H | Cl | $CF_3$ | $NO_2$ | Et | |
| 5.36 | $CF_2CF_3$ | H | H | H | $CF_3$ | $NO_2$ | Et | |
| 5.37 | $CF_2CF_3$ | H | H | Cl | $CF_3$ | $NO_2$ | Et | |
| 5.38 | $CCl_3$ | H | Cl | H | $CF_3$ | $NO_2$ | $CH_3$ | |
| 5.39 | $CCl_3$ | H | Cl | Cl | $CF_3$ | $NO_2$ | $CH_3$ | |
| 5.40 | $CCl_3$ | H | Cl | H | $CF_3$ | $NO_2$ | Et | |
| 5.41 | $CCl_3$ | H | Cl | Cl | $CF_3$ | $NO_2$ | Et | |
| 5.42 | $CCl_3$ | H | Cl | H | C≡N | $NO_2$ | $CH_3$ | |
| 5.43 | $CCl_3$ | H | Cl | Cl | C≡N | $NO_2$ | $CH_3$ | |
| 5.44 | $CCl_3$ | H | Cl | H | $CO_2Et$ | $NO_2$ | $CH_3$ | |
| 5.45 | $CCl_3$ | H | Cl | Cl | $CO_2Et$ | $NO_2$ | $CH_3$ | |
| 5.46 | $CCl_3$ | H | Cl | H | $CCl_3$ | $NO_2$ | $CH_3$ | |
| 5.47 | $CCl_3$ | H | Cl | Cl | $CCl_3$ | $NO_2$ | $CH_3$ | |
| 5.48 | $CCl_3$ | H | Cl | H | $CH_3$ | $NO_2$ | $CH_3$ | |
| 5.49 | $CCl_3$ | H | Cl | H | $NO_2$ | $CF_3$ | $CH_3$ | |
| 5.50 | $CCl_3$ | H | Cl | Cl | $NO_2$ | $CF_3$ | $CH_3$ | |
| 5.51 | $CF_2Cl$ | H | Cl | H | $CF_2Cl$ | $NO_2$ | $n\text{-}C_3H_7$ | |

Tabelle 5 (Fortsetzung)

| Nr. | $R_1$ | $R_2$ | $R_3$ | $R_4$ | $R_5$ | $R_6$ | $R_{10}$ | physik. Konstanten [°C] |
|---|---|---|---|---|---|---|---|---|
| 5.52 | $CF_2Cl$ | H | Cl | Cl | $CF_2Cl$ | $NO_2$ | n-$C_3H_7$ | |
| 5.53 | $CHCl_2$ | H | Cl | H | $CF_3$ | $NO_2$ | $CH_3$ | |
| 5.54 | $CHCl_2$ | H | Cl | Cl | $CF_3$ | $NO_2$ | $CH_3$ | |
| 5.55 | $CCl_2CCl_3$ | H | Cl | H | C≡N | $NO_2$ | $CH_3$ | |
| 5.56 | $CCl_2CCl_3$ | H | Cl | Cl | $CO_2Et$ | $NO_2$ | Et | |
| 5.57 | Cl | H | Cl | H | $CCl_3$ | $NO_2$ | $CH_3$ | |
| 5.58 | Cl | H | Cl | Cl | $CHCl_2$ | $NO_2$ | $CH_3$ | |
| 5.59 | $CH_3$ | $CH_3$ | Cl | H | $CF_2CF_3$ | $NO_2$ | $CH_3$ | |
| 5.60 | $CH_3$ | $CH_3$ | Cl | Cl | $CH_3$ | $NO_2$ | n-$C_3H_7$ | |
| 5.61 | $CF_2CHF_2$ | H | Cl | H | $CF_3$ | $NO_2$ | $CH_3$ | |
| 5.62 | $CF_2CHF_2$ | H | Cl | Cl | $CF_3$ | $NO_2$ | n-$C_3H_7$ | |
| 5.63 | $CF_2CHF_2$ | H | Cl | H | $CO_2Et$ | $NO_2$ | $CH_3$ | |
| 5.64 | $CF_2CHF_2$ | H | Cl | Cl | $CO_2Et$ | $NO_2$ | $CH_3$ | |
| 5.65 | $CF_2CHF_2$ | H | Cl | H | C≡N | $NO_2$ | $CH_3$ | |
| 5.66 | $CF_2CHF_2$ | H | Cl | Cl | C≡N | $NO_2$ | $CH_3$ | |
| 5.67 | $CF_2CHF_2$ | H | Cl | H | $CCl_3$ | $NO_2$ | $CH_3$ | |
| 5.68 | $CF_2CHF_2$ | H | Cl | Cl | $CCl_3$ | $NO_2$ | $CH_3$ | |
| 5.69 | $CF_2CF_3$ | H | Cl | H | $CF_3$ | $NO_2$ | $CH_3$ | |
| 5.70 | $CF_2CF_3$ | H | Cl | Cl | $CF_3$ | $NO_2$ | $CH_3$ | |
| 5.71 | $CF_2CF_3$ | H | Cl | H | $CCl_3$ | $NO_2$ | n-$C_4H_9$ | |
| 5.72 | $CF_2CF_3$ | H | Cl | H | $CO_2Et$ | $NO_2$ | $CH_3$ | |
| 5.73 | $CF_2CF_3$ | H | Cl | H | C≡N | $NO_2$ | $CH_3$ | |
| 5.74 | $CF_2CHF_2$ | H | Cl | H | $CF_3$ | $NO_2$ | Et | |
| 5.75 | $CF_2CHF_2$ | H | Cl | Cl | $CF_3$ | $NO_2$ | n-$C_3H_7$ | |
| 5.76 | $C_4F_9$ | H | Cl | Cl | $CF_3$ | $NO_2$ | Et | |
| 5.77 | $C_3F_7$ | H | Cl | H | $CF_3$ | $NO_2$ | $CH_3$ | |
| 5.78 | $C_3F_7$ | H | Cl | Cl | $CF_3$ | $NO_2$ | $CH_3$ | |
| 5.79 | $C_7F_{15}$ | H | Cl | Cl | $CF_3$ | $NO_2$ | $CH_3$ | |
| 5.80 | $CF_2CHClF$ | H | Cl | Cl | $CF_3$ | $NO_2$ | $CH_3$ | |
| 5.81 | $CF_3$ | H | Br | H | $CF_3$ | $NO_2$ | $C_2H_5$ | |
| 5.82 | $CF_3$ | H | Br | Cl | C≡N | $NO_2$ | $CH_3$ | |

Tabelle 5 (Fortsetzung)

| Nr. | $R_1$ | $R_2$ | $R_3$ | $R_4$ | $R_5$ | $R_6$ | $R_{10}$ | physik. Konstanten [°C] |
|---|---|---|---|---|---|---|---|---|
| 5.83 | $CF_3$ | H | Br | H | $CO_2Et$ | $NO_2$ | $CH_3$ | |
| 5.84 | $CF_3$ | H | F | Cl | $CO_2Bu$ | $NO_2$ | $CH_3$ | |
| 5.85 | $C_2H_5$ | H | $CH_3$ | H | $CF_3$ | $NO_2$ | $CH_3$ | |
| 5.86 | $C_2H_5$ | H | $CH_3$ | Cl | $CF_3$ | $NO_2$ | $CH_3$ | |
| 5.87 | $C_2H_5$ | H | $CH_3$ | H | C≡N | $NO_2$ | $CH_3$ | |
| 5.88 | $C_2H_5$ | H | $CH_3$ | Cl | $CO_2Et$ | $NO_2$ | $CH_3$ | |
| 5.89 | $CH_3$ | H | $CH_3$ | H | $CCl_3$ | $NO_2$ | $CH_3$ | |
| 5.90 | $CH_3$ | H | $CH_3$ | Cl | $CH_3$ | $NO_2$ | $CH_3$ | |
| 5.91 | 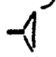 | H | F | H | $CF_3$ | $NO_2$ | $CH_3$ | |
| 5.92 | 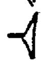 | H | F | Cl | C≡N | $NO_2$ | $CH_3$ | |
| 5.93 | $OCF_3$ | H | Et | H | $CO_2Et$ | $NO_2$ | $CH_3$ | |
| 5.94 | 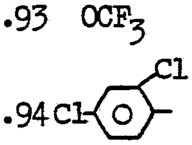 | H | Et | Cl | $CCl_3$ | $NO_2$ | Et | |
| 5.95 | $OCH_3$ | H | $OCH_3$ | H | $CF_3$ | $NO_2$ | $CH_3$ | |
| 5.96 | 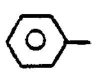 | H | C≡N | H | $CF_3$ | $NO_2$ | $CH_3$ | |
| 5.97 | $CF_2Cl$ | H | C≡N | Cl | $CCl_3$ | $NO_2$ | $CH_3$ | |
| 5.98 | $CF_2Cl$ | H | C≡N | H | $CO_2Et$ | $NO_2$ | $CH_3$ | |
| 5.99 | $CF_2Cl$ | H | C≡N | Cl | $CH_3$ | $NO_2$ | $CH_3$ | |
| 5.100 | $CH_2Cl$ | H | 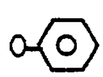 | Cl | $CCl_3$ | $NO_2$ | $CH_3$ | |
| 5.101 | $CCl_3$ | H | C≡N | H | $CF_3$ | $NO_2$ | $CH_3$ | |
| 5.102 | $CCl_3$ | H | C≡N | Cl | $CF_3$ | $NO_2$ | $CH_3$ | |
| 5.103 | $CCl_3$ | $CH_3$ | H | H | $CO_2Et$ | $NO_2$ | $CH_3$ | Smp. 148-150 |
| 5.104 | $CCl_3$ | $CH_3$ | H | H | C≡N | $NO_2$ | $CH_3$ | Smp. 216-218 |

C. Biologische Beispiele

1. Fungizide wirkung

Beispiel 1

Biomalzagar wurde mit den beanspruchten Verbindungen (Tabelle 1) in Konzentrationen von jeweils 2000 und 500 ppm Wirkstoff versetzt. Nach dem Erstarren des Agars erfolgte die Beimpfung mit verschiedenen Botrytis cinereaKulturen, die Benzimidazol-und Iprodion-sensibel bzw. -resistent sind. Jeweils 20 µl einer Sporensuspension wurden auf das Zentrum der Agarplatte aufgebracht. Die Versuche wurden 6 Tage nach Beimpfung ausgewertet. Der Wirkungsgrad der Wirkstoffe wird ausgedrückt in % im Vergleich zur Kontrolle (Agarmedium ohne Wirkstoff).

Tabelle 1

| Verbindung gemäß Beispiel Nr. | Botrytis cinerea, BCM- und Iprodion-sensibler (s) und -resistenter (r)-Stamm<br>Wirkungsgrad in % bei ppm Wirkstoff | | | |
|---|---|---|---|---|
| | 2000 | | 500 | |
| | s | r | s | r |
| 2.10 | 100 | 100 | 50 | 80 |
| 2.12 | 100 | 100 | 100 | 100 |
| 2.9 | 100 | 100 | 50 | 80 |
| 2.7 | 100 | 80 | 50 | 80 |
| 2.5 | 100 | 100 | 80 | 50 |
| 4.1 | 100 | 100 | 50 | 50 |
| 4.47 | 100 | 100 | 100 | 100 |
| 4.116 | 100 | 100 | 100 | 100 |
| 4.24 | 100 | 80 | 50 | 80 |
| 4.2 | 100 | 100 | 100 | 100 |
| 4.48 | 100 | 100 | 100 | 100 |
| 4.117 | 100 | 100 | 100 | 100 |
| 4.53 | 100 | 100 | 100 | 50 |
| 4.55 | 100 | 50 | 100 | 50 |
| 4.159 | 100 | 100 | 100 | 80 |
| 4.157 | 100 | 100 | 100 | 100 |
| 4.98 | 100 | 100 | 100 | 100 |
| 4.190 | 100 | 100 | 100 | 80 |
| 4.168 | 100 | 100 | 100 | 100 |
| 4.167 | 100 | 100 | 50 | 100 |
| 4.93 | 100 | 100 | 50 | 80 |
| 2.19 | 100 | 100 | 80 | 100 |
| 5.17 | 100 | 100 | 100 | 100 |
| 2.12 | 100 | 100 | 80 | 50 |
| 3.5 | 100 | 100 | 100 | 100 |
| 3.1 | 100 | 100 | 50 | 100 |
| 3.10 | 100 | 100 | 50 | 100 |
| 3.6 | 100 | 100 | 100 | 100 |
| 3.13 | 100 | 100 | 100 | 100 |
| 3.27 | 100 | 100 | 100 | 100 |
| 3.8 | 100 | 100 | 100 | 100 |
| 3.26 | 100 | 100 | 100 | 100 |
| 3.51 | 100 | 100 | 100 | 100 |
| 3.50 | 100 | 100 | 100 | 100 |
| 3.48 | 100 | 100 | 100 | 100 |
| 2.51 | 100 | 100 | - | - |
| 2.74 | 100 | 100 | 100 | 100 |
| 11.74 | 100 | 100 | 100 | 100 |
| 2.18 | 100 | 100 | 100 | 100 |
| 3.48 | 100 | 100 | 100 | 100 |
| 4.94 | 100 | 100 | 100 | 100 |
| 4.168 | 100 | 100 | 100 | 100 |
| Kontrolle | | 0 | | |

Beispiel 2

Biomalzagar wurde mit den beanspruchten Verbindungen (Tabelle 2) in Konzentrationen von jeweils 125, 30 und 8 ppm Wirkstoff versetzt. Nach dem Erstarren des Agars erfolgte die Beimpfung mit Pseudocercosporella-Kulturen (BCM-sensibel und BCM-resistent).

Jeweils 20 µl einer Sporensuspension wurden auf das Zentrum der Agarplatte aufgebracht. Die Versuche wurden 6 Tage nach Beimpfung ausgewertet. Der Wirkungsgrad der Wirkstoffe wird ausgedrückt in % im Vergleich zur Kontrolle (Agarmedium ohne Wirkstoff).

Tabelle 2a

| Verbindung gemäß Beispiel Nr. | Wirkungsgrad gegenüber Pseudocercosporella herpotrichoides in % bei ppm Wirkstoff (BCM: sensibler Stamm) 125 | 30 | 8 |
|---|---|---|---|
| 2.9 | 100 | 100 | 100 |
| 2.7 | 100 | 100 | 100 |
| 2.5 | 100 | 100 | 80 |
| 2.8 | 100 | 100 | 80 |
| 2.10 | 100 | 100 | 80 |
| 2.12 | 100 | 100 | 100 |
| 4.1 | 100 | 80 | |
| 4.47 | 100 | 100 | 100 |
| 4.116 | 100 | 100 | 100 |
| 4.24 | 100 | 100 | 80 |
| 4.2 | 100 | 100 | 100 |
| 4.117 | 100 | 100 | 100 |
| 4.51 | 100 | 80 | - |
| 4.53 | 100 | 100 | 80 |
| 4.55 | 100 | 100 | 80 |
| 4.159 | 100 | 80 | - |
| 4.157 | 100 | 100 | 100 |
| 4.190 | 100 | 100 | 80 |
| 4.167 | 100 | 100 | - |
| 4.93 | 100 | 100 | 100 |
| 2.19 | 100 | 100 | 100 |
| 5.17 | 100 | 100 | 100 |
| 3.5 | 100 | 100 | - |
| 3.1 | 100 | 100 | - |
| 3.6 | 100 | 100 | - |
| 3.13 | 100 | 100 | - |
| 3.27 | 100 | 100 | - |
| 3.8 | 100 | 100 | - |
| 3.26 | 100 | 100 | - |
| 3.9 | 100 | 80 | |
| 4.48 | 100 | 100 | 100 |
| 4.158 | 100 | 100 | 100 |
| Kontrolle | | 0 | |

Tabelle 2b

| Verbindung gemäß Beispiel Nr. | Wirkungsgrad gegenüber Pseudocerosporella herpotrichoides in % bei ppm Wirkstoff | | | | | |
|---|---|---|---|---|---|---|
| | BCM-sensibler Stamm | | | BCM-resistenter Stamm | | |
| | 125 | 30 | 8 | 125 | 30 | 8 |
| 3.8 | 100 | 100 | 100 | 100 | 100 | 100 |
| 3.50 | 100 | 100 | 100 | 100 | 100 | 100 |
| 1.32 | 100 | 100 | 100 | 100 | 100 | 100 |
| 1.48 | 100 | 100 | 100 | 100 | 100 | 100 |
| 1.179 | 100 | 100 | 100 | 100 | 100 | 100 |
| 3.48 | 100 | 100 | 100 | 100 | 100 | 100 |
| 2.46 | 100 | 100 | 100 | 100 | 100 | 100 |
| 2.51 | 100 | 100 | 100 | - | - | - |
| 2.50 | 100 | 100 | 100 | 100 | 100 | 100 |
| 1.209 | 100 | 100 | 100 | 100 | 100 | 100 |
| 1.213 | 100 | 100 | 100 | - | - | - |
| 1.46 | 100 | 100 | 100 | 100 | 100 | 100 |
| 1.214 | 100 | 100 | 100 | - | - | - |
| 1.192 | 100 | 100 | 100 | - | - | - |
| 1.92 | 100 | 100 | 100 | 100 | 100 | 100 |
| 1.268 | 100 | 100 | 100 | 100 | 100 | 100 |
| 1.200 | 100 | 100 | 100 | 100 | 100 | 100 |
| 1.139 | 100 | 100 | 100 | 100 | 100 | 100 |
| 1.49 | 100 | 100 | 100 | 100 | 100 | 100 |
| 2.56 | 100 | 100 | 100 | - | - | - |
| 2.7 | 100 | 100 | 100 | - | - | - |
| 4.94 | 100 | 100 | 100 | - | - | - |
| 4.93 | 100 | 100 | 100 | - | - | - |
| 2.77 | 100 | 100 | 100 | - | - | - |
| 4.98 | 100 | 100 | 100 | - | - | - |
| 4.168 | 100 | 100 | 100 | 100 | 100 | 100 |
| Kontrolle | | | | 0 | | |

Beispiel 3

Weinpflanzen, die aus Stecklingen der Plasmopara-anfälligen Sorte Müller-Thurgau gezogen waren, wurden im 4-Blattstadium mit wäßrigen Suspensionen der beanspruchten Verbindungen tropfnaß behandelt. Die Anwendungskonzentrationen betrugen 500, 250 und 125 mg Wirkstoff pro Liter Spritzbrühe.

Nach dem Antrocknen des Spritzbelages wurden die Pflanzen mit einer Zoosporangiensuspension von Plasmopara viticola inokuliert und tropfnaß in eine Klimakammer bei einer Temperatur von ca. 20°C und einer relativen Luftfeuchtigkeit von ca. 100 % gestellt. Nach 24 Stunden wurden die infizierten Pflanzen der Klimakammer entnommen und in ein Gewächshaus mit einer Temperatur von ca. 23°C und einer Luftfeuchtigkeit von ca. 80 - 90 % gebracht.

Nach einer Inkubationszeit von 7 Tagen wurden die Pflanzen über Nacht in die Klimakammer gestellt und die Krankheit zum Ausbruch gebracht. Anschließend erfolgte die Befallsauswertung. Der Befallsgrad wurde in % befallener Blattfläche im Vergleich zu den unbehandelten, infizierten Kontrollpflanzen ausgedrückt und ist in Tabelle 3 wiedergegeben.

Tabelle 3

| Verbindung gemäß Beispiel Nr. | % Plasmopora viticola - Befall bei ppm Wirkstoff | | |
|---|---|---|---|
| | 500 | 250 | 125 |
| 1.60 | 0 | 0 | 0 |
| 2.51 | 0 | 0 | 0 |
| 2.50 | 0 | 0 | 0 |
| 1.190 | 0 | 0 | 0 |
| 1.48 | 0 | 0 | 0 |
| 1.179 | 0 | 0 | 0 |
| 1.213 | 0 | 0 | 0 |
| 1.8 | 0 | 0 | 0 |
| 1.46 | 0 | 0 | 0 |
| 1.214 | 0 | 0 | 0 |
| 1.643 | 0 | 0 | 0 |

Fortsetzung Tabelle 3

| Verbindung gemäß Beispiel Nr. | % Plasmopora viticola - Befall bei ppm Wirkstoff | | |
|---|---|---|---|
| | 500 | 250 | 125 |
| 1.192 | 0 | 0 | 0 |
| 1.92 | 0 | 0 | 0 |
| 1.251 | 0 | 0 | 0 |
| 1.250 | 0 | 0 | 0 |
| 1.49 | 0 | 0 | 0 |
| 4.93 | 0 | 0 | 0 |
| 2.77 | 0 | 0 | 0 |
| 4.98 | 0 | 0 | 0 |
| unbehandelte infizierte Pflanzen | | 100 | |

Beispiel 4

Apfelunterlagen (EM IX) wurden im 4-Blattstadium mit den beanspruchten Verbindungen in den Anwendungskonzentrationen von 500, 250 und 125 mg Wirkstoff/Liter Spritzbrühe gleichmäßig behandelt.

Nach Antrocknen des Wirkstoffbelages wurden Pflanzen mit Konidien des Apfelschorfs (Venturia inaequalis) stark inokuliert und tropfnaß in eine Klimakammer gestellt, deren Temperatur ca. 22°C und deren relative Luftfeuchtigkeit ca. 100 % betrug. Nach einer Infektionszeit von 48 Stunden kamen die Pflanzen in ein Gewächshaus mit ca. 18°C und einer relativen Luftfeuchtigkeit von 95-100 %.

Nach einer Inkubationszeit von 14 Tagen wurden die Pflanzen auf Befall mit Apfelschorf (Venturia inaequalis) untersucht. Die Beurteilung des Befalls erfolgte wie üblich nach Augenschein. Der Befallsgrad der Pflanzen mit Apfelschorf wurde in % befallener Blattfläche, bezogen auf unbehandelte, infizierte Pflanzen, ausgedrückt und ist in Tabelle 4 wiedergegeben.

Tabelle 4

| Verbindung gemäß Beispiel Nr. | % Schorfbefall bei mg Wirkstoff/ Liter Spritzbrühe | | |
|---|---|---|---|
| | 500 | 250 | 125 |
| 1.209 | 0 | 0 | 0 |
| 1.213 | 0 | 0 | 0 |
| unbehandelte infizierte Pflanzen | | 100 | |

Beispiel 5

Weizenpflanzen werden im 3-Blattstadium mit Konidien des Weizenmehltaus (Erysiphe graminis) stark inokuliert und in einem Gewächshaus bei 20°C und einer relativen Luftfeuchte von 90-95 % aufgestellt. 3 Tage nach Inokulation werden die Pflanzen mit den in Tabelle 5 aufgeführten Verbindungen und Wirkstoffkonzentrationen behandelt. Nach einer Inkubationszeit von 10 Tagen werden die Pflanzen auf Befall mit Weizenmehltau untersucht. Der Befallsgrad wird ausgedrückt in % befallener Blattfläche, bezogen auf unbehandelte infizierte Kontrollpflanzen (= 100 % Befall). Das Ergebnis ist in der Tabelle 5 zusammengefaßt.

Tabelle 5

| Verbindung gemäß Beispiel Nr. | mit Weizenmehltau befallene Blattfläche in % bei mg Wirkstoff/Liter Spritzbrühe | | |
|---|---|---|---|
| | 500 | 250 | 125 |
| 2.77 | 0 | 0 | 0 |
| 1.201 | 0 | 0 | 0 |
| unbehandelte infizierte Pflanzen | | | 100 |

Beispiel 6

Gurkenpflanzen (Sorte Delikateß) werden im 2-Blattstadium mit einer Konidiensuspension von Gurkenmehltau (Erysiphe cichoracearum) stark inokuliert. Nach einer Abtrocknungszeit der Sporensuspension von 30 Minuten werden die Pflanzen in einem Gewächshaus bei 22°C und 90 % relativer Luftfeuchte aufgestellt.

3 Tage nach Infektion werden die Pflanzen mit den in Tabelle 6 genannten Verbindungen und Wirkstoffkonzentrationen behandelt.

Nach 10 Tagen erfolgt die Bonitur. Der Beffalsgrad wird ausgedrückt in % befallener Blattfläche, bezogen auf unbehandelte, infizierte Kontrollpflanzen (= 100 % Befall). Das Ergebnis ist in Tabelle 6 zusammengefaßt.

Tabelle 6

| Verbindung gemäß Beispiel Nr. | mit Gurkenmehltau befallene Blattfläche in % bei mg Wirkstoff/Liter Spritzbrühe | | |
|---|---|---|---|
| | 500 | 250 | 125 |
| 1.201 | 0 | 0 | 0 |
| unbehandelte infizierte Pflanzen | | | 100 |

Beispiel 7

Synergistische Wirkung am Beispiel von Iprodion

Kreuzstreifen-Test (Methode in J. Gen. Microbiol. 126 (1981), Seite 1-7) in Petrischalen.
Synergistische Beziehungen zwischen verschiedenen Wirkstoffen künnen in einem in vitro Versuch durch den sogenannten Kreuzstreifen-Test bestimmt werden.

Filterpapierstreifen (10 mm breit und 90 mm lang) werden mit den formulierten Wirkstoffen der Formel I und dem Kombinationspartner (Iprodion) in verschiedenen Konzentrationen gleichmäßig benetzt (ca. 200 µl/Streifen) und auf ein je nach Pilzart unterschiedliches Agar-Medium aufgelegt. Dem Agar wurden zuvor in noch flüssigem Zustand je Petrischale 0,5 ml Suspensionskultur des Testortganismus (ca. $10^5$ -$10^6$ Konidien/1 ml) zugegeben. Jede Petrischale enthält einen Filterpapierstreifen mit der je einem Vertreter der Formel I und senkrecht dazu einen zweiten Streifen mit dem Kombinationspartner (Iprodion) die Wirkstoffkonzentrationen sind durch entsprechende Vorversuche mit den Einzelkomponenten so gewählt, daß sie gegenüber dem betreffenden Testorganismus der sogenannten minimalen Hemmstoffkonzentration entsprechen.

Nach ca. 3-bis 4-tägiger Inkubation der Petrischalen bei 22 - 25°C werden die Inhibitionszonen im Bereich der sich kreuzenden Teststreifen diametral ausgemessen und die Hemmzone als Maß für den Synergismus bewertet.

Tabelle 7

Kreuzstreifen-Test
Testobjekt: Botrytis cinerea

| Wirkstoffe bzw. Kombinationen (Bsp. Nr.) | | Konz. Wirkstoff ppm | Hemmzonen in mm |
|---|---|---|---|
| 4.117 | a) | 500 | 0 |
| | b) | 250 | 0 |
| Iprodion | c) | 500 | 8 |
| | d) | 250 | 0 |
| Wirkstoffkombinationen aus 4.117 + Iprodion | a + c | 500 + 500 | 16 |
| | a + d | 500 + 250 | 8 |
| | b + c | 250 + 500 | 10 - 12 |
| | b + d | 250 + 250 | 6 |

2. Insektizide Wirkung

Beispiel 8

Mit Bohnenspinnmilben (Tetranychus urticae, Vollpopulation) stark befallene Bohnenpflanzen (Phaseolus v.) wurden mit der wäßrigen Verdünnung eines Emulsionskonzentrates, das 100 ppm, des jeweiligen Wirkstoffes enthielt, gespritzt.

Die Mortalität der Milben wurde nach 7 Tagen kontrolliert. 100 % Abtötung wurde den Verbindungen gemäß Beispiel 1.643; 1.200; 1.251; 1.471; 1.481 und 1.291 erzielt.

Beispiel 9

Mit Obstbaumspinnmilben (Panonychus ulmi) stark befallene Apfelbäumchen (Malus communis) wurden wie im Beispiel 1 behandelt.

Die Mortalität der Milben wurde nach 8 Tagen kontrolliert. 100 % Abtötung wurde mit den Verbindungen gemäß Beispiel 1.92; 1.200 und 1.481 erzielt.

Beispiel 10

Mit Kundebohnenblattlaus (Aphis craccivora) stark besetzte Ackerbohnen (Vivia fabe) wurden it wäßrigen Verdünnungen von Spritzpulverkonzentraten mit 1000 ppm Wirkstoffgehalt bis zum Stadium des beginnenden Abtropfens besprüht.

Die Mortalität der Blattläuse wurde nach 3 Tagen bestimmt. Eine 100 %ige Abtötung konnte mit den Verbindungen gemäß Beispiel 1.46, 1.214, 1.643, 1.192, 1.200, 1.201, 1.250, 1.291 und 1.471 erzielt werden.

Beispiel 11

Mit Weißer Fliege (Trialeurodes vaporariorum) stark besetzte Bohnenpflanzen wurden mit wäßrigen Suspensionen von Spritzpulverkonzentraten (1000 ppm Wirkstoffgehalt) bis zum beginnenden Abtropfen gespritzt. Nach Aufstellung der Pflanzen im Gewächshaus erfolge nach 14 Tagen die mikroskopische Kontrolle mit dem Ergebnis jeweils 100 %iger Mortalität bei den Präparaten mit den Wirkstoffen der Beispiele 2.51, 2.50, 1.189 und 1.200.

Beispiel 12

Auf die Innenseite des Deckels und des Bodens einer Petrischale werden jeweils 1 ml der zu testenden Formulierung emulgiert in Wasser gleichmäßig aufgetragen und nach dem Abtrocknen des belages jeweils 10 Imagines der Hausfliege (Musca domestica) eingegeben. Nach dem Verschließen der Schale werden diese bei Raumtemperatur aufbewahrt und nach 3 Stunden die Mortalität der Versuchstiere bestimmt.
Bei 1000 ppm (bezogen auf den Gehalt an Wirkstoff) zeigten die Präparate 1.255, 1.2, 1.256, 1.8, 1.298, 1.214, 1.643, 1.192, 1.92, 1.442, 1.140, 1.267, 1.268, 1.200, 1.201, 1.222, 1.223, 1.197, 1.215, 1.10, 1.38, 1.4, 1.139, 3.28, 1.251, 1.1, 1.250, 1.101, 1.291, 1.471, 1.470, 1.481 und 1.284 eine 100%-Wirkung.

Beispiel 13

In wie in Beispiel 12 behandelte Petrischale werden je 10 Larven (L4) der Deutschen Schabe (Blatella germanica) gesetzt, die Schalen verschlossen und 5 Tagen die Mortalität der Versuchstiere bestimmt.
Bei 1000 ppm (bezogen auf den Gehalt an Wirkstoff) zeigten die Präparate 1.194, 1.125, 1.256, 1.8, 1.298, 1.643, 1.442, 1.200, 1.222, 1.4, 1.139, 1.251, 1.284 und 1.2 100 % Wirkung.

**Ansprüche**

1. Verbindungen der Formel I oder deren Salze,

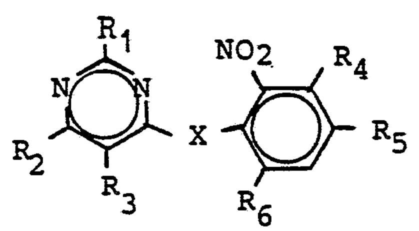

(I),

worin

$R_1$ und $R_2$ unabhängig voneinander Wasserstoff; Halogen, Cyano; ($C_1$-$C_8$)-Alkyl; ($C_1$-$C_8$)-Haloalkyl; ($C_1$-$C_8$)-Alkyl, das ein-oder zweifach durch Nitro, Cyano, ($C_1$-$C_4$)-Alkoxy, ($C_1$-$C_4$)-Alkylthio oder -N($R_7$)($R_8$) substituiert ist; ($C_3$-$C_8$)-Cycloalkyl, das durch ($C_1$-$C_4$)Alkyl substituiert sein kann; ($C_2$-$C_4$)-Alkenyl; ($C_2$-$C_4$)-Haloalkenyl; ($C_3$-$C_4$)-Alkinyl; ($C_3$-$C_4$)-Haloalkinyl; ($C_5$-$C_6$)-Cycloalkenyl; eine Gruppe -N($R_7$)($R_8$); ($C_1$-$C_8$)-Alkylthio; ($C_1$-$C_8$)-Alkoxy; ($C_1$-$C_8$)Haloalkoxy; ($C_1$-$C_8$)Alkylsulfinyl; ($C_1$-$C_8$)-Alkylsulfonyl; Phenyl, das gegebenenfalls durch Halogen, Nitro, Cyano, ($C_1$-$C_4$)-Alkyl, ($C_1$-$C_4$)-Alkoxy, ($C_1$-$C_4$)-Alkylthio oder ($C_1$-$C_4$)-Haloalkyl substituiert ist; oder Phenoxy, das gegebenenfalls durch Halogen, $NO_2$, ($C_1$-$C_4$)Alkyl, ($C_1$-$C_4$)Haloalkyl oder ($C_1$-$C_4$)Alkoxy substituiert ist;

$R_3$ Wasserstoff, Halogen, Cyano, Nitro, ($C_1$-$C_8$)-Alkyl, ($C_1$-$C_8$)-Haloalkyl, ($C_2$-$C_4$)-Alkenyl, ($C_2$-$C_4$)-Haloalkenyl, Phenyl, das gegebenenfalls durch Halogen, Nitro, Cyano, ($C_1$-$C_4$)-Alkyl, ($C_1$-$C_4$)-Alkoxy, ($C_1$-$C_4$)-Alkylthio oder ($C_1$-$C_4$)-Haloalkyl substituiert ist, oder Phenoxy, das gegebenenfalls durch Halogen, $NO_2$, ($C_1$-$C_4$)Alkyl, ($C_1$-$C_4$)Haloalkyl, ($C_1$-$C_4$)Alkoxy substituiert ist;

$R_4$ Wasserstoff, Halogen, ($C_1$-$C_4$)Alkoxy, ($C_1$-$C_4$)Alkylthio, eine Gruppe N($R_7$)($R_8$) oder Phenoxy, das durch Halogen substituiert sein kann;

$R_5$ ($C_1$-$C_4$)Alkyl, ($C_1$-$C_8$-Alkoxycarbonyl), ($C_1$-$C_4$-Alkenyloxy)carbonyl, -$CO_2H$,($C_3$-$C_4$-Alkinyloxy)carbonyl; ($C_1$-$C_4$-Haloalkoxy)carbonyl; ($C_2$-$C_4$-Haloalkenyloxy)carbonyl; ($C_3$-$C_4$-Haloalkinyloxy)carbonyl; -CON($R_7$)($R_8$); ($C_1$-$C_4$-Alkyl)carbonyl; ($C_2$-$C_4$-Alkenyl)carbonyl; ($C_3$-$C_4$-Alkinyl)carbonyl; ($C_3$-$C_4$-Haloalkyl)carbonyl; ($C_2$-$C_4$-Haloalkenyl)carbonyl, ($C_3$-$C_4$-Haloalkinyl)carbonyl, S(O) $_n R_9$; $S(O)_nOR_9$ oder ($S(O)_nN(R_7)(R_8)$; Halogen, Cyano, Nitro; ($C_1$-$C_4$)Haloalkoxy; ($C_1$-$C_4$)Haloalkyl; Formyl;

n eine der Zahlen O, 1 oder 2;
$R_6$ $NO_2$ oder $CF_3$;
$R_7$ und $R_8$ unabhängig voneinander Wasserstoff, ($C_1$-$C_4$)-Alkyl; ($C_3$-$C_7$)-Cycloalkyl, das durch ($C_1$-$C_4$)-alkyl substituiert sein kann; oder Phenyl, das durch Halogen, ($C_1$-$C_4$)-Alkyl, ($C_1$-$C_4$)-Haloalkyl, ($C_1$-$C_4$)-Alkoxy; ($C_1$-$C_4$)Alkylthio, Nitro oder Cyano substituiert sein kann;
$R_9$ ($C_1$-$C_4$)-Alkyl; ($C_2$-$C_4$)-Alkenyl, ($C_2$-$C_4$)-Alkinyl oder ($C_3$-$C_7$)-Cycloalkyl, das durch ($C_1$-$C_4$)-Alkyl substituiert sein kann;
$R_{10}$ unabhängig voneinander Wasserstoff; ($C_1$-$C_4$)-Alkyl; ($C_3$-$C_7$)-Cycloakyl, das durch ($C_1$-$C_4$)-Alkyl substituiert sein kann; ($C_1$-$C_4$)Alkoxy)carbonyl, ($C_1$-$C_4$-Alkyl)carbonyl; ($C_1$-$C_4$)-Alkoxy; Phenyl, das gegebenenfalls durch Halogen, Nitro, Cyano, ($C_1$-$C_4$)-Alkyl, ($C_1$-$C_4$)-Alkoxy, ($C_1$-$C_4$)-Alkylthio oder ($C_1$-$C_4$)-Haloalkyl substituiert sein kann, und
X eine der Gruppen -$NR_{10}$-$NR_{10}$, -$NR_{10}$-oder -O-bedeuten mit der Maßgabe, daß diejenigen Verbindungen der Formel I, worin X eine Gruppe -$NR_{10}$-und $R_5$ $CF_3$ oder $NO_2$ bedeuten, ausgenommen sind.

2.Verbindungen der Formel I von Anspruch 1, wobei $R_1$ Wasserstoff; Halogen; Cyano; ($C_1$-$C_8$)-Alkyl; ($C_1$-$C_8$)-Haloalkyl; ($C_1$-$C_8$)-Alkyl, das ein-oder zweifach durch ($C_1$-$C_4$)-Alkoxy oder ($C_1$-$C_4$)-Alkylthio substituiert ist; ($C_3$-$C_8$)-Cycloalkyl, das durch ($C_1$-$C_4$)Alkyl substituiert sein kann; ($C_1$-$C_8$)-Alkylthio; ($C_1$-$C_8$)-Alkoxy; ($C_1$-$C_8$)-Alkylsulfinyl; ($C_1$-$C_8$)-Alkylsulfonyl; Phenyl, das gegebenenfalls ein-oder mehrfach durch Halogen und/oder ein-oder dreifach durch Nitro, Cyano, ($C_1$-$C_4$)-Alkyl, ($C_1$-$C_4$)-Alkoxy oder ($C_1$-$C_4$)-Haloalkyl substituiert ist,

$R_2$ die Bedeutung von $R_1$ außer gegebenenfalls substituiertes Phenyl,

$R_3$ Wasserstoff; Halogen; Cyano; Nitro; ($C_1$-$C_8$)-Alkyl; ($C_1$-$C_8$)-Haloalkyl oder Phenoxy, das gegebenenfalls ein-bis dreimal durch Halogen $NO_2$, ($C_1$-$C_4$)Alkyl, ($C_1$-$C_4$)Haloalkyl, oder ($C_1$-$C_4$)Alkoxy substituiert ist; $R_4$ Wasserstoff; Halogen; ($C_1$-$C_4$)Alkoxy;

$R_5$ ($C_1$-$C_4$)Alkyl, ($C_1$-$C_4$-Alkoxy)carbonyl, -$CO_2H$; -CON($R_7$)($R_8$); ($C_1$-$C_4$-Alkyl)carbonyl; ($C_3$-$C_4$-Haloalkyl)-carbonyl; Cyano, Halogen, Nitro; oder $S(O)_nN(R_7)(R_8)$;
n eine der Zahlen 0, 1 oder 2;
$R_6$ $NO_2$ oder $CF_3$;
$R_7$ und $R_8$ unabhängig voneinander Wasserstoff; ($C_1$-$C_4$)-Alkyl; ($C_3$-$C_7$)-Cycloalkyl, das durch ($C_1$-$C_4$)-Alkyl substituiert sein kann; oder Phenyl, das ein-bis dreifach durch Halogen, ($C_1$-$C_4$)-Alkyl, ($C_1$-$C_4$)-Haloalkyl oder ($C_1$-$C_4$)-Alkoxy, substituiert sein kann;
$R_{10}$ unabhängig von einander Wasserstoff; ($C_1$-$C_4$)-Alkyl; und
X eine der Gruppen -$NR_{10}$-$NR_{10}$ oder -$NR_{10}$-oder -O-bedeuten sowie deren Salze.

3. Verbindungen der Formel von Anspruch 1, wobei
$R_1$ ($C_1$-$C_8$)Haloalkyl, ($C_1$-$C_4$)-Alkyl; ($C_3$-$C_6$)-Cycloalkyl, Phenyl oder Phenyl, das wie oben angegeben substituiert ist, insbesondere hiervon ($C_1$-$C_8$)-Haloalkyl,
$R_2$ H oder ($C_1$-$C_4$)-Alkyl,
$R_3$ H oder Halogen,
$R_4$ H oder Halogen,
$R_5$ ($C_1$-$C_4$)Alkoxycarbonyl, Cyano; ($C_1$-$C_4$)-Haloalkoxy, ($C_1$-$C_4$)-Haloalkyl oder Chlor,
$R_6$ $NO_2$ und X = NH oder -O-bedeuten sowie deren Salze.

4. Verfahren zur Herstellung der Verbindungen der Formel I dadurch gekennzeichnet, daß man eine Verbindung der Formel II mit einer Verbindung der Formel III

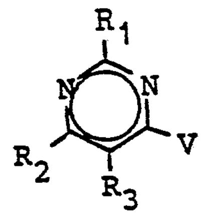
(II)

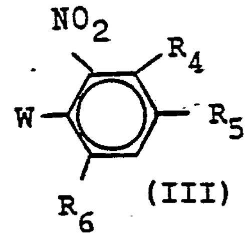
(III)

worin die Reste V, W für Halogen, Hydroxy, eine Gruppe $NR_{10}NH_2$ oder $-NHR_{10}$ stehen, wobei für den Fall, daß W für Halogen steht, V Hydroxy, eine Gruppe $-NR_{10}-NHR_{10}$ oder eine Gruppe $-NHR_{10}$ bedeuten muß und für den Fall, daß V für Halogen steht, W Hydroxy, eine Gruppe $-NR_{10}NHR_{10}$ oder eine Gruppe $-NHR_{10}$ bedeuten muß
in Gegenwart einer Base umsetzt und die erhaltenen Verbindungen gegebenenfalls in ihre Salze überführt.

5. Schädlingsbekämpfungsmittel, dadurch gekennzeichnet, daß es eine Verbindung der Formel I oder deren Salze von Ansprüchen 1, 2 oder 3 in einer wirksamen Menge neben geeigneten Formulierungshilfsmitteln enthält.

6. Fungizide Mittel, dadurch gekennzeichnet, daß es eine Verbindung der Formel I oder deren Salz von Ansprüchen 1,2 oder 3 in einer wirksamen Menge neben geeigneten Formulierungshilfsmitteln enthält.

7. Verwendung der Verbindungen der Formel I oder deren Salze von Ansprüchen 1, 2 oder 3 zur Bekämpfung von tierischen Schädlingen oder Schadpilzen.

8. Verfahren zur Bekämpfung von tierischen Schädlingen oder Schadpilzen, dadurch gekennzeichnet, daß man auf diese oder die von ihnen befallenen Pflanzen, oder Anbauflächen eine wirksame Menge einer Verbindung der Formel I oder deren Salze von Ansprüchen 1, 2 oder 3 appliziert.

Patentansprüche für folgende Vertragsstaaten : AT; ES

1. Verfahren zur Herstellung von Verbindungen der Formel I oder dern Salze,

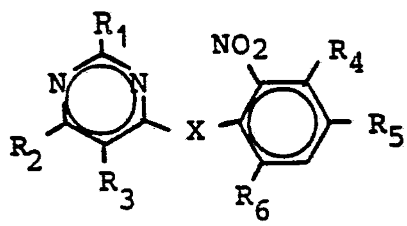
(I),

worin

$R_1$ und $R_2$ unabhängig voneinander Wasserstoff; Halogen, Cyano; $(C_1-C_8)$-Alkyl; $(C_1-C_8)$-Haloalkyl; $(C_1-C_8)$-Alkyl, das ein-oder zweifach durch Nitro, Cyano, $(C_1-C_4)$-Alkoxy, $(C_1-C_4)$-Alkylthio oder $-N(R_7)$ $(R_8)$ substituiert ist; $(C_3-C_8)$-Cycloalkyl, das durch $(C_1-C_4)$Alkyl substituiert sein kann, $(C_2-C_4)$-Alkenyl; $(C_2-C_4)$-Haloalkenyl; $(C_3-C_4)$-Alkinyl; $(C_3-C_4)$-Haloalkinyl; $(C_5-C_6)$-Cycloalkenyl; eine Gruppe $-N(R_7)$ $(R_8)$; $(C_1-C_8)$-Alkylthio; $(C_1-C_8)$-Alkoxy; $(C_1-C_8)$Haloalkoxy; $(C_1-C_8)$Alkylsulfinyl; $(C_1-C_8)$-Alkylsulfonyl; Phenyl, das gegebenenfalls
durch Halogen, Nitro, Cyano, $(C_1-C_4)$-Alkyl, $(C_1-C_4)$-Alkoxy, $(C_1-C_4)$-Alkylthio oder $(C_1-C_4)$-Haloalkyl substituiert ist, oder Phenoxy, das gegebenenfalls
durch Halogen, $NO_2$, $(C_1-C_4)$Alkyl, $(C_1-C_4)$Haloalkyl oder $(C_1-C_4)$Alkoxy substituiert ist;

$R_3$ Wasserstoff, Halogen, Cyano, Nitro, $(C_1-C_8)$-Alkyl, $(C_1-C_8)$-Haloalkyl, $(C_2-C_4)$-Alkenyl, $(C_2-C_4)$-Haloalkyenyl, Phenyl, das gegebenenfalls durch Halogen, Nitro, Cyano, $(C_1-C_4)$-Alkyl, $(C_1-C_4)$-Alkoxy, $(C_1-C_4)$-Alkylthio oder $(C_1-C_4)$-Haloalkyl substituiert ist, oder Phenoxy, das gegebenenfalls durch Halogen $NO_2$, $(C_1-C_4)$Alkyl, $(C_1-C_4)$-Haloalkyl, $(C_1-C_4)$Alkoxy substituiert ist;

$R_4$ Wasserstoff, Halogen, $(C_1-C_4)$Alkoxy, $(C_1-C_4)$Alkylthio, eine Gruppe $N(R_7)(R_8)$ oder Phenoxy, das durch Halogen substituiert sein kann;

$R_5$ $(C_1-C_4)$Alkyl, ($C_1$-$C_8$-Alkoxycarbonyl), ($C_1$-$C_4$-Alkenyloxy)carbonyl, $-CO_2H$ ($C_3$-$C_4$-Alkinyloxy)carbonyl; ($C_1$-$C_4$-Haloalkoxy)carbonyl; ($C_2$-$C_4$-Haloalkenyloxy)carbonyl; ($C_3$-$C_4$-Haloalkinyloxy)carbonyl; - $CON(R_7)(R_8)$; ($C_1$-$C_4$-

Alkyl)carbonyl; ($C_2$-$C_4$-Alkenyl)carbonyl; ($C_3$-$C_4$-Alkinyl)carbonyl; ($C_3$-$C_4$-Haloalkyl)carbonyl; ($C_2$-$C_4$-Haloalkenyl)carbonyl, ($C_3$-$C_4$-Haloalkinyl)-carbonyl, $S(O)_nR_9$; $S(O)_n$ $OR_9$ oder $S(O)_nN(R_7)(R_8)$; Halogen; Cyano, Nitro ($C_1$-$C_4$)Haloalkoxy; ($C_1$-$C_4$)Haloalkyl; Formyl;

S(O) $_nR_9$; $S(O)_nOR_9$ oder $S(O)_nN(R_7)$ ($R_8$);

n eine der Zahlen 0, 1 oder 2;

$R_6$ $NO_2$ oder $CF_3$;

$R_7$ und $R_8$ unabhängig voneinander Wasserstoff, ($C_1$-$C_4$)-Alkyl; ($C_3$--$C_7$)-Cycloalkyl, das durch ($C_1$-$C_4$)-Alkyl substituiert sein kann; oder Phenyl, das durch Halogen, ($C_1$-$C_4$)-Alkyl, ($C_1$-$C_4$)-Haloalkyl, ($C_1$-$C_4$)-Alkoxy; ($C_1$-$C_4$)Alkylthio, Nitro oder Cyano substituiert sein kann; $R_9$ ($C_1$-$C_4$)-Alkyl; ($C_2$-$C_4$)-Alkenyl, ($C_2$-$C_4$)-Alkinyl oder ($C_3$-$C_7$)-Cycloalkyl, das durch ($C_1$-$C_4$)-Alkyl substituiert sein kann;

$R_{10}$ unabhängig voneinander Wasserstoff; ($C_1$-$C_4$)-Alkyl; ($C_3$-$C_7$)-Cycloalkyl, das durch ($C_1$-$C_4$)-Alkyl substituiert sein kann; ($C_1$-$C_4$)Alkoxy)carbonyl, ($C_1$-$C_4$-Alkyl)carbonyl; ($C_1$-$C_4$)-Alkoxy; Phenyl, das gegebenenfalls durch Halogen, Nitro, Cyano ($C_1$-$C_4$)-Alkyl, ($C_1$-$C_4$)-Alkoxy, ($C_1$-$C_4$)-Alkylthio oder ($C_1$-$C_4$)-Haloalkyl substituiert sein kann, und

X eine der Gruppen -$NR_{10}$-$NR_{10}$, -$NR_{10}$-oder-O-bedeuten mit der Maßgabe, daß diejenigen Verbindungen der Formel I, worin X eine Gruppe -$NR_{10}$-und $R_5$ $CF_3$ oder $NO_2$ bedeuten, ausgenommen sind, dadurch gekennzeichnet, daß man eine Verbindung der Formel II mit einer Verbindung der Formel III

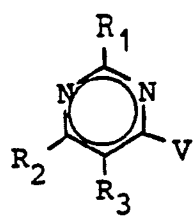
(II)

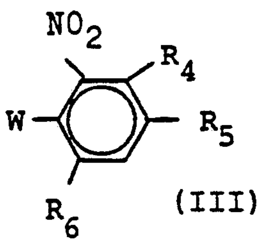
(III)

worine die Reste V, W für Halogen, Hydroxy, ein Gruppe $NR_{10}NH_2$ oder -$NHR_{10}$ stehen, wobei für den Fall, daß W für Halogen steht, V Hydroxy, eine Gruppe -$NR_{10}$-$NHR_{10}$ oder eine Gruppe -$NHR_{10}$ bedeuten muß und für den Fall, daß V für Halogen steht, W Hydroxy, eine Gruppe -$NR_{10}NHR_{10}$ oder eine Gruppe -$NHR_{10}$ bedeuten muß,

in Gegenwart einer Base umsetzt und die erhaltenen Verbindungen gegebenenfalls in ihre Salze überführt.

2. Verfahren zur Herstellung von Verbindungen der Formel I gemäß Anspruch 1 oder deren Salze, wobei

$R_1$ Wasserstoff; Halogen; Cyano; ($C_1$-$C_8$)-Alkyl;

($C_1$-$C_8$)-Haloalkyl; ($C_1$-$C_8$)-Alkyl, das ein-oder zweifach durch ($C_1$-$C_4$)-Alkoxy oder ($C_1$-$C_4$)-Alkylthio substituiert ist;

($C_3$-$C_8$)-Cycloalkyl, das durch ($C_1$-$C_4$)Alkyl substituiert sein kann; ($C_1$-$C_8$)-Alkylthio; ($C_1$-$C_8$)-Alkoxy;

($C_1$-$C_8$)Alkylsulfinyl; ($C_1$-$C_8$)-Alkylsulfonyl; Phenyl, das gegebenenfalls ein-oder mehrfach durch Halogen und/oder ein-oder dreifach durch Nitro, Cyano, ($C_1$-$C_4$)-Alkyl, ($C_1$-$C_4$)-Alkoxy oder ($C_1$-$C_4$)-Haloalkyl substituiert ist,

$R_2$ die Bedeutung von $R_1$ außer gegebenenfalls substituiertes Phenyl.

$R_3$ Wasserstoff; Halogen; Cyano; Nitro; ($C_1$-$C_8$)-Alkyl; ($C_1$-$C_8$)-Haloalkyl oder Phenoxy, das gegebenenfalls einbis dreimal durch Halogen $NO_2$, ($C_1$-$C_4$)Alkyl, ($C_1$-$C_4$)Haloalkyl, oder ($C_1$-$C_4$)Alkoxy substituiert ist;

$R_4$ Wasserstoff; Halogen; ($C_1$-$C_4$)Alkoxy;

$R_5$ ($C_1$-$C_4$)Alkyl, ($C_1$-$C_4$-Alkoxy)carbonyl, -$CO_2H$; -$CON(R_7)(R_8)$; $C_1$-$C_4$-Alkyl)carbonyl; ($C_3$-$C_4$-Haloalkyl)carbonyl; Cyano, Halogen, Nitro; oder $S(O)_nN(R_7)$ ($R_8$);

n eine der Zahlen 0, 1 oder 2;

$R_6$ $NO_2$ oder $CF_3$;

$R_7$ und $R_8$ unabhängig voneinander Wasserstoff; ($C_1$-$C_4$)-Alkyl; ($C_3$-$C_7$)-Cycloalkyl, das durch ($C_1$-$C_4$)-Alkyl substituiert sein kann; oder Phenyl, das ein-bis dreifach durch Halogen, ($C_1$-$C_4$)-Haloalkyl oder ($C_1$-$C_4$)-Alkoxy, substituiert sein kann;

$R_{10}$ unabhängig von einander Wasserstoff; ($C_1$-$C_4$)-Alkyl; und

X eine der Gruppen -$NR_{10}$-$NR_{10}$ oder -$NR_{10}$-oder -0-bedueuten.

3. Verfahren zur Herstellung der Verbindungen Formel I gemäß Anspruch 1, oder deren Salze, wobei $R_1$ $(C_1-C_8)$Haloalkyl, $(C_1-C_4)$-alkyl; $(C_3-C_6)$-Cycloalkyl, Phenyl oder Phenyl, das wie oben angegeben substituiert ist, insbesondere hiervon $(C_1-C_8)$-Haloalkyl, $R_2$ H oder $(C_1-C_4)$-Alkyl,
$R_3$ H oder Halogen,
$R_4$ H oder Halogen.
$R_5$ $(C_1-C_4)$Alkoxycarbonyl, Cyano; $(C_1-C_4)$-Haloalkoxy, $(C_1-C_4)$-Haloalkyl oder Chlor,
$R_6$ $NO_2$ und X = NH oder -0-bedeuten.

4. Schädlingsbekämpfungsmittel, dadurch gekennzeichnet, daß es eine Verbindung der Formel I oder deren Salze von Ansprüchen 1, 2 oder 3 in einer wirksamen Menge neben geeigneten Formulierungshilfsmitteln enthält.

5. Fungizide Mittel, dadurch gekennzeichnet, daß es eine Verbindung der Formel I oder dern Salz von Ansprüchen 1, 2 oder 3 in einer wirksamen Menge neben geeigneten Formulierungshilfsmitteln enthält.

6. Verwendung der Verbindungen der Formel I oder deren Salze von Ansprüchen 1, 2 oder 3 zur Bekämpfung von tierischen Schädlingen oder Schadpilzen.

7. Verfahren zur Bekämpfung von tierischen Schädlingen oder Schadpilzen, dadurch gekennzeichnet, daß man auf diese oder die von ihnen befallenen Pflanzen, oder Anbauflächen eine wirksame Menge einer Verbindung der Formel I oder deren Salze von Ansprüchen 1, 2 oder 3 appliziert.